# EUROPEAN PATENT SPECIFICATION

(11) **EP 3 567 118 B1**
(45) Date of publication and mention of the grant of the patent: **14.08.2024**
(21) Application number: 17880997.6
(22) Date of filing: 15.12.2017
(51) Int. Cl.: C12Q 1/6883, C12Q 1/689

(54) **METHOD FOR DIAGNOSING CARDIAC DISEASE THROUGH BACTERIAL METAGENOME ANALYSIS**
VERFAHREN ZUR DIAGNOSE VON HERZKRANKHEITEN DURCH BAKTERIELLE METAGENOMANALYSE
PROCÉDÉ DE DIAGNOSTIC D'UNE MALADIE CARDIAQUE PAR ANALYSE DE MÉTAGÉNOME BACTÉRIEN

(30) Priority: 16.12.2016 KR 20160172414; 14.12.2017 KR 20170172461
(43) Date of publication of application: 13.11.2019
(73) Proprietor: MD Healthcare Inc., Seoul 03923 (KR)
(72) Inventor: KIM, Yoon-Keun, Namyangju-si Gyeonggi-do12146 (KR)
(74) Representative: Dehns
(86) International application number: PCT/KR2017/014815
(87) International publication number: WO 2018/111028

(56) References cited:
- WO-A1-2012/050513
- CN-A- 106 192 022
- KR-A- 20160 073 157
- KR-A- 20160 073 157
- US-A1- 2014 086 954
- US-A1- 2015 232 934
- YOU-SUN KIM ET AL: "IgG Sensitization to Extracellular Vesicles in Indoor Dust Is Closely Associated With the Prevalence of Non-Eosinophilic Asthma, COPD, and Lung Cancer", ALLERGY, ASTHMA & IMMUNOLOGY RESEARCH : AAIR, vol. 8, no. 3, 1 May 2016 (2016-05-01), US, pages 198, XP055713012, ISSN: 2092-7355, DOI: 10.4168/aair.2016.8.3.198
- JAE YOUNG YOO ET AL: "16S rRNA gene-based metagenomic analysis reveals differences in bacteria-derived extracellular vesicles in the urine of pregnant and non-pregnant women", EXPERIMENTAL & MOLECULAR MEDICINE, vol. 48, no. 2, 5 February 2016 (2016-02-05), pages e208, XP055321074, DOI: 10.1038/emm.2015.110
- HYUN JUNG KIM ET AL: "The microbiome of the lung and its extracellular vesicles in nonsmokers, healthy smokers and COPD patients", EXPERIMENTAL & MOLECULAR MEDICINE, vol. 49, no. 4, 1 April 2017 (2017-04-01), pages e316 - e316, XP055732390, DOI: 10.1038/emm.2017.7
- JIN-YOUNG PARK ET AL: "Metagenome Analysis of Bodily Microbiota in a Mouse Model of Alzheimer Disease Using Bacteria-derived Membrane Vesicles in Blood", EXPERIMENTAL NEUROBIOLOGY, vol. 26, no. 6, 26 July 2017 (2017-07-26), pages 369, XP055547921, ISSN: 1226-2560, DOI: 10.5607/en.2017.26.6.369
- DAYLE JOHNSTON ET AL: "Illumina MiSeq 16S amplicon sequence analysis of bovine respiratory disease associated bacteria in lung and mediastinal lymph node tissue", BMC VETERINARY RESEARCH, vol. 13, no. 1, 2 May 2017 (2017-05-02), XP055713124, DOI: 10.1186/s12917-017-1035-2
- YUANA, Y. ET AL.: "Extracellular Vesicles in Physiological and Pathological Conditions", BLOOD REVIEWS, vol. 27, no. 1, 1 January 2013 (2013-01-01), pages 31 - 39, XP055495785, Retrieved from the Internet <URL:https://doi.org/10.1016/j.blre.2012.12.002>

## Description

### [Technical Field]

The present disclosure relates to a method of diagnosing a heart disease through bacterial metagenomic analysis, and more particularly, to a method of diagnosing a risk factor for a heart disease by analyzing an increase or decrease in content of extracellular vesicles derived from specific bacteria by bacterial metagenomic analysis using a sample derived from a subject.

### [Background Art]

Heart disease is a disease occurring in the heart, and examples of major diseases thereof include ischemic heart disease, coronary artery disease, angina, myocardial infarction, arrhythmia, and the like. Among these, coronary artery disease is a group of diseases including angina, myocardial infarction, and the like and is also known as ischemic heart disease. Myocardial infarction is a disease in which an infarction occurs in heart muscles due to blocking of blood vessels that is caused by thrombosis in the coronary arteries. As risk factors for coronary artery disease, hypertension, smoking, diabetes, lack of exercise, obesity, hyperlipidemia, excessive drinking, and the like are known. It has been reported that the above-described disease is prevented when fruit- and vegetable-rich foods are consumed, and a risk for the disease is increased when trans fat-rich foods are consumed.

Dilated cardiomyopathy, which is a disease in which the heart is expanded and unable to contract properly, is known as the most common cause of non-coronary artery disease and cardiomyopathy. Toxins, metabolic disorders, infectious factors, and the like have been suggested as causative factors, but causative factors have not yet been found.

Angina is a disease occurring due to narrowing of the coronary arteries, through which blood is supplied to the heart, causing the volume of blood flowing to heart muscles to fall short of the required amount. When the coronary arteries are narrowed, blood supply is insufficient when in need thereof, and thus chest pain symptoms occur, and when angina remains untreated, it develops into acute myocardial infarction, thus causing sudden death. Angina may be broadly divided into stable angina, unstable angina, and variant angina. Among these three types, while stable angina and unstable angina occur mostly by atherosclerotic plaques, variant angina occurs due to a different cause, which thus has its name. Unlike general angina occurring due to narrowing of blood vessels, variant angina is a disease occurring due to the occurrence of spasms and poor blood circulation although the diameter of blood vessels of the coronary arteries is normal. Variant angina causes chest pain due to a decrease in or blocking of blood flow by spasm-derived contractions, and these symptoms occur early the next morning when drinking or receiving a lot of stress, or the like.

Atrial fibrillation is a condition in which the atria beat continuously and irregularly and a rapid and irregular heart rate occurs. The atria beat 300 to 400 times per minute, while most stimuli are blocked by the atrioventricular (AV) node, and stimuli delivered to the ventricles are approximately 75 to 175 per minute. Causes of the disease include fundamental heart problems, i.e., coronary artery disease, myocardial infarction, hypertension, and mitral valve, and further include pericarditis, pulmonary embolisms, hyperthyroidism or hypothyroidism, septicaemia, diabetes, excessive drinking, pheochromocytoma, and the like.

According to data released by the Korea National Statistical Office in 2013, the three leading causes of death in Korea are malignant neoplasm (cancer) accounting for 28.3%, cerebrovascular disease accounting for 9.6%, and heart disease accounting for 9.5%, and these account for about 50% of all causes of death. It has also been reported that coronary artery disease and cardiomyopathy account for 50% or greater of the causes of death due to heart disease. Modern people face stress caused by conflicts such as economic problems, workplace problems, and the like, and thus blood pressure, blood sugar, and blood fat are increased, and coronary artery disease and cardiomyopathy are very closely associated with diseases such as obesity, smoking, atherosclerosis, and the like, which continue to increase in modern people, and therefore, heart disease is a disease that may occur suddenly by the above-described causes and has a very serious prognosis. Therefore, there is an urgent need to develop a method capable of diagnosing heart disease early and increasing therapeutic efficiency, and prior to this, it is very important to differentiate countermeasures for early diagnosis and treatment by predicting the onset of heart disease, and thus research thereon and technology development thereof are required.

Meanwhile, it is known that the number of microorganisms symbiotically living in the human body is 100 trillion which is 10 times the number of human cells, and the number of genes of microorganisms exceeds 100 times the number of human genes. A microbiota is a microbial community that includes bacteria, archaea, and eukaryotes present in a given habitat. The intestinal microbiota is known to play a vital role in human's physiological phenomena and significantly affect human health and diseases through interactions with human cells. Bacteria co-existing in human bodies secrete nanometer-sized vesicles to exchange information about genes, proteins, and the like with other cells. The mucous membranes form a physical barrier membrane that does not allow particles with the size of 200 nm or more to pass therethrough, and thus bacteria symbiotically living in the mucous membranes are unable to pass therethrough, but bacterial-derived extracellular vesicles have a size of approximately 100 nm or less and thus relatively freely pass through the mucous membranes and are absorbed into the human body.

Metagenomics, also called environmental genomics, may be analytics for metagenomic data obtained from samples collected from the environment, and collectively refers to a total genome of all microbiota in the natural environment in which microorganisms exist and was first used by Jo Handelsman in 1998 (Handelsman et al., 1998 Chem. Biol. 5, R245-249). Recently, the bacterial composition of human microbiota has been listed using a method based on 16s ribosomal RNA (16s rRNA) base sequences, and 16s ribosomal RNA analyzes sequences using a 454FLX titanium platform. In the onset of heart disease, however, identification of causative factors such as heart disease, particularly myocardial infarction, cardiomyopathy, variant angina, atrial fibrillation, and the like through metagenomic analysis of bacteria-derived vesicles isolated from a human-derived substance, such as blood, urine, or the like, and a method of diagnosing the heart disease have never been reported.

### [Disclosure]

### [Technical Problem]

To diagnose heart disease such as myocardial infarction, cardiomyopathy, variant angina, atrial fibrillation, and the like, the inventors of the present invention extracted DNA from bacteria-derived vesicles using serum, which is a subject-derived sample, and performed metagenomic analysis on the extracted DNA, and, as a result, identified bacteria-derived extracellular vesicles capable of acting as a causative factor of heart disease, thus completing the present invention.

Therefore, it is an object of the present invention to provide a method of providing information for heart disease diagnosis by metagenomic analysis of bacteria-derived extracellular vesicles.

However, the technical goals of the present invention are not limited to the aforementioned goals, and other unmentioned technical goals will be clearly understood by those of ordinary skill in the art from the following description.

### [Technical Solution]

Described herein but not forming part of the invention is a method of providing information for heart disease diagnosis, comprising the following processes:
(a) extracting DNA from extracellular vesicles isolated from a subject sample;
(b) performing polymerase chain reaction (PCR) on the extracted DNA using a pair of primers having SEQ ID NO:1 and SEQ ID NO: 2; and
(c) comparing an increase or decrease in content of bacteria-derived extracellular vesicles of the subject sample with that of a normal individual-derived sample through sequencing of a product of the PCR.

The methods of the invention of diagnosing heart disease have been described in the appended claims. Described herein but not forming part of the invention is a method of diagnosing heart disease, comprising the following processes:
(a) extracting DNA from extracellular vesicles isolated from a subject sample;
(b) performing polymerase chain reaction (PCR) on the extracted DNA using a pair of primers having SEQ ID NO:1 and SEQ ID NO: 2; and
(c) comparing an increase or decrease in content of bacteria-derived extracellular vesicles of the subject sample with that of a normal individual-derived sample through sequencing of a product of the PCR.

Described herein but not forming part of the invention is a method of predicting a risk for heart disease, comprising the following processes:
(a) extracting DNA from extracellular vesicles isolated from a subject sample;
(b) performing polymerase chain reaction (PCR) on the extracted DNA using a pair of primers having SEQ ID NO:1 and SEQ ID NO: 2; and
(c) comparing an increase or decrease in content of bacteria-derived extracellular vesicles of the subject sample with that of a normal individual-derived sample through sequencing of a product of the PCR.

The subject sample is blood. Comparing an increase or decrease in content of extracellular vesicles have been described in the appended claims. Embodiments described herein do not form part of the invention and may comprise comparing an increase or decrease in content of:
extracellular vesicles derived from one or more bacteria selected from the group consisting of the phylum *Acidobacteria,* the phylum *Firmicutes,* the phylum *Crenarchaeota,* the phylum *Planctomycetes,* the phylum *Chloroflexi,* the phylum *Euryarchaeota,* the phylum WS3, the phylum *Nitrospirae,* the phylum WPS-2, the phylum AD3, the phylum *Verrucomicrobia,* the phylum *Gemmatimonadetes,* the phylum *Proteobacteria,* the phylum TM7, and the phylum *Cyanobacteria;*
extracellular vesicles derived from one or more bacteria selected from the group consisting of the class *Acidobacteriia,* the class DA052, the class *Methanomicrobia,* the class *Thaumarchaeota,* the class *Clostridia,* the class *Coriobacteriia,* the class *Betaproteobacteria,* the class *Ktedonobacteria,* the class *Planctomycetia,* the class *Solibacteres,* the class *Erysipelotrichi,* the class *Verrucomicrobiae,* the class TM7-3, the class *Bacteroidia,* the class *Phycisphaerae,* the class MCG, the class *Nitrospira,* the class *Pedosphaerae,* the class *Thermoleophilia,* the class *Saprospirae,* the class PRR-12, the class *Spartobacteria,* the class *Acidimicrobiia,* the class TM1, the class *Deltaproteobacteria,* the class *Anaerolineae,* the class *Thermoplasmata,* the class *Chthonomonadetes,* the class *Acidobacteria-b,* the class *Fusobacteriia,* the class *Fimbriimonadia,* the class *Actinobacteria,* the class *Flavobacteriia,* and the class *Chloroplast;*
extracellular vesicles derived from one or more bacteria selected from the group consisting of the order *Lactobacillales,* the order *Acidobacteriales,* the order *Enterobacteriales,* the order *Xanthomonadales,* the order *Clostridiales,* the order *Coriobacteriales,* the order Ellin6513, the order *Burkholderiales,* the order *Erysipelotrichales,* the order *Solibacterales,* the order *Verrucomicrobiales,* the order *Rhodospirillales,* the order *Gemmatales,* the order *Thermogemmatisporales,* the order *Saprospirales,* the order *Acidimicrobiales,* the order *Pedosphaerales,* the order *Bifidobacteriales,* the order *Chthoniobacterales,* the order *Solžrubrobacterales,* the order *Syntrophobacterales,* the order *Bacteroidales,* the order *Nitrospirales,* the order *Ktedonobacterales,* the order WD2101, the order iii1-15, the order Ellin329, the order *Thiotrichales,* the order *Myxococcales,* the order *Stramenopiles,* the order *Vibrionales,* the order *Pseudomonadales,* the order *Bacillales,* the order *Sphingomonadales,* the order *Turicibacterales,* the order *Fimbriimonadales,* the order *Deinococcales,* the order *Pasteurellales,* the order *Flavobacteriales,* the order *Actinomycetales,* the order *Rhodobacterales,* and the order *Streptophyta*;
extracellular vesicles derived from one or more bacteria selected from the group consisting of the family *Koribacteraceae,* the family *Comamonadaceae,* the family *Enterobacteriaceae,* the family *Streptococcaceae,* the family *Coriobacteriaceae,* the family *Lachnospiraceae,* the family *Prevotellaceae,* the family *Ruminococcaceae,* the family *Xanthomonadaceae,* the family *Propionibacteriaceae,* the family *Hyphomicrobiaceae,* the family *Verrucomicrobiaceae,* the family *Solibacteraceae,* the family *Acidobacteriaceae,* the family *Erysipelotrichaceae,* the family *Ktedonobacteraceae,* the family *Thermogemmatisporaceae,* the family *Moraxellaceae,* the family *Veillonellaceae,* the family *Burkholderiaceae,* the family *Rhodospirillaceae,* the family *Bifidobacteriaceae,* the family *Gemmataceae,* the family *Streptomycetaceae,* the family *Chitinophagaceae,* the family *Brucellaceae,* the family *Rhizobiaceae,* the family *Chthoniobacteraceae,* the family *Sinobacteraceae,* the family *Conexžbacteraceae,* the family *Oxalobacteraceae,* the family *Isosphaeraceae,* the family Ellin515, the family *Piscirickettsiaceae,* the family *Methylocystaceae,* the family *Pseudomonadaceae,* the family *Clostridiaceae,* the family *Sphingomonadaceae,* the family *Turicibacteraceae,* the family *Staphylococcaceae,* the family *Deinococcaceae,* the family *Methylobacteriaceae,* the family *Aerococcaceae,* the family *Fusobacteriaceae,* the family *Fimbriimonadaceae,* the family *Bacillaceae,* the family *Planococcaceae,* the family *BacteroidaceaeAxc* family *Weeksellaceae,* the family *Desulfovibrionaceae,* the family *Flavobacteriaceae,* the family *Rikenellaceae,* the family S24-7, the family *Pasteurellaceae,* the family *Rhodobacteraceae,* the family *Gordoniaceae,* and the family *Enterococcaceae*; or
extracellular vesicles derived from one or more bacteria selected from the group consisting of the genus *Delftia,* the genus *Agrobacterium,* the genus *Stenotrophomonas,* the genus *Faecalibacterium,* the genus *Candidatus Koribacter,* the genus *Akkermansia,* the genus *Streptococcus,* the genus *Salinispora,* the genus *Candidatus Solibacter,* the genus *Citrobacter,* the genus *Collinsella,* the genus *Burkholderia,* the genus *Coprococcus,* the genus *Rhodoplanes,* the genus *Acinetobacter,* the genus *Prevotella,* the genus *Propionibacterium,* the genus *Lactococcus,* the genus *Bifidobacterium,* the genus *Methanobacterium,* the genus *Micrococcus,* the genus *Methanocella,* the genus *Brevibacterium,* the genus *Streptacidiphilus,* the genus *Streptomyces,* the genus *Ochrobactrum,* the genus *Methanosaeta,* the genus *Lysinibacillus,* the genus *Cupriavidus,* the genus *Pseudomonas,* the genus *Clostridium,* the genus *Sphingomonas,* the genus *Staphylococcus,* the genus *Thermoanaerobacterium,* the genus *Neisseria,* the genus *Enhydrobacter,* the genus *Actinomyces,* the genus *Turicibacter,* the genus *Phascolarctobacterium,* the genus *Catenibacterium,* the genus *Bacillus,* the genus Deinococcus, the genus Fusobacterium, the genus Adlercreutzia, the genus *Chryseobacterium,* the genus *Enterococcus,* the genus *Alcanivorax,* the genus *Psychrobacter,* the genus *Bacteroides,* the genus *Geobacillus,* the genus *Haemophilus,* the genus *Veillonella,* the genus *Paracoccus,* the genus *Kocuria,* the genus *Halomonas,* the genus *Ruminococcus,* and the genus *Porphyromonas.*

The heart disease is myocardial infarction, cardiomyopathy, variant angina, or atrial fibrillation.

Embodiments described herein do not form part of the invention.

In another embodiment of the present invention, in process (c), myocardial infarction may be diagnosed by comparing an increase or decrease in content of extracellular vesicles derived from one or more bacteria selected from the group consisting of the phylum *Acidobacteria,* the phylum *Firmicutes,* the phylum *Crenarchaeota,* the phylum *Planctomycetes,* the phylum *Chloroflexi,* the phylum *Euryarchaeota,* the phylum WS3, the phylum *Nitrospirae,* the phylum WPS-2, and the phylum AD3.

In another embodiment of the present invention, in process (c), myocardial infarction may be diagnosed by comparing an increase or decrease in content of extracellular vesicles derived from one or more bacteria selected from the group consisting of the class *Acidobacteriia,* the class DA052, the class *Methanomicrobia,* the class *Thaumarchaeota,* the class *Clostridia,* the class *Coriobacteriia,* the class *Betaproteobacteria,* the class *Ktedonobacteria,* the class *Planctomycetia,* the class *Solibacteres,* the class *Erysipelotrichi,* the class *Verrucomicrobiae,* the class TM7-3, the class *Bacteroidia,* the class *Phycisphaerae,* the class MCG, the class *Nitrospira,* the class *Pedosphaerae,* the class *Thermoleophilia,* the class *Saprospirae,* the class PRR-12, the class *Spartobacteria,* the class *Acidimicrobiia,* the class TM1, the class *Deltaproteobacteria,* the class *Anaerolineae,* the class *Thermoplasmata,* the class *Chthonomonadetes,* and the class *Acidobacteria*-6.

In another embodiment of the present invention, in process (c), myocardial infarction may be diagnosed by comparing an increase or decrease in content of extracellular vesicles derived from one or more bacteria selected from the group consisting of the order *Lactobacillales,* the order *Acidobacteriales,* the order *Enterobacteriales,* the order *Xanthomonadales,* the order *Clostridiales,* the order *Coriobacteriales,* the order Ellin6513, the order *Burkholderiales,* the order *Erysipelotrichales,* the order *Solibacterales,* the order *Verrucomicrobiales,* the order *Rhodospirillales,* the order *Gemmatales,* the order *Thermogemmatisporales,* the order *Saprospirales,* the order *Acidimicrobiales,* the order *Pedosphaerales,* the order *Bifidobacteriales,* the order *Chthoniobacterales,* the order *Solirubrobacterales,* the order *Syntrophobacterales,* the order *Bacteroidales,* the order *Nitrospirales,* the order *Ktedonobacterales,* the order WD2101, the order iii1-15, the order Ellin329, the order *Thiotrichales,* the order *Myxococcales,* the order *Stramenopiles,* and the order *Vibrionales.*

In another embodiment of the present invention, in process (c), myocardial infarction may be diagnosed by comparing an increase or decrease in content of extracellular vesicles derived from one or more bacteria selected from the group consisting of the family *Koribacteraceae,* the family *Comamonadaceae,* the family *Enterobacteriaceae,* the family *Streptococcaceae,* the family *Coriobacteriaceae,* the family *Lachnospiraceae,* the family *Prevotellaceae,* the family *Ruminococcaceae,* the family *Xanthomonadaceae,* the family *Propionibacteriaceae,* the family *Hyphomicrobiaceae,* the family *Verrucomicrobiaceae,* the family *Solibacteraceae,* the family *Acidobacteriaceae,* the family *Erysipelotrichaceae,* the family *Ktedonobacteraceae,* the family *Thermogemmatisporaceae,* the family *Moraxellaceae,* the family *Veillonellaceae,* the family *Burkholderiaceae,* the family *Rhodospirillaceae,* the family *Bifidobacteriaceae,* the family *Gemmataceae,* the family *Streptomycetaceae,* the family *Chitinophagaceae,* the family *Brucellaceae,* the family *Rhizobiaceae,* the family *Chthoniobacteraceae,* the family *Sinobacteraceae,* the family *Conexibacteraceae,* the family *Oxalobacteraceae,* the family *Isosphaeraceae,* the family Ellin515, the family *Piscirickettsiaceae,* and the family *Methylocystaceae.*

In another embodiment of the present invention, in process (c), myocardial infarction may be diagnosed by comparing an increase or decrease in content of extracellular vesicles derived from one or more bacteria selected from the group consisting of the genus *Delftia,* the genus *Agrobacterium,* the genus *Stenotrophomonas,* the genus *Faecalibacterium,* the genus *Candidatus Koribacter,* the genus *Akkermansia,* the genus *Streptococcus,* the genus *Salinispora,* the genus *Candidatus Solibacter,* the genus *Citrobacter,* the genus *Collinsella,* the genus *Burkholderia,* the genus *Coprococcus,* the genus *Rhodoplanes,* the genus *Acinetobacter,* the genus *Prevotella,* the genus *Propionibacterium,* the genus *Lactococcus,* the genus *Bifidobacterium,* the genus *Methanobacterium,* the genus *Micrococcus,* the genus *Methanocella,* the genus *Brevibacterium,* the genus *Streptacidiphilus,* the genus *Streptomyces,* the genus *Ochrobactrum,* the genus *Methanosaeta,* the genus *Lysinibacillus,* and the genus *Cupriavidus.*

In another embodiment of the present invention, in process (c), cardiomyopathy may be diagnosed by comparing an increase or decrease in content of extracellular vesicles derived from one or more bacteria selected from the group consisting of the phylum *Verrucomicrobia,* the phylum *Acidobacteria,* the phylum *Gemmatimonadetes,* and the phylum *Planctomycetes.*

In another embodiment of the present invention, in process (c), cardiomyopathy may be diagnosed by comparing an increase or decrease in content of extracellular vesicles derived from one or more bacteria selected from the group consisting of the class *Verrucomicrobiae,* the class *Fusobacteriia,* the class *Acidobacteriia,* the class *Planctomycetia,* the class DA052, the class *Deltaproteobacteria,* and the class *Acidimicrobiia.*

In another embodiment of the present invention, in process (c), cardiomyopathy may be diagnosed by comparing an increase or decrease in content of extracellular vesicles derived from one or more bacteria selected from the group consisting of the order *Pseudomonadales,* the order *Bacillales,* the order *Acidobacteriales,* the order *Sphingomonadales,* the order *Verrucomicrobiales,* the order *Turicibacterales,* the order *Acidimicrobiales,* the order Ellin6513, the order *Xanthomonadales,* and the order *Gemmatales.*

In another embodiment of the present invention, in process (c), cardiomyopathy may be diagnosed by comparing an increase or decrease in content of extracellular vesicles derived from one or more bacteria selected from the group consisting of the family *Pseudomonadaceae,* the family *Clostridiaceae,* the family *Comamonadaceae,* the family *Oxalobacteraceae,* the family *Moraxellaceae,* the family *Verrucomicrobiaceae,* the family *Koribacteraceae,* the family *Sphingomonadaceae,* the family *Turicibacteraceae,* the family *Xanthomonadaceae,* the family *Gemmataceae,* and the family *Staphylococcaceae.*

In another embodiment of the present invention, in process (c), cardiomyopathy may be diagnosed by comparing an increase or decrease in content of extracellular vesicles derived from one or more bacteria selected from the group consisting of the genus *Pseudomonas,* the genus *Clostridium,* the genus *Cupriavidus,* the genus *Acinetobacter,* the genus *Citrobacter,* the genus *Sphingomonas,* the genus *Candidatus Koribacter,* the genus *Staphylococcus,* the genus *Thermoanaerobacterium,* the genus *Micrococcus,* the genus *Akkermansia,* the genus *Neisseria,* the genus *Enhydrobacter,* the genus *Actinomyces,* the genus *Turicibacter,* the genus *Phascolarctobacterium,* the genus *Lactococcus,* the genus *Delftia,* and the genus *Stenotrophomonas.*

In another embodiment of the present invention, in process (c), variant angina may be diagnosed by comparing an increase or decrease in content of extracellular vesicles derived from one or more bacteria selected from the group consisting of the phylum *Verrucomicrobia,* the phylum *Acidobacteria,* the phylum *Planctomycetes,* the phylum *Gemmatimonadetes,* the phylum *Chloroflexi,* and the phylum *Euryarchaeota.*

In another embodiment of the present invention, in process (c), variant angina may be diagnosed by comparing an increase or decrease in content of extracellular vesicles derived from one or more bacteria selected from the group consisting of the class *Verrucomicrobiae,* the class *Acidobacteriia,* the class *Fimbriimonadia,* the class *Erysipelotrichi,* the class *Ktedonobacteria,* and the class *Deltaproteobacteria.*

In another embodiment of the present invention, in process (c), variant angina may be diagnosed by comparing an increase or decrease in content of extracellular vesicles derived from one or more bacteria selected from the group consisting of the order *Pseudomonadales,* the order *Erysipelotrichales,* the order *Fimbriimonadales,* the order *Acidobacteriales,* the order *Verrucomicrobiales,* the order *Xanthomonadales,* the order *Myxococcales,* the order *Deinococcales,* and the order *Rhodospirillales.*

In another embodiment of the present invention, in process (c), variant angina may be diagnosed by comparing an increase or decrease in content of extracellular vesicles derived from one or more bacteria selected from the group consisting of the family *Koribacteraceae,* the family *Oxalobacteraceae,* the family *Comamonadaceae,* the family *Moraxellaceae,* the family *Pseudomonadaceae,* the family *Hyphomicrobiaceae,* the family *Erysipelotrichaceae,* the family *Deinococcaceae,* the family *Clostridiaceae,* the family *Verrucomicrobiaceae,* the family *Sinobacteraceae,* the family *Rhodospirillaceae,* the family *Methylobacteriaceae,* the family *Aerococcaceae,* the family *Fusobacteriaceae,* the family *Fimbriimonadaceae,* the family *Bacillaceae,* and the family *Planococcaceae.*

In another embodiment of the present invention, in process (c), variant angina may be diagnosed by comparing an increase or decrease in content of extracellular vesicles derived from one or more bacteria selected from the group consisting of the genus *Citrobacter,* the genus *Acinetobacter,* the genus *Cupriavidus,* the genus *Clostridium,* the genus *Catenibacterium,* the genus *Pseudomonas,* the genus *Lactococcus,* the genus *Stenotrophomonas,* the genus *Akkermansia,* the genus *Bacillus,* the genus *Delftia,* the genus *Agrobacterium,* the genus *Deinococcus,* the genus *Fusobacterium,* and the genus *Adlercreutzia.*

In another embodiment of the present invention, in process (c), atrial fibrillation may be diagnosed by comparing an increase or decrease in content of extracellular vesicles derived from one or more bacteria selected from the group consisting of the phylum *Proteobacteria,* the phylum TM7, the phylum *Chloroflexi,* the phylum *Acidobacteria,* and the phylum *Cyanobacteria.*

In another embodiment of the present invention, in process (c), atrial fibrillation may be diagnosed by comparing an increase or decrease in content of extracellular vesicles derived from one or more bacteria selected from the class *Clostridia,* the class *Bacteroidia,* the class *Actinobacteria,* the class *Flavobacteriia,* the class *Erysipelotrichi,* the class TM7-3, and the class *Chloroplast.*

In another embodiment of the present invention, in process (c), atrial fibrillation may be diagnosed by comparing an increase or decrease in content of extracellular vesicles derived from one or more bacteria selected from the group consisting of the order *Pseudomonadales,* the order *Clostridiales,* the order *Bacteroidales,* the order *Enterobacteriales,* the order *Xanthomonadales,* the order *Bifidobacteriales,* the order *Pasteurellales,* the order *Flavobacteriales,* the order *Actinomycetales,* the order *Rhodobacterales,* the order *Coriobacteriales,* the order *Erysipelotrichales,* and the order *Streptophyta.*

In another embodiment of the present invention, in process (c), atrial fibrillation may be diagnosed by comparing an increase or decrease in content of extracellular vesicles derived from one or more bacteria selected from the group consisting of the family *Lachnospiraceae,* the family *Bacillaceae,* the family *Streptococcaceae,* the family *Bacteroidaceae,* the family *Moraxellaceae,* the family *Ruminococcaceae,* the family *Weeksellaceae,* the family *Bifidobacteriaceae,* the family *Clostridiaceae,* the family *Desulfovibrionaceae,* the family *Veillonellaceae,* the family *Coriobacteriaceae,* the family *Flavobacteriaceae,* the family *Rikenellaceae,* the family S24-7, the family *Pasteurellaceae,* the family *Rhodobacteraceae,* the family *Pseudomonadaceae,* the family *Gordoniaceae,* and the family *Enterococcaceae.*

In another embodiment of the present invention, in process (c), atrial fibrillation may be diagnosed by comparing an increase or decrease in content of extracellular vesicles derived from one or more bacteria selected from the group consisting of the genus *Acinetobacter,* the genus *Stenotrophomonas,* the genus *Chryseobacterium,* the genus *Enterococcus,* the genus *Pseudomonas,* the genus *Delftia,* the genus *Alcanivorax,* the genus *Psychrobacter,* the genus *Streptococcus,* the genus *Ochrobactrum,* the genus *Bifidobacterium,* the genus *Coprococcus,* the genus *Bacteroides,* the genus *Faecalibacterium,* the genus *Enhydrobacter,* the genus *Agrobacterium,* the genus *Citrobacter,* the genus *Prevotella,* the genus *Geobacillus,* the genus *Clostridium,* the genus *Bacillus,* the genus *Haemophilus,* the genus *Veillonella,* the genus *Actinomyces,* the genus *Paracoccus,* the genus *Kocuria,* the genus *Halomonas,* the genus *Micrococcus,* the genus *Ruminococcus,* and the genus *Porphyromonas.*

In another embodiment of the present invention, the blood may be whole blood, serum, or plasma.

### [Advantageous Effects]

According to the present invention, a risk group for heart disease can be diagnosed early and predicted by diagnosing a causative factor of heart disease through metagenomic analysis of bacteria-derived extracellular vesicles from a human body-derived sample, and thus the onset of heart disease can be delayed or heart disease may be prevented through appropriate management, and, even after heart disease occurs, early diagnosis for heart disease can be implemented, thereby lowering a disease rate and increasing therapeutic effects.

In addition, patients diagnosed with heart disease are able to avoid exposure to causative factors predicted by metagenomic analysis, whereby the progression of heart disease is ameliorated, or recurrence of heart disease can be prevented.

### [Description of Drawings]

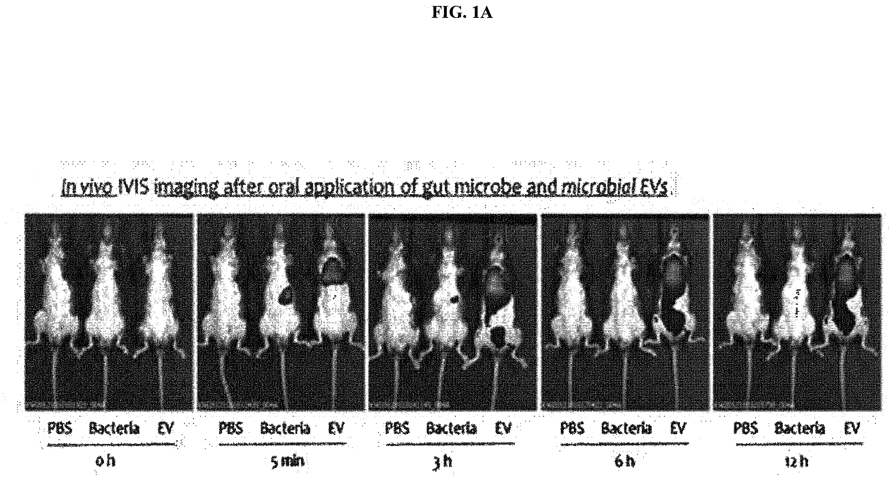

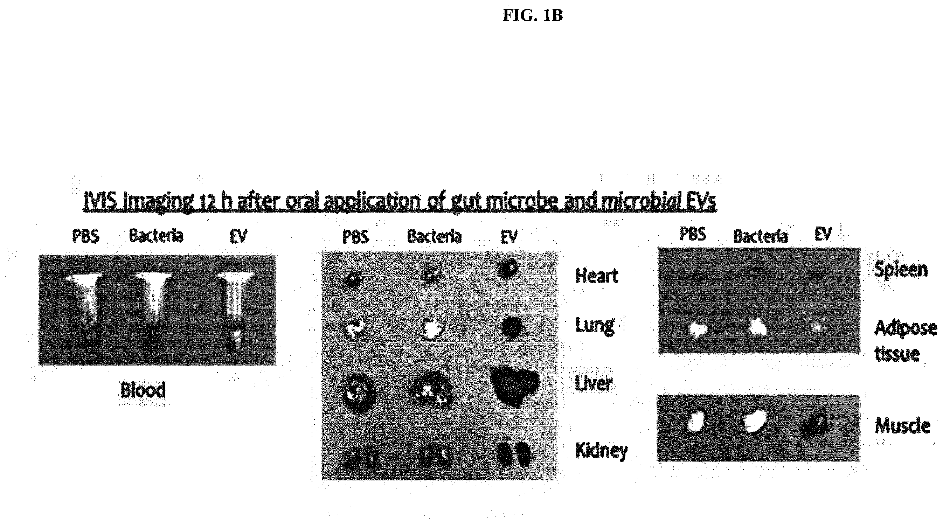

FIGS. 1A and 1B are views for evaluating the distribution pattern of extracellular vesicles (EVs) derived from bacteria *in vivo.* FIG. 1A illustrates images showing the distribution pattern of intestinal bacteria and EVs derived from bacteria per time (0 h, 5 min, 3 h, 6 h, and 12 h) after being orally administered to mice. FIG. 1B illustrates images showing the distribution pattern of gut bacteria and EVs derived from bacteria after being orally administered to mice and, after 12 hours, blood and various organs (heart, lung, liver, kidney, spleen, adipose tissue, and muscle) of the mice were extracted.
FIG. 2 illustrates distribution results of bacteria-derived EVs exhibiting significant diagnostic performance at a phylum level, after metagenomic analysis of bacteria-derived EVs isolated from myocardial infarction patient-derived blood and normal individual-derived blood.
FIG. 3 illustrates distribution results of bacteria-derived EVs exhibiting significant diagnostic performance at a class level, after metagenomic analysis of bacteria-derived EVs isolated from myocardial infarction patient-derived blood and normal individual-derived blood.
FIG. 4 illustrates distribution results of bacteria-derived EVs exhibiting significant diagnostic performance at an order level, after metagenomic analysis of bacteria-derived EVs isolated from myocardial infarction patient-derived blood and normal individual-derived blood.
FIG. 5 illustrates distribution results of bacteria-derived EVs exhibiting significant diagnostic performance at a family level, after metagenomic analysis of bacteria-derived EVs isolated from myocardial infarction patient-derived blood and normal individual-derived blood.
FIG. 6 illustrates distribution results of bacteria-derived EVs exhibiting significant diagnostic performance at a genus level, after metagenomic analysis of bacteria-derived EVs isolated from myocardial infarction patient-derived blood and normal individual-derived blood.
FIG. 7 illustrates distribution results of bacteria-derived EVs exhibiting significant diagnostic performance at a phylum level, after metagenomic analysis of bacteria-derived EVs isolated from cardiomyopathy patient-derived blood and normal individual-derived blood.
FIG. 8 illustrates distribution results of bacteria-derived EVs exhibiting significant diagnostic performance at a class level, after metagenomic analysis of bacteria-derived EVs isolated from cardiomyopathy patient-derived blood and normal individual-derived blood.
FIG. 9 illustrates distribution results of bacteria-derived EVs exhibiting significant diagnostic performance at an order level, after metagenomic analysis of bacteria-derived EVs isolated from cardiomyopathy patient-derived blood and normal individual-derived blood.
FIG. 10 illustrates distribution results of bacteria-derived EVs exhibiting significant diagnostic performance at a family level, after metagenomic analysis of bacteria-derived EVs isolated from cardiomyopathy patient-derived blood and normal individual-derived blood.
FIG. 11 illustrates distribution results of bacteria-derived EVs exhibiting significant diagnostic performance at a genus level, after metagenomic analysis of bacteria-derived EVs isolated from cardiomyopathy patient-derived blood and normal individual-derived blood.
FIG. 12 illustrates distribution results of bacteria-derived EVs exhibiting significant diagnostic performance at a phylum level, after metagenomic analysis of bacteria-derived EVs isolated from variant angina patient-derived blood and normal individual-derived blood.
FIG. 13 illustrates distribution results of bacteria-derived EVs exhibiting significant diagnostic performance at a class level, after metagenomic analysis of bacteria-derived EVs isolated from variant angina patient-derived blood and normal individual-derived blood.
FIG. 14 illustrates distribution results of bacteria-derived EVs exhibiting significant diagnostic performance at an order level, after metagenomic analysis of bacteria-derived EVs isolated from variant angina patient-derived blood and normal individual-derived blood.
FIG. 15 illustrates distribution results of bacteria-derived EVs exhibiting significant diagnostic performance at a family level, after metagenomic analysis of bacteria-derived EVs isolated from variant angina patient-derived blood and normal individual-derived blood.
FIG. 16 illustrates distribution results of bacteria-derived EVs exhibiting significant diagnostic performance at a genus level, after metagenomic analysis of bacteria-derived EVs isolated from variant angina patient-derived blood and normal individual-derived blood.
FIG. 17 illustrates distribution results of bacteria-derived EVs exhibiting significant diagnostic performance at a phylum level, after metagenomic analysis of bacteria-derived EVs isolated from atrial fibrillation patient-derived blood and normal individual-derived blood.
FIG. 18 illustrates distribution results of bacteria-derived EVs exhibiting significant diagnostic performance at a class level, after metagenomic analysis of bacteria-derived EVs isolated from atrial fibrillation patient-derived blood and normal individual-derived blood.
FIG. 19 illustrates distribution results of bacteria-derived EVs exhibiting significant diagnostic performance at an order level, after metagenomic analysis of bacteria-derived EVs isolated from atrial fibrillation patient-derived blood and normal individual-derived blood.
FIG. 20 illustrates distribution results of bacteria-derived EVs exhibiting significant diagnostic performance at a family level, after metagenomic analysis of bacteria-derived EVs isolated from atrial fibrillation patient-derived blood and normal individual-derived blood.
FIG. 21 illustrates distribution results of bacteria-derived EVs exhibiting significant diagnostic performance at a genus level, after metagenomic analysis of bacteria-derived EVs isolated from atrial fibrillation patient-derived blood and normal individual-derived blood.

### [Mode of the Invention]

The present invention relates to a method of diagnosing heart disease through bacterial metagenomic analysis. The inventors of the present invention extracted genes from bacteria-derived extracellular vesicles present in subject-derived samples, performed metagenomic analysis thereon, and identified bacteria-derived extracellular vesicles capable of acting as a causative factor of heart disease.

Described herein but not forming part of the invention is a method of providing information on heart disease diagnosis, the method including:
(a) extracting DNA from extracellular vesicles isolated from a subject sample;
(b) performing polymerase chain reaction (PCR) on the extracted DNA using a pair of primers having SEQ ID NOS: 1 and 2; and
(c) comparing an increase or decrease in content of bacteria-derived extracellular vesicles of the subject sample with that of a normal individual-derived sample through sequencing of a product of the PCR.

The term "prediction of a risk for heart disease" as used herein refers to determining whether a patient has a risk for heart disease, whether the risk for heart disease is relatively high, or whether heart disease has already occurred. The method of the present invention may be used to delay the onset of heart disease through special and appropriate care for a specific patient, which is a patient having a high risk for heart disease or prevent the onset of heart disease. In addition, the method may be clinically used to determine treatment by selecting the most appropriate treatment method through early diagnosis of heart disease.

The heart disease is myocardial infarction, cardiomyopathy, variant angina, or atrial fibrillation.

The term "metagenome" as used herein refers to the total of genomes including all viruses, bacteria, fungi, and the like in isolated regions such as soil, the intestines of animals, and the like, and is mainly used as a concept of genomes that explains identification of many microorganisms at once using a sequencer to analyze non-cultured microorganisms. In particular, a metagenome does not refer to a genome of one species, but refers to a mixture of genomes, including genomes of all species of an environmental unit. This term originates from the view that, when defining one species in a process in which biology is advanced into omics, various species as well as existing one species functionally interact with each other to form a complete species. Technically, it is the subject of techniques that analyzes all DNAs and RNAs regardless of species using rapid sequencing to identify all species in one environment and verify interactions and metabolism. In the present invention, metagenomic analysis is performed using bacteria-derived extracellular vesicles isolated from blood.

In the present invention, the subject sample may be whole blood, serum, or plasma, but the present invention is not limited therto.

In an embodiment of the present invention, metagenomic analysis was performed on the bacteria-derived extracellular vesicles, and the bacteria-derived extracellular vesicles capable of acting as a cause of the onset of heart disease were actually identified by analysis at phylum, class, order, family, and genus levels.

More particularly, in one embodiment of the present invention, as a result of performing metagenomic analysis on bacteria-derived extracellular vesicles present in myocardial infarction patient-derived blood and normal individual-derived blood at a phylum level, the content of extracelluar vesicles derived from bacteria belonging to the phylum *Acidobacteria,* the phylum *Firmicutes,* the phylum *Crenarchaeota,* the phylum *Planctomycetes,* the phylum *Chloroflexi,* the phylum *Euryarchaeota,* the phylum WS3, the phylum *Nitrospirae,* the phylum WPS-2, and the phylum AD3 was significantly different between myocardial infarction patients and normal individuals (see Example 4).

More particularly, in one embodiment of the present invention, as a result of performing metagenomic analysis on bacteria-derived extracellular vesicles present in myocardial infarction patient-derived blood and normal individual-derived blood at a class level, the content of extracelluar vesicles derived from bacteria belonging to the class *Acidobacteriia,* the class DA052, the class *Methanomicrobia,* the class *Thaumarchaeota,* the class *Clostridia,* the class *Coriobacteriia,* the class *Betaproteobacteria,* the class *Ktedonobacteria,* the class *Planctomycetia,* the class *Solibacteres,* the class *Erysipelotrichi,* the class *Verrucomicrobiae,* the class TM7-3, the class *Bacteroidia,* the class *Phycisphaerae,* the class MCG, the class *Nitrospira,* the class *Pedosphaerae,* the class *Thermoleophilia,* the class *Saprospirae,* the class PRR-12, the class *Spartobacteria,* the class *Acidimicrobiia,* the class TM1, the class *Deltaproteobacteria,* the class *Anaerolineae,* the class *Thermoplasmata,* the class *Chthonomonadetes,* and the class *Acidobacteria*-6 was significantly different between myocardial infarction patients and normal individuals (see Example 4).

More particularly, in one embodiment of the present invention, as a result of performing metagenomic analysis on bacteria-derived extracellular vesicles present in myocardial infarction patient-derived blood and normal individual-derived blood at an order level, the content of extracelluar vesicles derived from bacteria belonging to the order *Lactobacillales,* the order *Acidobacteriales,* the order *Enterobacteriales,* the order *Xanthomonadales,* the order *Clostridiales,* the order *Coriobacteriales,* the order Ellin6513, the order *Burkholderiales,* the order *Erysipelotrichales,* the order *Solibacterales,* the order *Verrucomicrobiales,* the order *Rhodospirillales,* the order *Gemmatales,* the order *Thermogemmatisporales,* the order *Saprospirales,* the order *Acidimicrobiales,* the order *Pedosphaerales,* the order *Bifidobacteriales,* the order *Chthoniobacterales,* the order *Solirubrobacterales,* the order *Syntrophobacterales,* the order *Bacteroidales,* the order *Nitrospirales,* the order *Ktedonobacterales,* the order WD2101, the order iii1-15, the order Ellin329, the order *Thiotrichales,* the order *Myxococcales,* the order *Stramenopiles,* and the order *Vibrionales* was significantly different between myocardial infarction patients and normal individuals (see Example 4).

More particularly, in one embodiment of the present invention, as a result of performing metagenomic analysis on bacteria-derived extracellular vesicles present in myocardial infarction patient-derived blood and normal individual-derived blood at a family level, the content of extracelluar vesicles derived from bacteria belonging to the family *Koribacteraceae,* the family *Comamonadaceae,* the family *Enterobacteriaceae,* the family *Streptococcaceae,* the family *Coriobacteriaceae,* the family *Lachnospiraceae,* the family *Prevotellaceae,* the family *Ruminococcaceae,* the family *Xanthomonadaceae,* the family *Propionibacteriaceae,* the family *Hyphomicrobiaceae,* the family *Verrucomicrobiaceae,* the family *Solibacteraceae,* the family *Acidobacteriaceae,* the family *Erysipelotrichaceae,* the family *Ktedonobacteraceae,* the family *Thermogemmatisporaceae,* the family *Moraxellaceae,* the family *Veillonellaceae,* the family *Burkholderiaceae,* the family *Rhodospirillaceae,* the family *Bifidobacteriaceae,* the family *Gemmataceae,* the family *Streptomycetaceae,* the family *Chitinophagaceae,* the family *Brucellaceae,* the family *Rhizobiaceae,* the family *Chthoniobacteraceae,* the family *Sinobacteraceae,* the family *Conexibacteraceae,* the family *Oxalobacteraceae,* the family *Isosphaeraceae,* the family Ellin515, the family *Piscirickettsiaceae,* and the family *Methylocystaceae* was significantly different between myocardial infarction patients and normal individuals (see Example 4).

More particularly, in one embodiment of the present invention, as a result of performing metagenomic analysis on bacteria-derived extracellular vesicles present in myocardial infarction patient-derived blood and normal individual-derived blood at a genus level, the content of extracelluar vesicles derived from bacteria belonging to the genus *Delftia,* the genus *Agrobacterium,* the genus *Stenotrophomonas,* the genus *Faecalibacterium,* the genus *Candidatus Koribacter,* the genus *Akkermansia,* the genus *Streptococcus,* the genus *Salinispora,* the genus *Candidatus Solibacter,* the genus *Citrobacter,* the genus *Collinsella,* the genus *Burkholderia,* the genus *Coprococcus,* the genus *Rhodoplanes,* the genus *Acinetobacter,* the genus *Prevotella,* the genus *Propionibacterium,* the genus *Lactococcus,* the genus *Bifidobacterium,* the genus *Methanobacterium,* the genus *Micrococcus,* the genus *Methanocella,* the genus *Brevibacterium,* the genus *Streptacidiphilus,* the genus *Streptomyces,* the genus *Ochrobactrum,* the genus *Methanosaeta,* the genus *Lysinibacillus,* and the genus *Cupriavidus* was significantly different between myocardial infarction patients and normal individuals (see Example 4).

More particularly, in one embodiment of the present invention, as a result of performing metagenomic analysis on bacteria-derived extracellular vesicles present in cardiomyopathy patient-derived blood and normal individual-derived blood at a phylum level, the content of extracelluar vesicles derived from bacteria belonging to the phylum *Verrucomicrobia,* the phylum *Acidobacteria,* the phylum *Gemmatimonadetes,* and the phylum *Planctomycetes* was significantly different between cardiomyopathy patients and normal individuals (see Example 5).

More particularly, in one embodiment of the present invention, as a result of performing metagenomic analysis on bacteria-derived extracellular vesicles present in cardiomyopathy patient-derived blood and normal individual-derived blood at a class level, the content of extracelluar vesicles derived from bacteria belonging to the class *Verrucomicrobiae,* the class *Fusobacteriia,* the class *Acidobacteriia,* the class *Planctomycetia,* the class DA052, the class *Deltaproteobacteria,* and the class *Acidimicrobiia* was significantly different between cardiomyopathy patients and normal individuals (see Example 5).

More particularly, in one embodiment of the present invention, as a result of performing metagenomic analysis on bacteria-derived extracellular vesicles present in cardiomyopathy patient-derived blood and normal individual-derived blood at an order level, the content of extracelluar vesicles derived from bacteria belonging to the order *Pseudomonadales,* the order *Bacillales,* the order *Acidobacteriales,* the order *Sphingomonadales,* the order *Verrucomicrobiales,* the order *Turicibacterales,* the order *Acidimicrobiales,* the order Ellin6513, the order *Xanthomonadales,* and the order *Gemmatales* was significantly different between cardiomyopathy patients and normal individuals (see Example 5).

More particularly, in one embodiment of the present invention, as a result of performing metagenomic analysis on bacteria-derived extracellular vesicles present in cardiomyopathy patient-derived blood and normal individual-derived blood at a family level, the content of extracelluar vesicles derived from bacteria belonging to the family *Pseudomonadaceae,* the family *Clostridiaceae,* the family *Comamonadaceae,* the family *Oxalobacteraceae,* the family *Moraxellaceae,* the family *Verrucomicrobiaceae,* the family *Koribacteraceae,* the family *Sphingomonadaceae,* the family *Turicibacteraceae,* the family *Xanthomonadaceae,* the family *Gemmataceae,* and the family *Staphylococcaceae* was significantly different between cardiomyopathy patients and normal individuals (see Example 5).

More particularly, in one embodiment of the present invention, as a result of performing metagenomic analysis on bacteria-derived extracellular vesicles present in cardiomyopathy patient-derived blood and normal individual-derived blood at a genus level, the content of extracelluar vesicles derived from bacteria belonging to the genus *Pseudomonas,* the genus *Clostridium,* the genus *Cupriavidus,* the genus *Acinetobacter,* the genus *Citrobacter,* the genus *Sphingomonas,* the genus *Candidatus Koribacter,* the genus *Staphylococcus,* the genus *Thermoanaerobacterium,* the genus *Micrococcus,* the genus *Akkermansia,* the genus *Neisseria,* the genus *Enhydrobacter,* the genus *Actinomyces,* the genus *Turicibacter,* the genus *Phascolarctobacterium,* the genus *Lactococcus,* the genus *Delftia,* and the genus *Stenotrophomonas* was significantly different between cardiomyopathy patients and normal individuals (see Example 5).

More particularly, in one embodiment of the present invention, as a result of performing metagenomic analysis on bacteria-derived extracellular vesicles present in variant angina patient-derived blood and normal individual-derived blood at a phylum level, the content of extracelluar vesicles derived from bacteria belonging to the phylum *Verrucomicrobia,* the phylum *Acidobacteria,* the phylum *Planctomycetes,* the phylum *Gemmatimonadetes,* the phylum *Chloroflexi,* and the phylum *Euryarchaeota* was significantly different between variant angina patients and normal individuals (see Example 6).

More particularly, in one embodiment of the present invention, as a result of performing metagenomic analysis on bacteria-derived extracellular vesicles present in variant angina patient-derived blood and normal individual-derived blood at a class level, the content of extracelluar vesicles derived from bacteria belonging to the class *Verrucomicrobiae,* the class *Acidobacteriia,* the class *Fimbriimonadia,* the class *Erysipelotrichi,* the class *Ktedonobacteria,* and the class *Deltaproteobacteria* was significantly different between variant angina patients and normal individuals (see Example 6).

More particularly, in one embodiment of the present invention, as a result of performing metagenomic analysis on bacteria-derived extracellular vesicles present in variant angina patient-derived blood and normal individual-derived blood at an order level, the content of extracelluar vesicles derived from bacteria belonging to the order *Pseudomonadales,* the order *Erysipelotrichales,* the order *Fimbriimonadales,* the order *Acidobacteriales,* the order *Verrucomicrobiales,* the order *Xanthomonadales,* the order *Myxococcales,* the order *Deinococcales,* and the order *Rhodospirillales* was significantly different between variant angina patients and normal individuals (see Example 6).

More particularly, in one embodiment of the present invention, as a result of performing metagenomic analysis on bacteria-derived extracellular vesicles present in variant angina patient-derived blood and normal individual-derived blood at a family level, the content of extracelluar vesicles derived from bacteria belonging to the family *Koribacteraceae,* the family *Oxalobacteraceae,* the family *Comamonadaceae,* the family *Moraxellaceae,* the family *Pseudomonadaceae,* the family *Hyphomicrobiaceae,* the family *Erysipelotrichaceae,* the family *Deinococcaceae,* the family *Clostridiaceae,* the family *Verrucomicrobiaceae,* the family *Sinobacteraceae,* the family *Rhodospirillaceae,* the family *Methylobacteriaceae,* the family *Aerococcaceae,* the family *Fusobacteriaceae,* the family *Fimbriimonadaceae,* the family *Bacillaceae,* and the family *Planococcaceae* was significantly different between variant angina patients and normal individuals (see Example 6).

More particularly, in one embodiment of the present invention, as a result of performing metagenomic analysis on bacteria-derived extracellular vesicles present in variant angina patient-derived blood and normal individual-derived blood at a genus level, the content of extracelluar vesicles derived from bacteria belonging to the genus *Citrobacter,* the genus *Acinetobacter,* the genus *Cupriavidus,* the genus *Clostridium,* the genus *Catenibacterium,* the genus *Pseudomonas,* the genus *Lactococcus,* the genus *Stenotrophomonas,* the genus *Akkermansia,* the genus *Bacillus,* the genus *Delftia,* the genus *Agrobacterium,* the genus *Deinococcus,* the genus *Fusobacterium,* and the genus *Adlercreutzia* was significantly different between variant angina patients and normal individuals (see Example 6).

More particularly, in one embodiment of the present invention, as a result of performing metagenomic analysis on bacteria-derived extracellular vesicles present in atrial fibrillation patient-derived blood and normal individual-derived blood at a phylum level, the content of extracelluar vesicles derived from bacteria belonging to the phylum *Proteobacteria,* the phylum TM7, the phylum *Chloroflexi,* the phylum *Acidobacteria,* and the phylum *Cyanobacteria* was significantly different between atrial fibrillation patients and normal individuals (see Example 7).

More particularly, in one embodiment of the present invention, as a result of performing metagenomic analysis on bacteria-derived extracellular vesicles present in atrial fibrillation patient-derived blood and normal individual-derived blood at a class level, the content of extracelluar vesicles derived from bacteria belonging to the class *Clostridia,* the class *Bacteroidia,* the class *Actinobacteria,* the class *Flavobacteriia,* the class *Erysipelotrichi,* the class TM7-3, and the class *Chloroplast* was significantly different between atrial fibrillation patients and normal individuals (see Example 7).

More particularly, in one embodiment of the present invention, as a result of performing metagenomic analysis on bacteria-derived extracellular vesicles present in atrial fibrillation patient-derived blood and normal individual-derived blood at an order level, the content of extracelluar vesicles derived from bacteria belonging to the order *Pseudomonadales,* the order *Clostridiales,* the order *Bacteroidales,* the order *Enterobacteriales,* the order *Xanthomonadales,* the order *Bifidobacteriales,* the order *Pasteurellales,* the order *Flavobacteriales,* the order *Actinomycetales,* the order *Rhodobacterales,* the order *Coriobacteriales,* the order *Erysipelotrichales,* and the order *Streptophyta* was significantly different between atrial fibrillation patients and normal individuals (see Example 7).

More particularly, in one embodiment of the present invention, as a result of performing metagenomic analysis on bacteria-derived extracellular vesicles present in atrial fibrillation patient-derived blood and normal individual-derived blood at a family level, the content of extracelluar vesicles derived from bacteria belonging to the family *Lachnospiraceae,* the family *Bacillaceae,* the family *Streptococcaceae,* the family *Bacteroidaceae,* the family *Moraxellaceae,* the family *Ruminococcaceae,* the family *Weeksellaceae,* the family *Bifidobacteriaceae,* the family *Clostridiaceae,* the family *Desulfovibrionaceae,* the family *Veillonellaceae,* the family *Coriobacteriaceae,* the family *Flavobacteriaceae,* the family *Rikenellaceae,* the family S24-7, the family *Pasteurellaceae,* the family *Rhodobacteraceae,* the family *Pseudomonadaceae,* the family *Gordoniaceae,* and the family *Enterococcaceae* was significantly different between atrial fibrillation patients and normal individuals (see Example 7).

More particularly, in one embodiment of the present invention, as a result of performing metagenomic analysis on bacteria-derived extracellular vesicles present in atrial fibrillation patient-derived blood and normal individual-derived blood at a genus level, the content of extracelluar vesicles derived from bacteria belonging to the genus *Acinetobacter,* the genus *Stenotrophomonas,* the genus *Chryseobacterium,* the genus *Enterococcus,* the genus *Pseudomonas,* the genus *Delftia,* the genus *Alcanivorax,* the genus *Psychrobacter,* the genus *Streptococcus,* the genus *Ochrobactrum,* the genus *Bifidobacterium,* the genus *Coprococcus,* the genus *Bacteroides,* the genus *Faecalibacterium,* the genus *Enhydrobacter,* the genus *Agrobacterium,* the genus *Citrobacter,* the genus *Prevotella,* the genus *Geobacillus,* the genus *Clostridium,* the genus *Bacillus,* the genus *Haemophilus,* the genus *Veillonella,* the genus *Actinomyces,* the genus *Paracoccus,* the genus *Kocuria,* the genus *Halomonas,* the genus *Micrococcus,* the genus *Ruminococcus,* and the genus *Porphyromonas* was significantly different between atrial fibrillation patients and normal individuals (see Example 7).

From the above-described example results, it was confirmed that bacteria-derived extracellular vesicles exhibiting a significant change in content in patients with myocardial infarction, cardiomyopathy, variant angina, or atrial fibrillation compared to normal individuals, were identified by performing metagenomic analysis on extracellular vesicles isolated from blood, and heart disease may be diagnosed by analyzing an increase or decrease in the content of bacteria-derived vesicles at each level through metagenomic analysis.

Hereinafter, the present invention will be described with reference to exemplary examples to aid in understanding of the present invention. However, these examples are provided only for illustrative purposes and are not intended to limit the scope of the present invention.

### [Examples]

### Example 1. Analysis of In Vivo Absorption, Distribution, and Excretion Patterns of Intestinal Bacteria and Bacteria-Derived Extracellular Vesicles

To evaluate whether intestinal bacteria and bacteria-derived extracellular vesicles are systematically absorbed through the gastrointestinal tract, an experiment was conducted using the following method. More particularly, 50 µg of each of *Pseudomonas panacis,* which is an intestinal bacterium, and the bacteria-derived extracellular vesicles (EVs), labeled with fluorescence, were orally administered to the gastrointestinal tracts of mice, and fluorescence was measured at 0 h, and after 5 min, 3 h, 6 h, and 12 h.

As a result of observing the entire images of mice, as illustrated in FIG. 1A, the bacteria were not systematically absorbed when the bacteria was administered, while the bacteria-derived EVs were systematically absorbed at 5 min after administration, and, at 30 minutes after administration, fluorescence was strongly observed in the bladder, from which it was confirmed that the EVs were excreted via the urinary system through blood, and were present in the bodies up to 12 h after administration.

In addition to the above results, after intestinal bacteria-derived extracellular vesicles were systematically absorbed, to evaluate a pattern of invasion of the intestinal bacteria-derived EVs into various organs, 50 µg of each of the bacteria and bacteria-derived EVs, labeled with fluorescence, were administered using the same method as that used above, and then, at 12 h after administration, blood, the heart, the lungs, the liver, the kidneys, the spleen, adipose tissue, and muscle were extracted from each mouse. As a result of observing fluorescence in the extracted tissues, as illustrated in FIG. 1B, it was confirmed that the intestinal bacteria were not absorbed into each organ, while the intestinal bacteria-derived EVs were distributed in the blood, heart, lungs, liver, kidneys, spleen, adipose tissue, and muscle.

### Example 2. Vesicle Isolation and DNA Extraction from Blood

First, blood was added to a 10 ml tube and centrifuged at 3,500 x g and 4 □ for 10 min to precipitate a suspension, and only a supernatant was then placed in a new 10 ml tube. Bacteria and impurities were removed using a 0.22 µm filter, and then placed in centripreigugal filters (50 kD) and centrifuged at 1500 x g and 4□ for 15 min to discard materials with a smaller size than 50 kD, and then concentrated to 10 ml. Once again, bacteria and impurities were removed therefrom using a 0.22 µm filter, and then the resulting concentrate was subjected to ultra-high speed centrifugation at 150,000 x g and 4 □ to remove a supernatant, and the agglomerated pellet was dissolved with phosphate-buffered saline (PBS).

100 µl of the vesicles isolated according to the above-described method was boiled at 100 □ to allow the internal DNA to come out of the lipid and then cooled on ice for 5 minutes. Next, the resulting vesicles were centrifuged at 10,000 x g and 4 □ for 30 minutes to remove the remaining suspension, only the supernatant was collected, and then the amount of DNA extracted was quantified using a NanoDrop sprectrophotometer. In addition, to verify whether bacteria-derived DNA was present in the extracted DNA, PCR was performed using 16s rDNA primers shown in Table 1 below to verify whether bacteria-derived DNA was present in the extracted DNA.

**[Table 1]**

| primer | | sequence | SEQ ID NO. |
|---|---|---|---|
| 16S rDNA | 16S_V3_F | 5'-TCGTCGGCAGCGTCAGATGTGTATAAG AGACAGCCTACGGGNGGCWGCAG-3' | 1 |
| | 16S_V4_R | 5'-GTCTCGTGGGCTCGGAGATGTGTATAA GAGACAGGACTACHVGGGTATCTAATCC -3 | 2 |

### Example 3. 16S rDNA Sequencing Using Extracelluar Vesicle-Derived DNA Isolated from Blood

DNA was extracted using the same method as that used in Example 2, and then PCR was performed thereon using 16S rDNA primers shown in Table 1 to amplify DNA, followed by sequencing (Illumina MiSeq sequencer). The results were output as standard flowgram format (SFF) files, and the SFF files were converted into sequence files (.fasta) and nucleotide quality score files using GS FLX software (v2.9), and then credit rating for reads was identified, and portions with a window (20 bps) average base call accuracy of less than 99% (Phred score <20) were removed. After removing the low-quality portions, only reads having a length of 300 bps or more were used (Sickle version 1.33), and, for operational taxonomy unit (OTU) analysis, clustering was performed using UCLUST and USEARCH according to sequence similarity. In particular, clustering was performed based on sequence similarity values of 94% for genus, 90% for family, 85% for order, 80% for class, and 75% for phylum, and phylum, class, order, family, and genus levels of each OTU were classified, and bacteria with a sequence similarity of 97% or more were analyzed (QIIME) using 16S RNA sequence databases (108,453 sequences) of BLASTN and GreenGenes.

### Example 4. Myocardial infarction Diagnostic Model Based on Metagenomic Profiling of Bacteria-Derived EVs

EVs were isolated from blood samples of 57 ST elevation myocardial infarction (STEMI) patients and 163 normal individuals, the two groups matched in age and gender, and then metagenomic sequencing was performed thereon using the method of Example 3. For the development of a diagnostic model, first, a strain exhibiting a p value of less than 0.05 between two groups in a t-test and a difference of two-fold or more between two groups was selected, and then an area under curve (AUC), accuracy, sensitivity, and specificity, which are diagnostic performance indexes, were calculated by logistic regression analysis.

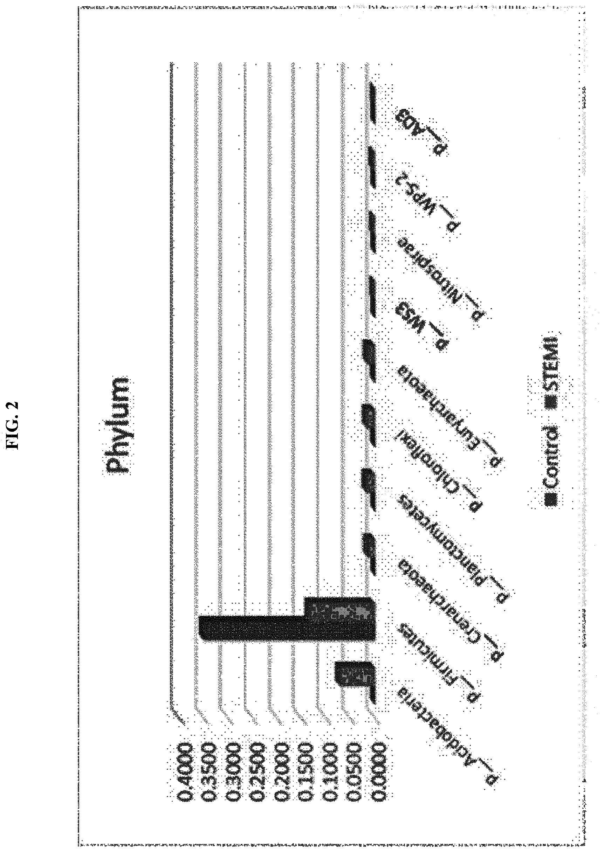

As a result of analyzing bacteria-derived EVs in blood at a phylum level, a diagnostic model developed using, as a biomarker, one or more bacteria from the phylum *Acidobacteria,* the phylum *Firmicutes,* the phylum *Crenarchaeota,* the phylum *Planctomycetes,* the phylum *Chloroflexi,* the phylum *Euryarchaeota,* the phylum WS3, the phylum *Nitrospirae,* the phylum WPS-2, and the phylum AD3 exhibited significant diagnostic performance for STEMI (see Table 2 and FIG. 2).

**[Table 2]**

| | Control | | STEMI | | t-tes t | | Training Set | | | | Test Set | | | |
|---|---|---|---|---|---|---|---|---|---|---|---|---|---|---|
| Taxon | Mea n | SD | Mea n | SD | p-va lue | Ratio | AU C | Acu rrac y | Sens itivit y | Spe cific ity | AU C | Acu rrac y | Sens itivit y | s p e ci fi ci ty |
| p_Firm icutes | 0.35 15 | 0.10 50 | 0.13 54 | 0.06 52 | 0.00 00 | 0.39 | 0.97 | 0.93 | 0.95 | 0.88 | 0.94 | 0.85 | 0.86 | 0. 8 1 |
| p_Eury archaeot a | 0.00 13 | 0.00 32 | 0.01 60 | 0.01 79 | 0.00 00 | 11.95 | 0.81 | 0.87 | 0.98 | 0.56 | 0.77 | 0.85 | 0.96 | 0. 5 0 |
| p_Chlo roflexi | 0.00 10 | 0.00 36 | 0.01 72 | 0.01 69 | 0.00 00 | 16.82 | 0.84 | 0.87 | 0.98 | 0.56 | 0.86 | 0.91 | 0.98 | 0. 6 9 |
| p_WP S-2 | 0.00 02 | 0.00 18 | 0.00 42 | 0.00 80 | 0.00 05 | 20.81 | 0.69 | 0.79 | 0.99 | 0.24 | 0.77 | 0.83 | 0.96 | 0. 4 4 |
| p_AD3 | 0.00 00 | 0.00 03 | 0.00 16 | 0.00 42 | 0.00 72 | 35.51 | 0.67 | 0.77 | 1.00 | 0.15 | 0.58 | 0.79 | 1.00 | 0. 1 3 |
| p_Plan ctomyce tes | 0.00 04 | 0.00 17 | 0.01 78 | 0.01 73 | 0.00 00 | 44.17 | 0.90 | 0.91 | 0.97 | 0.73 | 0.92 | 0.89 | 0.96 | 0. 6 9 |
| p_Acid obacteri a | 0.00 09 | 0.00 27 | 0.07 21 | 0.04 02 | 0.00 00 | 77.14 | 0.99 | 0.97 | 0.99 | 0.93 | 1.00 | 0.98 | 1.00 | 0. 9 4 |
| p_Nitr ospirae | 0.00 00 | 0.00 04 | 0.00 34 | 0.00 69 | 0.00 06 | 94.81 | 0.72 | 0.81 | 0.99 | 0.32 | 0.60 | 0.79 | 1.00 | 0. 1 3 |
| p_Cren archaeot a | 0.00 00 | 0.00 00 | 0.01 50 | 0.02 08 | 0.00 00 | >100 | 0.92 | 0.95 | 1.00 | 0.78 | 0.92 | 0.92 | 1.00 | 0. 7 5 |
| p_WS3 | 0.00 00 | 0.00 00 | 0.00 26 | 0.00 63 | 0.00 00 | >100 | 0.72 | 0.85 | 1.00 | 0.38 | 0.65 | 0.76 | 1.00 | 0. 2 0 |

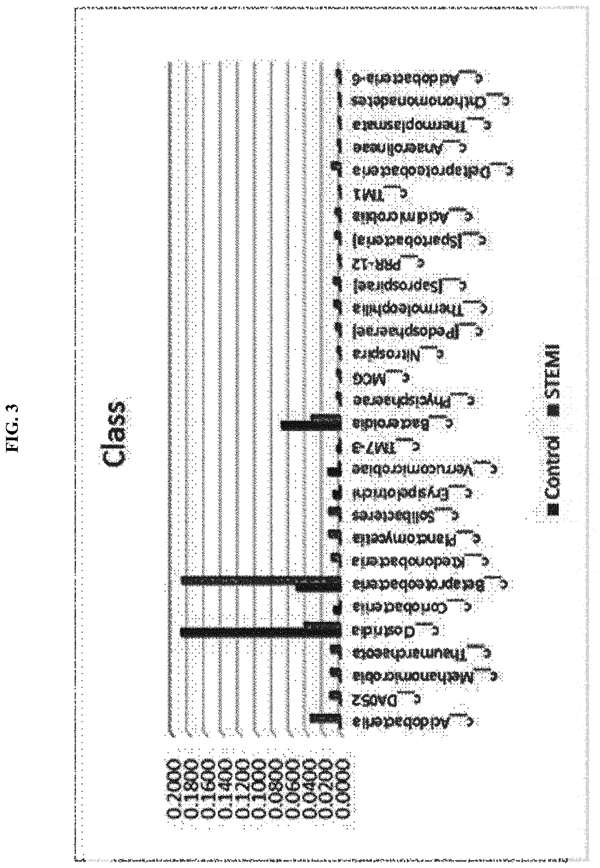

As a result of analyzing bacteria-derived EVs in blood at a class level, a diagnostic model developed using, as a biomarker, one or more bacteria from the class *Acidobacteriia,* the class DA052, the class *Methanomicrobia,* the class *Thaumarchaeota,* the class *Clostridia,* the class *Coriobacteriia,* the class *Betaproteobacteria,* the class *Ktedonobacteria,* the class *Planctomycetia,* the class *Solžbacteres,* the class *Erysipelotrichi,* the class *Verrucomicrobiae,* the class TM7-3, the class *Bacteroidia,* the class *Phycisphaerae,* the class MCG, the class *Nitrospira,* the class *Pedosphaerae,* the class *Thermoleophilia,* the class *Saprospirae,* the class PRR-12, the class *Spartobacteria,* the class *Acidimicrobiia,* the class TM1, the class *Deltaproteobacteria,* the class *Anaerolineae,* the class *Thermoplasmata,* the class *Chthonomonadetes,* and the class *Acidobacteria*-6 exhibited significant diagnostic performance for STEMI (see Table 3 and FIG. 3).

**[Table 3]**

| | Control | | STEMI | | t-tes t | | Training Set | | | | Test Set | | | |
|---|---|---|---|---|---|---|---|---|---|---|---|---|---|---|
| Taxon | Mea n | SD | Mea n | SD | p-va lue | Ratio | AU C | Acu rrac y | Sen sitiv ity | Spe cific ity | AU C | Acu rrac y | Sen sitiv ity | s p e c i f i c it y |
| c_Corioba cteriia | 0.00 75 | 0.00 91 | 0.00 01 | 0.00 03 | 0.00 00 | 0.01 | 0.90 | 0.81 | 0.80 | 0.81 | 0.86 | 0.86 | 0.88 | 0 .8 0 |
| c_Erysipel otricbi | 0.00 82 | 0.01 28 | 0.00 06 | 0.00 21 | 0.00 00 | 0.07 | 0.82 | 0.76 | 0.96 | 0.24 | 0.87 | 0.79 | 0.96 | 0 .2 0 |
| c_Verruco microbiae | 0.01 43 | 0.01 81 | 0.00 17 | 0.00 41 | 0.00 00 | 0.12 | 0.81 | 0.77 | 0.81 | 0.64 | 0.90 | 0.82 | 0.86 | 0 .6 7 |
| c_TM7-3 | 0.00 37 | 0.00 71 | 0.00 06 | 0.00 25 | 0.00 00 | 0.16 | 0.81 | 0.73 | 1.00 | 0.00 | 0.68 | 0.77 | 1.00 | 0 .0 0 |
| c_Clostridi a | 0.18 85 | 0.11 43 | 0.04 25 | 0.03 13 | 0.00 00 | 0.23 | 0.91 | 0.85 | 0.89 | 0.80 | 0.94 | 0.86 | 0.86 | 0 . 8 7 |
| c_Bacteroi dia | 0.06 95 | 0.04 88 | 0.03 43 | 0.02 44 | 0.00 00 | 0.49 | 0.79 | 0.77 | 0.92 | 0.36 | 0.67 | 0.74 | 0.88 | 0 .2 7 |
| c_Betaprot eobacteria | 0.05 23 | 0.04 22 | 0.18 74 | 0.14 71 | 0.00 00 | 3.58 | 0.88 | 0.82 | 0.95 | 0.50 | 0.94 | 0.86 | 1.00 | 0 .4 0 |
| c_Deltapro teobacteria | 0.00 11 | 0.00 23 | 0.01 04 | 0.01 49 | 0.00 00 | 9.60 | 0.67 | 0.81 | 0.96 | 0.40 | 0.84 | 0.86 | 0.98 | 0 .4 7 |
| c_[Saprosp irae] | 0.00 06 | 0.00 23 | 0.00 79 | 0.01 66 | 0.00 19 | 12.6 0 | 0.73 | 0.82 | 0.97 | 0.43 | 0.75 | 0.83 | 0.94 | 0 .4 7 |
| c_Thermol eophilia | 0.00 05 | 0.00 35 | 0.00 74 | 0.01 05 | 0.00 00 | 13.9 7 | 0.75 | 0.80 | 0.98 | 0.31 | 0.87 | 0.86 | 1.00 | 0 .4 0 |
| c_Acidimi crobiia | 0.00 03 | 0.00 13 | 0.00 56 | 0.00 91 | 0.00 00 | 20.0 3 | 0.70 | 0.82 | 0.97 | 0.40 | 0.73 | 0.82 | 1.00 | 0 .2 0 |
| c_Acidoba cteria-6 | 0.00 02 | 0.00 14 | 0.00 45 | 0.00 82 | 0.00 03 | 25.3 8 | 0.62 | 0.78 | 0.99 | 0.21 | 0.72 | 0.85 | 1.00 | 0 .33 |
| c_Plancto mycetia | 0.00 03 | 0.00 15 | 0.01 36 | 0.01 54 | 0.00 00 | 41.2 5 | 0.87 | 0.88 | 0.97 | 0.64 | 0.93 | 0.88 | 0.96 | 0 .6 0 |
| c_[Spartob acteria] | 0.00 01 | 0.00 09 | 0.00 62 | 0.01 16 | 0.00 03 | 49.8 9 | 0.72 | 0.81 | 0.98 | 0.33 | 0.86 | 0.86 | 1.00 | 0 .4 0 |
| c_Anaeroli neae | 0.00 01 | 0.00 05 | 0.00 30 | 0.00 65 | 0.00 15 | 51.9 6 | 0.66 | 0.78 | 1.00 | 0.19 | 0.87 | 0.82 | 0.98 | 0 .2 7 |
| c_[Pedosp haerae] | 0.00 01 | 0.00 06 | 0.00 55 | 0.00 84 | 0.00 00 | 56.3 1 | 0.75 | 0.83 | 0.98 | 0.43 | 0.84 | 0.86 | 1.00 | 0 .4 0 |
| c_Phycisp haerae | 0.00 01 | 0.00 06 | 0.00 42 | 0.00 87 | 0.00 08 | 56.6 3 | 0.78 | 0.82 | 0.99 | 0.36 | 0.75 | 0.79 | 0.96 | 0 .2 0 |
| c_Solibact eres | 0.00 02 | 0.00 16 | 0.01 35 | 0.01 55 | 0.00 00 | 56.6 4 | 0.86 | 0.87 | 0.99 | 0.55 | 0.69 | 0.89 | 1.00 | 0 .5 3 |
| c_Nitrospir a | 0.00 00 | 0.00 04 | 0.00 34 | 0.00 69 | 0.00 06 | 94.8 1 | 0.75 | 0.79 | 0.99 | 0.24 | 0.78 | 0.85 | 1.00 | 0 .3 3 |
| | | | | | | | | | | | | | | |
| c_Ktedono bacteria | 0.00 00 | 0.00 03 | 0.01 05 | 0.01 19 | 0.00 00 | 312. 66 | 0.87 | 0.89 | 0.99 | 0.62 | 0.94 | 0.91 | 1.00 | 0 .6 0 |
| c_Acidoba cteriia | 0.00 01 | 0.00 05 | 0.03 54 | 0.02 16 | 0.00 00 | 415. 08 | 0.97 | 0.97 | 1.00 | 0.88 | 1.00 | 0.98 | 1.00 | 0 .9 3 |
| c_DA052 | 0.00 00 | 0.00 01 | 0.01 21 | 0.01 37 | 0.00 00 | 1040 .81 | 0.96 | 0.95 | 0.99 | 0.86 | 0.94 | 0.91 | 0.92 | 0 .8 7 |
| c_Methano microbia | 0.00 00 | 0.00 00 | 0.01 13 | 0.01 45 | 0.00 00 | >100 | 0.93 | 0.94 | 1.00 | 0.79 | 0.99 | 0.97 | 1.00 | 0 .8 7 |
| c_Thaumar chaeota | 0.00 00 | 0.00 00 | 0.01 10 | 0.01 68 | 0.00 00 | >100 | 0.91 | 0.93 | 1.00 | 0.74 | 0.90 | 0.94 | 1.00 | 0 .7 3 |
| c_MCG | 0.00 00 | 0.00 00 | 0.00 39 | 0.01 18 | 0.00 00 | >100 | 0.76 | 0.83 | 1.00 | 0.38 | 0.72 | 0.89 | 1.00 | 0 .5 3 |
| c_PRR-12 | 0.00 00 | 0.00 00 | 0.00 26 | 0.00 63 | 0.00 00 | >100 | 0.72 | 0.81 | 1.00 | 0.29 | 0.63 | 0.86 | 1.00 | 0 .4 0 |
| c_TM1 | 0.00 00 | 0.00 00 | 0.00 18 | 0.00 46 | 0.00 00 | >100 | 0.69 | 0.81 | 1.00 | 0.29 | 0.62 | 0.85 | 1.00 | 0 .3 3 |
| c_Thcrmo plasmata | 0.00 00 | 0.00 00 | 0.00 23 | 0.00 90 | 0.00 14 | >100 | 0.66 | 0.79 | 1.00 | 0.21 | 0.57 | 0.85 | 1.00 | 0 .3 3 |
| c_Chthono monadetes | 0.00 00 | 0.00 00 | 0.00 18 | 0.00 62 | 0.00 03 | >100 | 0.65 | 0.77 | 1.00 | 0.14 | 0.55 | 0.82 | 1.00 | 0 .2 0 |

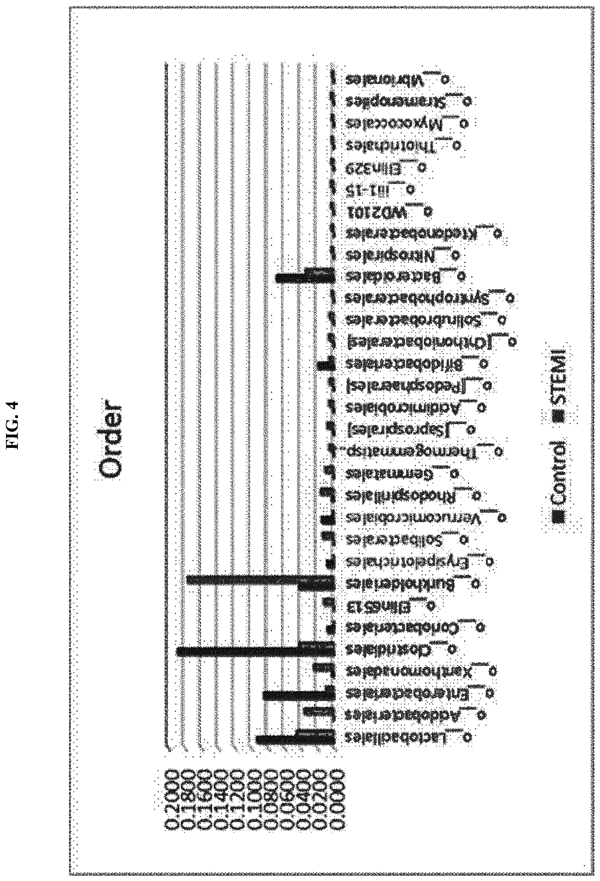

As a result of analyzing bacteria-derived EVs in blood at an order level, a diagnostic model developed using, as a biomarker, one or more bacteria from the order *Lactobacillales,* the order *Acidobacteriales,* the order *Enterobacteriales,* the order *Xanthomonadales,* the order *Clostridiales,* the order *Coriobacteriales,* the order Ellin6513, the order *Burkholderiales,* the order *Erysipelotrichales,* the order *Solibacterales,* the order *Verrucomicrobiales,* the order *Rhodospirillales,* the order *Gemmatales,* the order *Thermogemmatisporales,* the order *Saprospirales,* the order *Acidimicrobiales,* the order *Pedosphaerales,* the order *Bifidobacteriales,* the order *Chthoniobacterales,* the order *Solirubrobacterales,* the order *Syntrophobacterales,* the order *Bacteroidales,* the order *Nitrospirales,* the order *Ktedonobacterales,* the order WD2101, the order iii1-15, the order Ellin329, the order *Thiotrichales,* the order *Myxococcales,* the order *Stramenopiles,* and the order *Vibrionales* exhibited significant diagnostic performance for STEMI (see Table 4 and FIG. 4).

**[Table 4]**

| | Control | | STEMI | | t-tes t | | Training Set | | | | Test Set | | | |
|---|---|---|---|---|---|---|---|---|---|---|---|---|---|---|
| Taxon | Mea n | SD | Mea n | SD | p-va lue | Rati o | AU C | Acu rrac y | Sensi tivity | Speci ficity | A UC | Acu rrac y | Sensi tivity | e c i f i c i t |
| o_Corioba cteriales | 0.00 75 | 0.00 91 | 0.00 01 | 0.00 03 | 0.00 00 | 0.01 | 0.9 0 | 0.82 | 0.83 | 0.77 | 0.8 0 | 0.79 | 0.73 | . |
| o_Erysipel otrichales | 0.00 82 | 0.01 28 | 0.00 06 | 0.00 21 | 0.00 00 | 0.07 | 0.8 7 | 0.79 | 0.81 | 0.72 | 0.7 7 | 0.77 | 0.81 | . |
| o_Enterob acteriales | 0.08 48 | 0.06 18 | 0.00 97 | 0.01 54 | 0.00 00 | 0.11 | 0.9 4 | 0.86 | 0.89 | 0.79 | 0.9 3 | 0.82 | 0.81 | . |
| o_Verruco microbiales | 0.01 43 | 0.01 81 | 0.00 17 | 0.00 41 | 0.00 00 | 0.12 | 0.8 5 | 0.77 | 0.84 | 0.54 | 0.7 8 | 0.80 | 0.81 | . |
| o_Clostridi ales | 0.18 82 | 0.11 42 | 0.04 20 | 0.03 15 | 0.00 00 | 0.22 | 0.9 3 | 0.84 | 0.88 | 0.74 | 0.8 8 | 0.82 | 0.85 | . |
| o_Bifidoba cteriales | 0.01 89 | 0.02 79 | 0.00 57 | 0.00 85 | 0.00 00 | 0.30 | 0.7 7 | 0.81 | 0.97 | 0.33 | 0.7 1 | 0.64 | 0.83 | . |
| o_Lactoba cillales | 0.09 33 | 0.05 52 | 0.04 51 | 0.03 29 | 0.00 00 | 0.48 | 0.9 8 | 0.80 | 0.93 | 0.41 | 0.8 1 | 0.79 | 0.88 | . |
| o_Bacteroi dales | 0.06 95 | 0.04 88 | 0.03 43 | 0.02 44 | 0.00 00 | 0.49 | 0.7 5 | 0.73 | 0.91 | 0.21 | 0.7 5 | 0.74 | 0.96 | . |
| o_Burkhol deriales | 0.04 21 | 0.04 20 | 0.17 64 | 0.14 68 | 0.00 00 | 4.19 | 0.8 7 | 0.80 | 0.95 | 0.38 | 0.9 8 | 0.89 | 1.00 | . |
| o_Rhodosp irillales | 0.00 28 | 0.00 70 | 0.01 59 | 0.01 70 | 0.00 00 | 5.73 | 0.8 5 | 0.81 | 0.96 | 0.38 | 0.7 1 | 0.77 | 0.92 | . |
| o_Xantho monadales | 0.00 23 | 0.00 47 | 0.02 44 | 0.01 94 | 0.00 00 | 10.4 6 | 0.9 3 | 0.87 | 0.97 | 0.59 | 0.9 1 | 0.91 | 0.98 | . |
| o_Vibriona les | 0.00 02 | 0.00 09 | 0.00 23 | 0.00 56 | 0.00 70 | 11.0 5 | 0.6 0 | 0.78 | 0.99 | 0.15 | 0.5 0 | 0.76 | 0.98 | . |
| ο_Myxoco ccales | 0.00 02 | 0.00 10 | 0.00 27 | 0.00 52 | 0.00 09 | 12.0 7 | 0.6 4 | 0.81 | 0.98 | 0.31 | 0.6 2 | 0.79 | 1.00 | . |
| o_[Saprosp irales] | 0.00 06 | 0.00 23 | 0.00 79 | 0.01 66 | 0.00 19 | 12.6 0 | 0.8 0 | 0.85 | 0.98 | 0.46 | 0.6 2 | 0.74 | 0.88 | . |
| o_Solirubr obacterales | 0.00 04 | 0.00 34 | 0.00 55 | 0.00 85 | 0.00 01 | 13.7 7 | 0.7 6 | 0.86 | 1.00 | 0.44 | 0.6 2 | 0.77 | 0.92 | . |
| o_Acidimi crobiales | 0.00 03 | 0.00 13 | 0.00 56 | 0.00 91 | 0.00 00 | 20.0 3 | 0.7 9 | 0.87 | 0.98 | 0.54 | 0.4 8 | 0.74 | 0.94 | . |
| o_Stramen opiles | 0.00 02 | 0.00 16 | 0.00 41 | 0.01 08 | 0.00 93 | 22.2 9 | 0.6 2 | 0.77 | 0.98 | 0.15 | 0.6 1 | 0.79 | 1.00 | . |
| o_iiil-15 | 0.00 02 | 0.00 14 | 0.00 40 | 0.00 81 | 0.00 09 | 22.7 1 | 0.6 8 | 0.79 | 0.99 | 0.21 | 0.5 7 | 0.77 | 1.00 | . |
| o_[Chthoni obacterales] | 0.00 01 | 0.00 09 | 0.00 62 | 0.01 16 | 0.00 03 | 49.8 9 | 0.7 7 | 0.84 | 0.98 | 0.41 | 0.6 2 | 0.80 | 1.00 | . |
| o_Gemmat ales | 0.00 02 | 0.00 12 | 0.01 05 | 0.01 30 | 0.00 00 | 50.2 4 | 0.8 3 | 0.86 | 0.97 | 0.51 | 0.7 7 | 0.89 | 1.00 | . |
| o_[Pedosp haerales] | 0.00 01 | 0.00 06 | 0.00 55 | 0.00 84 | 0.00 00 | 56.3 1 | 0.7 9 | 0.85 | 0.97 | 0.49 | 0.7 2 | 0.80 | 1.00 | . |
| o_Solibact erales | 0.00 02 | 0.00 16 | 0.01 35 | 0.01 55 | 0.00 00 | 56.6 4 | 0.8 6 | 0.90 | 1.00 | 0.59 | 0.7 3 | 0.85 | 0.98 | . |
| o_Nitrospi rales | 0.00 00 | 0.00 04 | 0.00 34 | 0.00 69 | 0.00 06 | 94.8 1 | 0.7 4 | 0.83 | 1.00 | 0.33 | 0.5 7 | 0.76 | 0.98 | . |
| o_WD210 1 | 0.00 00 | 0.00 04 | 0.00 35 | 0.00 82 | 0.00 28 | 100. 30 | 0.6 9 | 0.81 | 0.99 | 0.28 | 0.5 0 | 0.76 | 1.00 | . |
| o_Ellin329 | 0.00 00 | 0.00 04 | 0.00 33 | 0.00 87 | 0.00 70 | 100. 55 | 0.6 7 | 0.79 | 0.99 | 0.21 | 0.5 3 | 0.79 | 1.00 | . |
| o_Ktedono bacterales | 0.00 00 | 0.00 02 | 0.00 34 | 0.00 69 | 0.00 05 | 174. 45 | 0.7 1 | 0.81 | 0.99 | 0.28 | 0.5 6 | 0.77 | 1.00 | . |
| o_Thermo gemmatispo rales | 0.00 00 | 0.00 02 | 0.00 57 | 0.00 82 | 0.00 00 | 407. 10 | 0.8 2 | 0.89 | 0.99 | 0.59 | 0.7 5 | 0.77 | 1.00 | . |
| o_Acidoba | 0.00 | 0.00 | 0.03 | 0.02 | 0.00 | 415. | 0.9 | 0.97 | 1.00 | 0.87 | 1.0 | 0.98 | 1.00 | . |
| cteriales | 01 | 05 | 54 | 16 | 00 | 08 | 6 | | | | 0 | | | |
| o_Thiotric hales | 0.00 00 | 0.00 00 | 0.00 30 | 0.00 64 | 0.00 08 | 652. 57 | 0.6 7 | 0.79 | 1.00 | 0.18 | 0.5 5 | 0.80 | 1.00 | . |
| o_Ellin651 3 | 0.00 00 | 0.00 01 | 0.01 21 | 0.01 37 | 0.00 00 | 104 0.81 | 0.8 8 | 0.93 | 1.00 | 0.72 | 0.7 6 | 0.89 | 1.00 | . |
| o_Syntrop hobacterales | 0.00 00 | 0.00 00 | 0.00 29 | 0.00 66 | 0.00 00 | >10 0 | 0.7 6 | 0.86 | 1.00 | 0.43 | 0.6 4 | 0.77 | 1.00 | . |

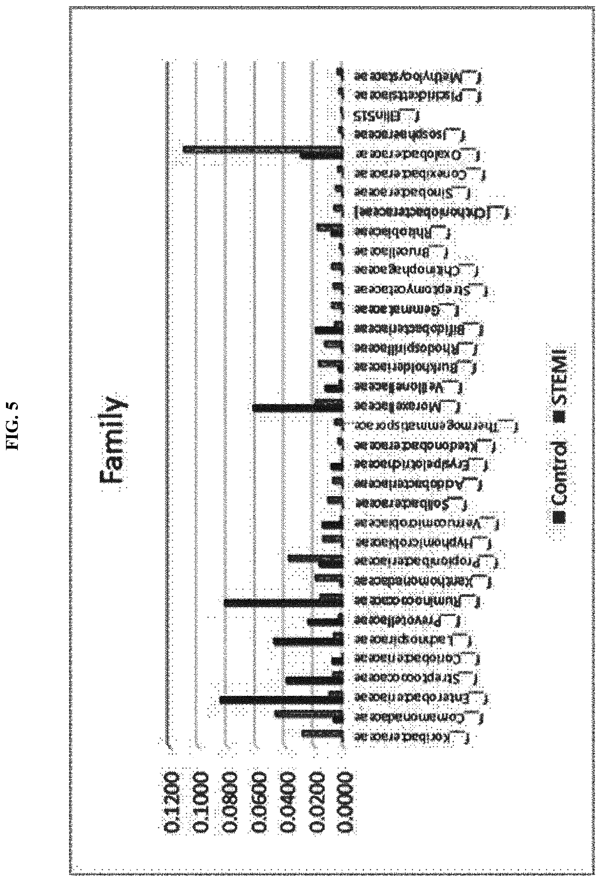

As a result of analyzing bacteria-derived EVs in blood at a family level, a diagnostic model developed using, as a biomarker, one or more bacteria from the family *Koribacteraceae,* the family *Comamonadaceae,* the family *Enterobacteriaceae,* the family *Streptococcaceae,* the family *Coriobacteriaceae,* the family *Lachnospiraceae,* the family *Prevotellaceae,* the family *Ruminococcaceae,* the family *Xanthomonadaceae,* the family *Propionibacteriaceae,* the family *Hyphomicrobiaceae,* the family *Verrucomicrobiaceae,* the family *Solibacteraceae,* the family *Acidobacteriaceae,* the family *Erysipelotrichaceae,* the family *Ktedonobacteraceae,* the family *Thermogemmatisporaceae,* the family *Moraxellaceae,* the family *Veillonellaceae,* the family *Burkholderiaceae,* the family *Rhodospirillaceae,* the family *Bifidobacteriaceae,* the family *Gemmataceae,* the family *Streptomycetaceae,* the family *Chitinophagaceae,* the family *Brucellaceae,* the family *Rhizobiaceae,* the family *Chthoniobacteraceae,* the family *Sinobacteraceae,* the family *Conexibacteraceae,* the family *Oxalobacteraceae,* the family *Isosphaeraceae,* the family Ellin515, the family *Piscirickettsiaceae,* and the family *Methylocystaceae* exhibited significant diagnostic performance for STEMI (see Table 5 and FIG. 5).

**[Table 5]**

| | Control | | STEMI | | t-tes t | | Training Set | | | | Test Set | | | |
|---|---|---|---|---|---|---|---|---|---|---|---|---|---|---|
| Taxon | Me an | SD | Me an | SD | p-va lue | Ra tio | AU C | Acurr acy | Sensit ivity | Speci ficity | AU C | Acu rrac y | Sensi tivity | S pe ci fi cit y |
| f_Coriob acteriaceae | 0.0 075 | 0.0 09 1 | 0.0 001 | 0.0 003 | 0.00 00 | 0. 01 | 0.9 0 | 0.81 | 0.81 | 0.82 | 0.8 8 | 0.7 6 | 0.74 | 0. 85 |
| f_Erysipe lotrichacea e | 0.0 082 | 0.0 12 8 | 0.0 006 | 0.0 021 | 0.00 00 | 0. 07 | 0.8 2 | 0.75 | 0.81 | 0.61 | 0.9 0 | 0.8 3 | 0.85 | 0. 77 |
| f_Entero bacteriace ae | 0.0 848 | 0.0 61 8 | 0.0 097 | 0.0 154 | 0.00 00 | 0. 11 | 0.9 4 | 0.86 | 0.88 | 0.82 | 0.9 5 | 0.8 5 | 0.85 | 0. 85 |
| f_Verruc omicrobia ceae | 0.0 143 | 0.0 18 1 | 0.0 017 | 0.0 041 | 0.00 00 | 0. 12 | 0.8 4 | 0.78 | 0.82 | 0.68 | 0.8 3 | 0.8 0 | 0.83 | 0. 69 |
| f_Prevote llaceae | 0.0 242 | 0.0 23 8 | 0.0 029 | 0.0 066 | 0.00 00 | 0. 12 | 0.8 7 | 0.84 | 0.87 | 0.75 | 0.8 5 | 0.8 2 | 0.79 | 0. 92 |
| f_Lachno spiraceae | 0.0 478 | 0.0 40 2 | 0.0 064 | 0.0 115 | 0.00 00 | 0. 13 | 0.8 9 | 0.82 | 0.85 | 0.73 | 0.9 2 | 0.8 6 | 0.85 | 0. 92 |
| f_Strepto coccaceae | 0.0 390 | 0.0 28 9 | 0.0 070 | 0.0 091 | 0.00 00 | 0. 18 | 0.9 1 | 0.84 | 0.88 | 0.73 | 0.8 8 | 0.8 5 | 0.92 | 0. 54 |
| f_Rumin ococcacea e | 0.0 816 | 0.0 59 9 | 0.0 158 | 0.0 211 | 0.00 00 | 0. 19 | 0.8 7 | 0.81 | 0.85 | 0.68 | 0.9 0 | 0.8 3 | 0.85 | 0. 77 |
| f_Veillon ellaceae | 0.0 124 | 0.0 113 | 0.0 026 | 0.0 047 | 0.00 00 | 0. 21 | 0.8 1 | 0.75 | 0.81 | 0.59 | 0.8 4 | 0.8 8 | 0.92 | 0. 69 |
| f_Bifidob acteriaceae | 0.0 189 | 0.0 27 9 | 0.0 057 | 0.0 085 | 0.00 00 | 0. 30 | 0.7 5 | 0.79 | 0.95 | 0.39 | 0.8 4 | 0.7 9 | 0.91 | 0. 31 |
| f_Moraxe llaceae | 0.0 621 | 0.0 65 7 | 0.0 193 | 0.0 331 | 0.00 00 | 0. 31 | 0.8 1 | 0.77 | 0.94 | 0.36 | 0.9 1 | 0.9 2 | 1.00 | 0. 62 |
| f_Rhizob iaceae | 0.0 081 | 0.0 09 3 | 0.0 179 | 0.0 134 | 0.00 00 | 2. 22 | 0.7 2 | 0.73 | 0.95 | 0.18 | 0.8 8 | 0.8 3 | 0.94 | 0. 38 |
| f_Propio nibacteriac eae | 0.0 166 | 0.0 20 0 | 0.0 374 | 0.0 274 | 0.00 00 | 2. 26 | 0.8 5 | 0.74 | 0.90 | 0.34 | 0.7 4 | 0.7 6 | 0.85 | 0. 38 |
| f_Oxalob acteraceae | 0.0 291 | 0.0 41 3 | 0.1 097 | 0.1 497 | 0.00 02 | 3. 77 | 0.7 0 | 0.75 | 0.95 | 0.25 | 0.7 2 | 0.8 6 | 0.98 | 0. 38 |
| f_Burkho Ideriaceae | 0.0 032 | 0.0 08 7 | 0.0 169 | 0.0 164 | 0.00 00 | 5. 26 | 0.8 0 | 0.77 | 0.94 | 0.36 | 0.8 5 | 0.8 3 | 0.98 | 0. 23 |
| f_Methyl ocystaceae | 0.0 006 | 0.0 02 8 | 0.0 040 | 0.0 083 | 0.00 33 | 7. 03 | 0.6 0 | 0.75 | 0.98 | 0.18 | 0.7 4 | 0.8 3 | 0.96 | 0. 31 |
| f_Coma monadace ae | 0.0 067 | 0.0 10 4 | 0.0 469 | 0.0 378 | 0.00 00 | 7. 03 | 0.9 6 | 0.90 | 0.95 | 0.75 | 0.9 3 | 0.8 8 | 0.92 | 0. 69 |
| f_Xantho monadace ae | 0.0 019 | 0.0 04 4 | 0.0 192 | 0.0 182 | 0.00 00 | 9. 95 | 0.8 7 | 0.86 | 0.97 | 0.59 | 0.9 8 | 0.9 2 | 0.98 | 0. 69 |
| f_Chitino phagaceae | 0.0 006 | 0.0 02 3 | 0.0 077 | 0.0 166 | 0.00 23 | 12 .5 9 | 0.7 3 | 0.84 | 0.99 | 0.45 | 0.6 7 | 0.7 9 | 0.89 | 0. 38 |
| f_Sinoba cteraceae | 0.0 004 | 0.0 01 9 | 0.0 052 | 0.0 078 | 0.00 00 | 12 .9 5 | 0.7 0 | 0.81 | 0.98 | 0.36 | 0.6 8 | 0.8 2 | 0.92 | 0. 38 |
| f_Rhodos pirillaceae | 0.0 009 | 0.0 03 8 | 0.0 127 | 0.0 157 | 0.00 00 | 13.8 0 | 0.7 7 | 0.86 | 0.98 | 0.57 | 0.8 7 | 0.9 1 | 0.94 | 0. 77 |
| f_Hypho microbiace ae | 0.0 008 | 0.0 03 6 | 0.0 139 | 0.0 150 | 0.00 00 | 16 .9 4 | 0.8 5 | 0.88 | 0.97 | 0.64 | 0.7 7 | 0.8 3 | 0.96 | 0. 31 |
| f_Brucell aceae | 0.0 001 | 0.0 00 9 | 0.0 024 | 0.0 058 | 0.00 53 | 20 .4 6 | 0.7 3 | 0.78 | 0.98 | 0.27 | 0.6 6 | 0.8 0 | 0.98 | 0. 08 |
| f_Ellin51 5 | 0.0 001 | 0.0 00 6 | 0.0 016 | 0.0 033 | 0.00 11 | 21 .9 6 | 0.6 7 | 0.77 | 0.98 | 0.23 | 0.7 3 | 0.8 3 | 1.00 | 0. 15 |
| f_Strcpto mycetacea e | 0.0 003 | 0.0 01 5 | 0.0 066 | 0.0 090 | 0.00 00 | 23 .9 9 | 0.7 4 | 0.84 | 0.98 | 0.50 | 0.7 1 | 0.8 5 | 0.98 | 0. 31 |
| f_Isospha eraceae | 0.0 001 | 0.0 01 0 | 0.0 028 | 0.0 060 | 0.00 15 | 26 .1 5 | 0.6 8 | 0.78 | 0.99 | 0.25 | 0.7 1 | 0.8 2 | 0.98 | 0. 15 |
| f_[Chtho niobactera ceae] | 0.0 001 | 0.0 00 9 | 0.0 062 | 0.0 116 | 0.00 03 | 49 .8 9 | 0.7 2 | 0.82 | 0.99 | 0.41 | 0.6 8 | 0.8 5 | 0.98 | 0. 31 |
| f_Gemm ataceae | 0.0 001 | 0.0 00 7 | 0.0 077 | 0.0 113 | 0.00 00 | 75 .2 9 | 0.7 5 | 0.85 | 1.00 | 0.48 | 0.7 6 | 0.8 5 | 0.96 | 0. 38 |
| f_Conexi bacteracea e | 0.0 000 | 0.0 00 2 | 0.0 036 | 0.0 076 | 0.00 07 | 15 0. 79 | 0.7 0 | 0.79 | 0.99 | 0.27 | 0.7 5 | 0.8 5 | 1.00 | 0. 23 |
| f_Ktedon obacterace ae | 0.0 000 | 0.0 00 2 | 0.0 034 | 0.0 069 | 0.00 05 | 17 4. 45 | 0.8 1 | 0.85 | 1.00 | 0.48 | 0.7 7 | 0.8 5 | 0.98 | 0. 31 |
| f_Acidob acteriaceae | 0.0 000 | 0.0 00 3 | 0.0 072 | 0.0 095 | 0.00 00 | 19 3. 78 | 0.8 2 | 0.85 | 0.99 | 0.50 | 0.7 3 | 0.8 8 | 0.98 | 0. 46 |
| f_Therm ogemmatis poraceae | 0.0 000 | 0.0 00 2 | 0.0 057 | 0.0 082 | 0.00 00 | 40 7. 10 | 0.8 1 | 0.85 | 1.00 | 0.48 | 0.7 9 | 0.8 6 | 0.98 | 0. 38 |
| f_Koriba cteraceae | 0.0 000 | 0.0 00 3 | 0.0 282 | 0.0 177 | 0.00 00 | 58 2. 60 | 0.9 7 | 0.97 | 1.00 | 0.89 | 0.9 6 | 0.9 5 | 1.00 | 0. 77 |
| f_Pisciric kettsiaceae | 0.0 000 | 0.0 00 0 | 0.0 028 | 0.0 062 | 0.00 16 | 59 4. 18 | 0.6 4 | 0.77 | 1.00 | 0.20 | 0.6 3 | 0.8 2 | 0.98 | 0. 15 |
| f_Solibac teraceae | 0.0 000 | 0.0 00 1 | 0.0 104 | 0.0 122 | 0.00 00 | 82 8. 65 | 0.8 3 | 0.88 | 1.00 | 0.57 | 0.7 1 | 0.8 8 | 0.98 | 0. 46 |

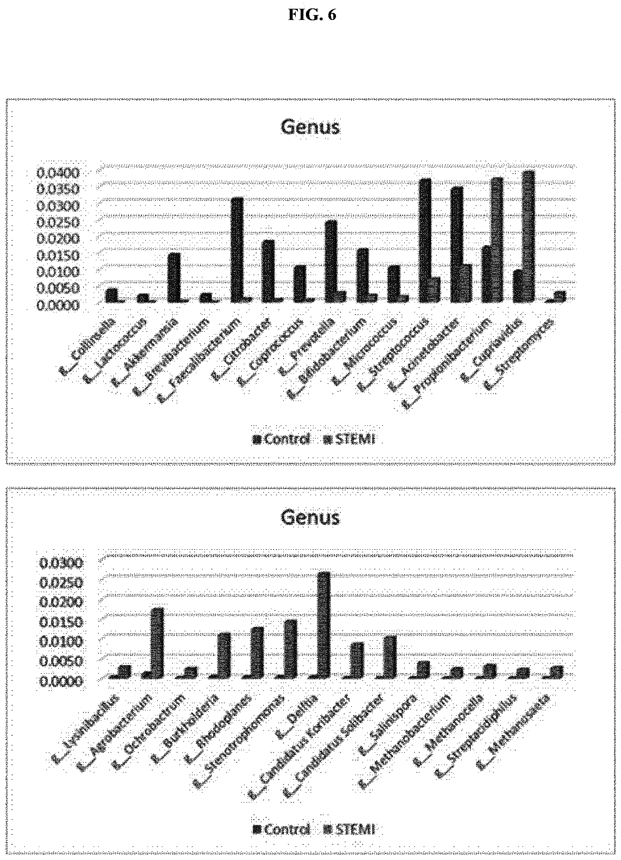

As a result of analyzing bacteria-derived EVs in blood at a genus level, a diagnostic model developed using, as a biomarker, one or more bacteria from the genus *Delftia,* the genus *Agrobacterium,* the genus *Stenotrophomonas,* the genus *Faecalibacterium,* the genus *Candidatus Koribacter,* the genus *Akkermansia,* the genus *Streptococcus,* the genus *Salinispora,* the genus *Candidatus Solibacter,* the genus *Citrobacter,* the genus *Collinsella,* the genus *Burkholderia,* the genus *Coprococcus,* the genus *Rhodoplanes,* the genus *Acinetobacter,* the genus *Prevotella,* the genus *Propionibacterium,* the genus *Lactococcus,* the genus *Bifidobacterium,* the genus *Methanobacterium,* the genus *Micrococcus,* the genus *Methanocella,* the genus *Brevibacterium,* the genus *Streptacidiphilus,* the genus *Streptomyces,* the genus *Ochrobactrum,* the genus *Methanosaeta,* the genus *Lysinibacillus,* and the genus *Cupriavidus* exhibited significant diagnostic performance for STEMI (see Table 6 and FIG. 6).

**[Table 6]**

| | Control | | STEMI | | t-tes t | | Training Set | | | | Test Set | | | |
|---|---|---|---|---|---|---|---|---|---|---|---|---|---|---|
| Taxon | Me an | SD | Me an | SD | p-v alue | Ratio | AU C | Acu rrac y | Sensi tivity | Speci ficity | AU C | Acu rrac y | Sens itivit y | s p e ci fi ci ty |
| g_Collin sella | 0.0 036 | 0.0 062 | 0.0 000 | 0.0 000 | 0.0 000 | 0.00 | 0.8 5 | 0.7 5 | 0.74 | 0.79 | 0.8 5 | 0.7 6 | 0.76 | 0. 7 3 |
| g_Lactoc occus | 0.0 020 | 0.0 038 | 0.0 000 | 0.0 000 | 0.0 001 | 0.00 | 0.8 2 | 0.7 6 | 0.94 | 0.29 | 0.8 2 | 0.8 0 | 0.96 | 0. 2 7 |
| g_Akker mansia | 0.0 143 | 0.0 181 | 0.0 002 | 0.0 013 | 0.0 000 | 0.01 | 0.8 8 | 0.8 1 | 0.82 | 0.79 | 0.9 2 | 0.8 6 | 0.84 | 0. 9 3 |
| g_Brevib acterium | 0.0 022 | 0.0 054 | 0.0 000 | 0.0 003 | 0.0 000 | 0.02 | 0.7 6 | 0.7 3 | 0.96 | 0.12 | 0.7 0 | 0.7 4 | 0.96 | 0. 0 0 |
| g_Faccal ibacterium | 0.0 311 | 0.0 310 | 0.0 010 | 0.0 037 | 0.0 000 | 0.03 | 0.9 0 | 0.8 3 | 0.79 | 0.93 | 0.8 9 | 0.8 6 | 0.88 | 0. 8 0 |
| g_Citrob actcr | 0.0 182 | 0.0 200 | 0.0 008 | 0.0 024 | 0.0 000 | 0.04 | 0.8 7 | 0.8 0 | 0.85 | 0.67 | 0.9 0 | 0.8 2 | 0.86 | 0. 6 7 |
| g_Copro coccus | 0.0 105 | 0.0 211 | 0.0 007 | 0.0 045 | 0.0 000 | 0.07 | 0.8 4 | 0.7 9 | 0.91 | 0.48 | 0.8 9 | 0.8 5 | 0.98 | 0. 4 0 |
| g_Prevot ella | 0.0 242 | 0.0 238 | 0.0 029 | 0.0 066 | 0.0 000 | 0.12 | 0.8 3 | 0.7 7 | 0.85 | 0.57 | 0.9 7 | 0.9 2 | 0.94 | 0. 8 7 |
| g_Bifido bacterium | 0.0 157 | 0.0 266 | 0.0 020 | 0.0 042 | 0.0 000 | 0.13 | 0.8 1 | 0.8 1 | 0.90 | 0.57 | 0.9 1 | 0.8 6 | 0.88 | 0. 8 0 |
| g_Micro coccus | 0.0 105 | 0.0 165 | 0.0 017 | 0.0 043 | 0.0 000 | 0.16 | 0.7 9 | 0.7 7 | 0.97 | 0.21 | 0.6 9 | 0.8 0 | 0.98 | 0. 2 0 |
| g_Strept ococcus | 0.0 368 | 0.0 287 | 0.0 070 | 0.0 091 | 0.0 000 | 0.19 | 0.8 8 | 0.8 2 | 0.89 | 0.64 | 0.9 2 | 0.8 5 | 0.90 | 0. 6 7 |
| g_Acinet obacter | 0.0 343 | 0.0 391 | 0.0 109 | 0.0 254 | 0.0 000 | 0.32 | 0.8 3 | 0.7 9 | 0.96 | 0.33 | 0.9 2 | 0.8 8 | 0.98 | 0. 5 3 |
| g_Propio nibacteriu m | 0.0 165 | 0.0 199 | 0.0 372 | 0.0 274 | 0.0 000 | 2.25 | 0.8 2 | 0.7 4 | 0.93 | 0.24 | 0.8 2 | 0.7 9 | 0.92 | 0. 3 3 |
| g_Cupria vidus | 0.0 092 | 0.0 145 | 0.0 393 | 0.0 663 | 0.0 014 | 4.25 | 0.6 2 | 0.7 7 | 0.96 | 0.26 | 0.5 4 | 0.7 9 | 0.96 | 0. 2 0 |
| g_Strept omyces | 0.0 003 | 0.0 015 | 0.0 029 | 0.0 056 | 0.0 011 | 10.8 4 | 0.7 5 | 0.7 7 | 0.97 | 0.24 | 0.7 0 | 0.7 9 | 1.00 | 0. 0 7 |
| g_Lysini bacillus | 0.0 002 | 0.0 013 | 0.0 028 | 0.0 064 | 0.0 035 | 12.2 8 | 0.6 8 | 0.7 9 | 0.99 | 0.24 | 0.6 6 | 0.8 0 | 0.98 | 0. 2 0 |
| g_Agrob acterium | 0.0 012 | 0.0 035 | 0.0 173 | 0.0 133 | 0.0 000 | 14.0 1 | 0.9 7 | 0.9 4 | 0.96 | 0.86 | 0.7 4 | 0.8 6 | 0.94 | 0. 6 0 |
| g_Ochro bactrum | 0.0 001 | 0.0 009 | 0.0 024 | 0.0 058 | 0.0 052 | 21.2 7 | 0.7 2 | 0.8 0 | 0.99 | 0.29 | 0.7 1 | 0.7 9 | 0.96 | 0. 2 0 |
| g_Burkh olderia | 0.0 004 | 0.0 019 | 0.0 109 | 0.0 136 | 0.0 000 | 27.0 2 | 0.8 4 | 0.8 7 | 0.99 | 0.55 | 0.8 4 | 0.8 8 | 0.98 | 0. 5 3 |
| g_Rhode planes | 0.0 003 | 0.0 024 | 0.0 125 | 0.0 150 | 0.0 000 | 49.8 5 | 0.8 3 | 0.8 9 | 0.99 | 0.62 | 0.8 4 | 0.8 6 | 1.00 | 0. 4 0 |
| g_Stenot rophomon as | 0.0 003 | 0.0 008 | 0.0 143 | 0.0 165 | 0.0 000 | 50.8 8 | 0.9 2 | 0.9 1 | 0.98 | 0.71 | 0.9 6 | 0.9 2 | 0.94 | 0. 8 7 |
| g_Delftia | 0.0 003 | 0.0 010 | 0.0 263 | 0.0 202 | 0.0 000 | 89.4 3 | 1.0 0 | 0.9 9 | 1.00 | 0.95 | 0.9 5 | 0.9 7 | 1.00 | 0. 8 7 |
| g_Candi datus Koribacter | 0.0 000 | 0.0 002 | 0.0 085 | 0.0 092 | 0.0 000 | 413. 93 | 0.9 0 | 0.9 0 | 0.99 | 0.67 | 0.7 4 | 0.8 5 | 0.98 | 0. 4 0 |
| g_Candi datus Solibacter | 0.0 000 | 0.0 001 | 0.0 101 | 0.0 120 | 0.0 000 | 803. 61 | 0.8 7 | 0.9 0 | 0.99 | 0.64 | 0.6 8 | 0.8 2 | 0.98 | 0. 2 7 |
| g_Salinis pora | 0.0 000 | 0.0 000 | 0.0 038 | 0.0 063 | 0.0 000 | 1032 9.07 | 0.8 7 | 0.9 2 | 1.00 | 0.71 | 0.8 7 | 0.9 1 | 0.98 | 0. 6 7 |
| g_Metha nobacteriu m | 0.0 000 | 0.0 000 | 0.0 023 | 0.0 061 | 0.0 000 | >100 | 0.8 0 | 0.8 4 | 1.00 | 0.43 | 0.6 6 | 0.8 3 | 1.00 | 0. 2 7 |
| g_Metha nocclla | 0.0 000 | 0.0 000 | 0.0 032 | 0.0 077 | 0.0 000 | >100 | 0.7 9 | 0.8 4 | 1.00 | 0.40 | 0.6 7 | 0.8 6 | 1.00 | 0. 4 0 |
| g_Strepta cidiphilus | 0.0 000 | 0.0 000 | 0.0 022 | 0.0 055 | 0.0 000 | >100 | 0.7 5 | 0.8 4 | 1.00 | 0.40 | 0.7 4 | 0.8 6 | 1.00 | 0. 4 0 |
| g_Metha nosaeta | 0.0 000 | 0.0 000 | 0.0 027 | 0.0 061 | 0.0 000 | >100 | 0.6 9 | 0.8 2 | 1.00 | 0.33 | 0.6 5 | 0.8 2 | 1.00 | 0. 2 0 |

### Example 5. Dilated Cardiomyopathy Diagnostic Model Based on Metagenomic Profiling of Bacteria-Derived EVs

EVs were isolated from blood samples of 72 dilated cardiomyopathy (DCMP) patients and 163 normal individuals, the two groups matched in age and gender, and then metagenomic sequencing was performed thereon using the method of Example 3. For the development of a diagnostic model, first, a strain exhibiting a p value of less than 0.05 between two groups in a t-test and a difference of two-fold or more between two groups was selected, and then an AUC, accuracy, sensitivity, and specificity, which are diagnostic performance indexes, were calculated by logistic regression analysis.

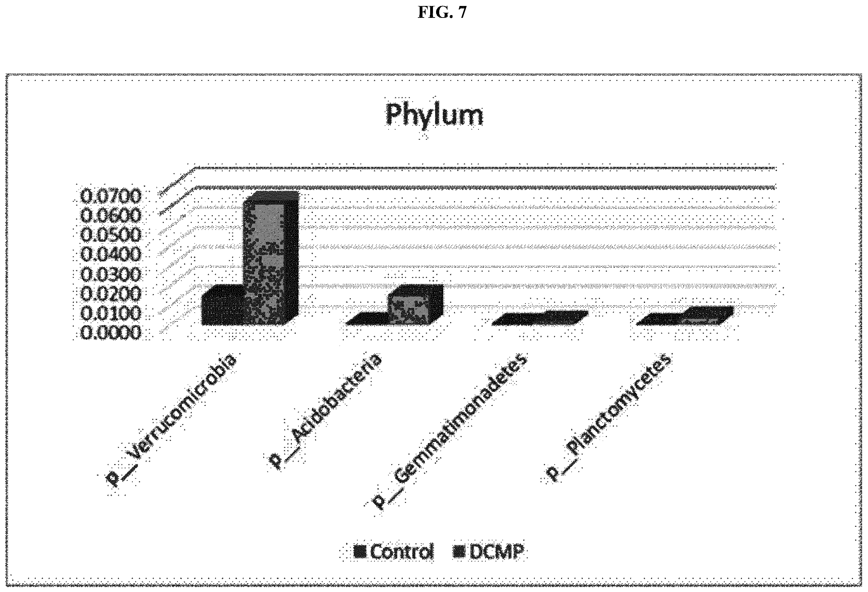

As a result of analyzing bacteria-derived EVs in blood at a phylum level, a diagnostic model developed using, as a biomarker, one or more bacteria from the phylum *Verrucomicrobia*, the phylum *Acidobacteria*, the phylum *Gemmatimonadetes*, and the phylum *Planctomycetes* exhibited significant diagnostic performance for DCMP (see Table 7 and FIG. 7).

**[Table 7]**

| | Control | | DCMP | | t-tes t | | Training Set | | | | Test Set | | | |
|---|---|---|---|---|---|---|---|---|---|---|---|---|---|---|
| Taxon | Me an | SD | Me an | SD | p-v alue | Rati o | AU C | Acu rrac y | Sensit ivity | Speci ficity | AU C | Acur racy | Sens itivit y | s pe ci fi ci ty |
| p_Verr ucomicr obia | 0.0 147 | 0.0 183 | 0.0 618 | 0.0 628 | 0.0 000 | 4.20 | 0.8 0 | 0.8 1 | 0.97 | 0.46 | 0.7 0 | 0.82 | 0.98 | 0. 4 0 |
| p_Gem matimon adetes | 0.0 004 | 0.0 021 | 0.0 019 | 0.0 030 | 0.0 004 | 4.44 | 0.7 1 | 0.7 1 | 0.97 | 0.13 | 0.7 4 | 0.73 | 0.98 | 0. 1 0 |
| p_Plan ctomycc tes | 0.0 004 | 0.0 017 | 0.0 040 | 0.0 110 | 0.0 079 | 9.88 | 0.6 4 | 0.7 1 | 0.96 | 0.17 | 0.6 5 | 0.75 | 0.98 | 0. 1 5 |
| p_Acid obacteri a | 0.0 009 | 0.0 027 | 0.0 148 | 0.0 278 | 0.0 001 | 15.8 7 | 0.7 8 | 0.7 6 | 0.96 | 0.33 | 0.8 4 | 0.85 | 0.98 | 0. 5 0 |

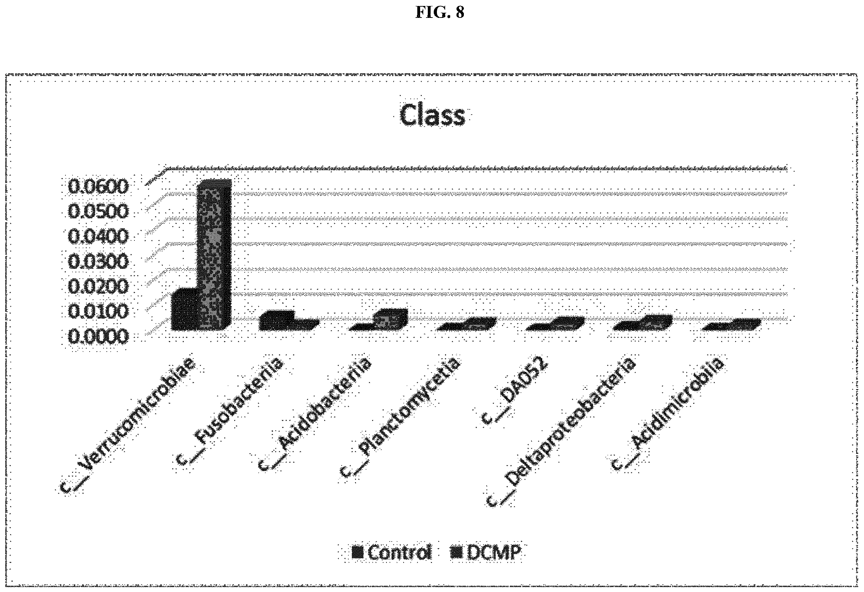

As a result of analyzing bacteria-derived EVs in blood at a class level, a diagnostic model developed using, as a biomarker, one or more bacteria from the class *Verrucomicrobiae*, the class *Fusobacteriia*, the class *Acidobacteriia*, the class *Planctomycetia*, the class DA052, the class *Deltaproteobacteria*, and the class *Acidimicrobiia* exhibited significant diagnostic performance for DCMP (see Table 8 and FIG. 8).

**[Table 8]**

| | Control | | DCMP | | t-tes t | | Training Set | | | | Test Set | | | |
|---|---|---|---|---|---|---|---|---|---|---|---|---|---|---|
| Taxon | Me an | SD | Me an | SD | p-va lue | Ratio | AU C | Acu rrac y | Sensit ivity | Specif icity | AU C | Acu rrac y | Sensi tivity | S p ec ifi ci ty |
| c_Fuso bacteriia | 0.0 053 | 0.0 085 | 0.0 016 | 0.0 031 | 0.00 00 | 0.30 | 0.7 0 | 0.6 6 | 0.95 | 0.08 | 0.6 0 | 0.7 0 | 0.92 | 0. 1 1 |
| c_Delta proteoba cteria | 0.0 011 | 0.0 023 | 0.0 035 | 0.0 074 | 0.00 73 | 3.30 | 0.6 0 | 0.7 1 | 0.97 | 0.15 | 0.6 3 | 0.7 5 | 0.92 | 0. 2 6 |
| c_Verr ucomicr obiae | 0.0 143 | 0.0 181 | 0.0 575 | 0.0 637 | 0.00 00 | 4.01 | 0.7 1 | 0.7 9 | 0.98 | 0.40 | 0.6 7 | 0.8 5 | 1.00 | 0. 4 2 |
| c_Acidi microbii a | 0.0 003 | 0.0 013 | 0.0 019 | 0.0 046 | 0.00 42 | 6.87 | 0.6 0 | 0.7 2 | 0.98 | 0.17 | 0.6 2 | 0.7 6 | 0.98 | 0. 1 6 |
| c_Planc tomyceti a | 0.0 003 | 0.0 015 | 0.0 026 | 0.0 067 | 0.00 64 | 7.82 | 0.6 3 | 0.6 8 | 0.96 | 0.09 | 0.6 2 | 0.7 6 | 0.98 | 0. 1 6 |
| c_Acid obacterii a | 0.0 001 | 0.0 005 | 0.0 061 | 0.0 129 | 0.00 02 | 71.46 | 0.6 9 | 0.7 7 | 0.96 | 0.38 | 0.7 4 | 0.8 7 | 1.00 | 0. 5 3 |
| c_DA0 52 | 0.0 000 | 0.0 001 | 0.0 029 | 0.0 091 | 0.00 85 | 251.0 8 | 0.6 3 | 0.7 4 | 0.99 | 0.23 | 0.6 3 | 0.8 3 | 1.00 | 0. 3 7 |

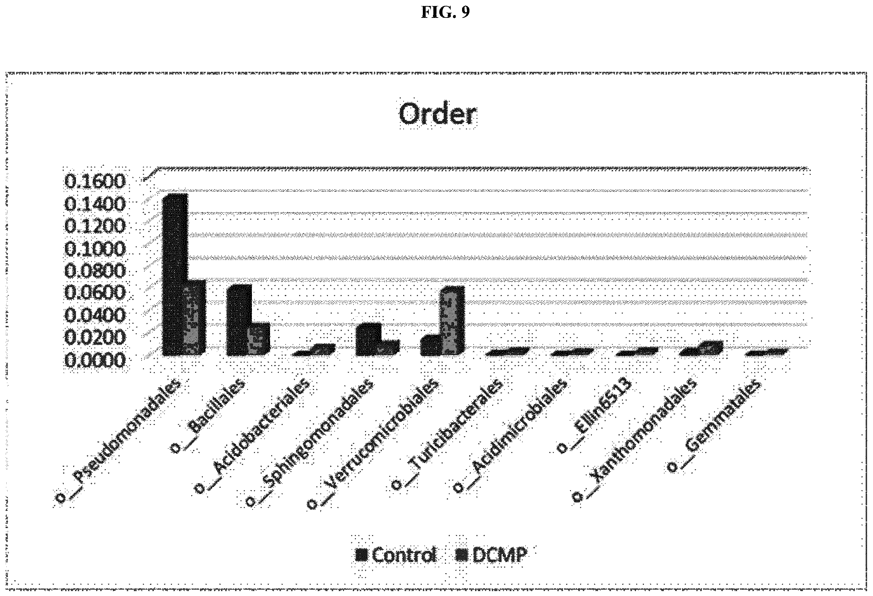

As a result of analyzing bacteria-derived EVs in blood at an order level, a diagnostic model developed using, as a biomarker, one or more bacteria from the order *Pseudomonadales*, the order *Bacillales*, the order *Acidobacteriales*, the order *Sphingomonadales*, the order *Verrucomicrobiales*, the order *Turicibacterales*, the order *Acidimicrobiales*, the order Ellin6513, the order *Xanthomonadales*, and the order *Gemmatales* exhibited significant diagnostic performance for DCMP (see Table 9 and FIG. 9).

**[Table 9]**

| | Control | | DCMP | | t-tes t | | Training Set | | | | Test Set | | | |
|---|---|---|---|---|---|---|---|---|---|---|---|---|---|---|
| Taxon | Me an | SD | Me an | SD | p-va lue | Ratio | AU C | Ac urra cy | Sensi tivity | Specif icity | AU C | Ac urra cy | Sens itivit y | Sp ec ifi cit Y |
| c_Fuso bacteriia | 0.0 053 | 0.0 085 | 0.0 016 | 0.0 031 | 0.00 00 | 0.30 | 0.7 0 | 0.6 6 | 0.95 | 0.08 | 0.6 0 | 0.7 0 | 0.92 | 0. 11 |
| c_Delta proteoba cteria | 0.0 011 | 0.0 023 | 0.0 035 | 0.0 074 | 0.00 73 | 3.30 | 0.6 0 | 0.7 1 | 0.97 | 0.15 | 0.6 3 | 0.7 5 | 0.92 | 0. 26 |
| c_Verru comicrob iae | 0.0 143 | 0.0 181 | 0.0 575 | 0.0 637 | 0.00 00 | 4.01 | 0.7 1 | 0.7 9 | 0.98 | 0.40 | 0.6 7 | 0.8 5 | 1.00 | 0. 42 |
| c_Acidi microbiia | 0.0 003 | 0.0 013 | 0.0 019 | 0.0 046 | 0.00 42 | 6.87 | 0.6 0 | 0.7 2 | 0.98 | 0.17 | 0.6 2 | 0.7 6 | 0.98 | 0. 16 |
| c_Planc tomyceti a | 0.0 003 | 0.0 015 | 0.0 026 | 0.0 067 | 0.00 64 | 7.82 | 0.6 3 | 0.6 8 | 0.96 | 0.09 | 0.6 2 | 0.7 6 | 0.98 | 0. 16 |
| c_Acid obacterii a | 0.0 001 | 0.0 005 | 0.0 061 | 0.0 129 | 0.00 02 | 71.46 | 0.6 9 | 0.7 7 | 0.96 | 0.38 | 0.7 4 | 0.8 7 | 1.00 | 0. 53 |
| c_DA0 52 | 0.0 000 | 0.0 001 | 0.0 029 | 0.0 091 | 0.00 85 | 251.0 8 | 0.6 3 | 0.7 4 | 0.99 | 0.23 | 0.6 3 | 0.8 3 | 1.00 | 0. 37 |

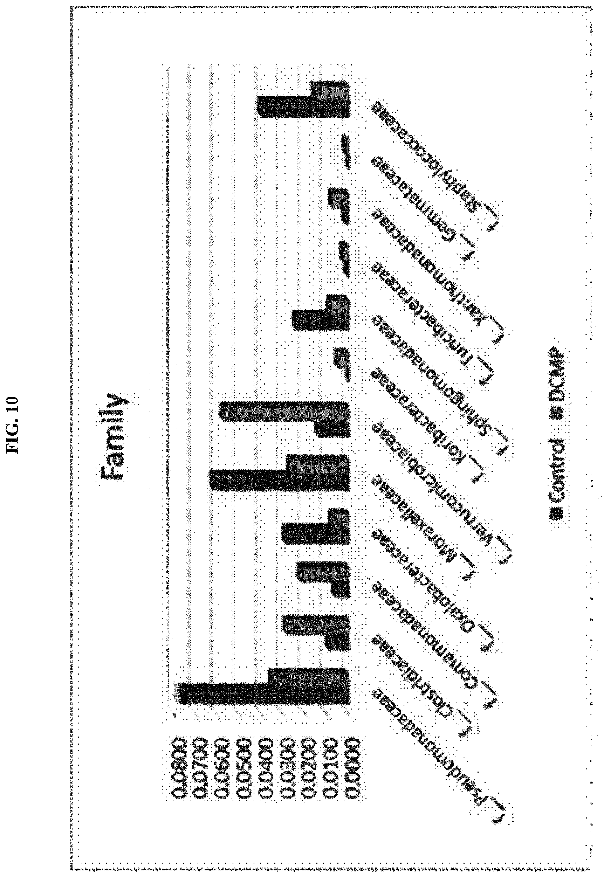

As a result of analyzing bacteria-derived EVs in blood at a family level, a diagnostic model developed using, as a biomarker, one or more bacteria from the family *Pseudomonadaceae*, the family *Clostridiaceae,* the family *Comamonadaceae,* the family *Oxalobacteraceae,* the family *Moraxellaceae,* the family *Verrucomicrobiaceae,* the family *Koribacteraceae,* the family *Sphingomonadaceae,* the family *Turicibacteraceae,* the family *Xanthomonadaceae,* the family *Gemmataceae,* and the family *Staphylococcaceae* exhibited significant diagnostic performance for DCMP (see Table 10 and FIG. 10).

**[Table 10]**

| | Control | | DCMP | | t-tes t | | Training Set | | | | Test Set | | | |
|---|---|---|---|---|---|---|---|---|---|---|---|---|---|---|
| Taxon | Me an | SD | Me an | SD | p-v alue | Ratio | AU C | Acur racy | Sensi tivity | Spec ificit y | AU C | Acu rrac y | Sensi tivity | s p e c if i c it y |
| f_Oxalo bacterace ae | 0.0 291 | 0.0 413 | 0.0 079 | 0.0 105 | 0.0 000 | 0.27 | 0.7 5 | 0.70 | 0.91 | 0.18 | 0.6 7 | 0.63 | 0.85 | 0 .1 7 |
| f_Sphin gomonad aceae | 0.0 244 | 0.0 258 | 0.0 089 | 0.0 116 | 0.0 000 | 0.37 | 0.7 1 | 0.76 | 1.00 | 0.18 | 0.7 2 | 0.65 | 0.90 | 0 .1 3 |
| f_Staph ylococca ceae | 0.0 403 | 0.0 549 | 0.0 160 | 0.0 134 | 0.0 000 | 0.40 | 0.6 4 | 0.70 | 0.98 | 0.02 | 0.6 8 | 0.66 | 0.98 | 0 .0 0 |
| f_Mora xellaceae | 0.0 621 | 0.0 657 | 0.0 272 | 0.0 282 | 0.0 000 | 0.44 | 0.7 4 | 0.73 | 0.93 | 0.27 | 0.7 6 | 0.65 | 0.81 | 0 .3 0 |
| f_Pseud omonada ceae | 0.0 781 | 0.0 481 | 0.0 356 | 0.0 339 | 0.0 000 | 0.46 | 0.8 4 | 0.83 | 0.92 | 0.61 | 0.6 7 | 0.65 | 0.77 | 0 .3 9 |
| f_Clostr idiaceae | 0.0 093 | 0.0 121 | 0.0 285 | 0.0 212 | 0.0 000 | 3.06 | 0.7 8 | 0.81 | 0.94 | 0.51 | 0.7 8 | 0.77 | 0.96 | 0 .3 9 |
| f_Turici bacterace ae | 0.0 009 | 0.0 026 | 0.0 030 | 0.0 060 | 0.0 052 | 3.28 | 0.6 8 | 0.70 | 0.96 | 0.10 | 0.6 6 | 0.69 | 1.00 | 0 .0 4 |
| f_Coma monadac eae | 0.0 066 | 0.0 104 | 0.0 221 | 0.0 293 | 0.0 000 | 3.34 | 0.7 6 | 0.75 | 0.94 | 0.31 | 0.6 8 | 0.73 | 0.90 | 0 .3 9 |
| f_Verru comicrob iaccac | 0.0 143 | 0.0 181 | 0.0 575 | 0.0 637 | 0.0 000 | 4.01 | 0.7 4 | 0.80 | 0.97 | 0.41 | 0.6 9 | 0.80 | 0.96 | 0 .4 8 |
| f_Xanth omonada ceae | 0.0 019 | 0.0 044 | 0.0 080 | 0.0 154 | 0.0 017 | 4.13 | 0.6 7 | 0.73 | 0.97 | 0.16 | 0.5 4 | 0.70 | 0.98 | 0 .1 3 |
| f_Gcm mataceae | 0.0 001 | 0.0 007 | 0.0 017 | 0.0 045 | 0.0 042 | 16.52 | 0.6 5 | 0.76 | 0.98 | 0.22 | 0.4 8 | 0.70 | 1.00 | 0 .0 9 |
| f_Korib acteracea e | 0.0 000 | 0.0 003 | 0.0 053 | 0.0 122 | 0.0 005 | 110.0 8 | 0.7 2 | 0.80 | 0.98 | 0.37 | 0.7 2 | 0.85 | 1.00 | 0 .5 2 |

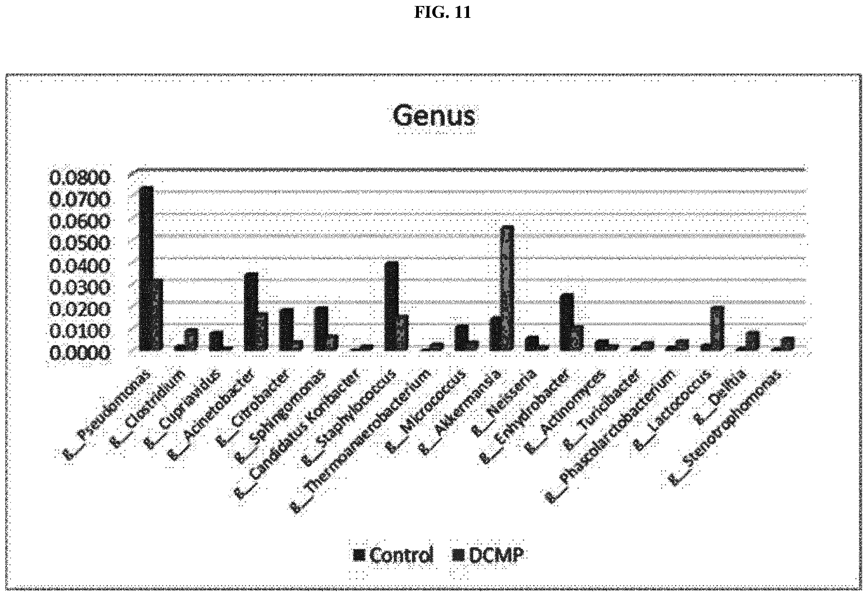

As a result of analyzing bacteria-derived EVs in blood at a genus level, a diagnostic model developed using, as a biomarker, one or more bacteria from the genus *Pseudomonas,* the genus *Clostridium,* the genus *Cupriavidus,* the genus *Acinetobacter,* the genus *Citrobacter,* the genus *Sphingomonas,* the genus *Candidatus Koribacter,* the genus *Staphylococcus,* the genus *Thermoanaerobacterium,* the genus *Micrococcus,* the genus *Akkermansia,* the genus *Neisseria,* the genus *Enhydrobacter,* the genus *Actinomyces,* the genus *Turicibacter,* the genus *Phascolarctobacterium,* the genus *Lactococcus,* the genus *Delftia,* and the genus *Stenotrophomonas* exhibited significant diagnostic performance for DCMP (see Table 11 and FIG. 11).

**[Table 11]**

| | Control | | DCMP | | t-tes t | | Training Set | | | | Test Set | | | |
|---|---|---|---|---|---|---|---|---|---|---|---|---|---|---|
| Taxon | Mea n | SD | Mea n | SD | p-v alue | Rati o | AU C | Acu rrac y | Sensi tivity | Speci ficity | AU C | Acu rrac y | Sensi tivity | s p e c if i c it y |
| g_Cupria vidus | 0.00 77 | 0.01 20 | 0.00 06 | 0.00 11 | 0.00 00 | 0.07 | 0.7 5 | 0.65 | 0.63 | 0.70 | 0.75 | 0.72 | 0.78 | 0 .5 0 |
| g_Citroba ctcr | 0.01 81 | 0.01 99 | 0.00 35 | 0.00 45 | 0.00 00 | 0.19 | 0.7 4 | 0.67 | 0.76 | 0.50 | 0.76 | 0.72 | 0.80 | 0 .4 4 |
| g_Neisser ia | 0.00 55 | 0.01 09 | 0.00 12 | 0.00 37 | 0.00 00 | 0.23 | 0.6 6 | 0.64 | 0.96 | 0.02 | 0.64 | 0.77 | 0.98 | 0 .0 6 |
| g_Microc occus | 0.01 05 | 0.01 65 | 0.00 33 | 0.00 52 | 0.00 00 | 0.32 | 0.6 7 | 0.63 | 0.95 | 0.00 | 0.66 | 0.79 | 1.00 | 0 .0 6 |
| g_Sphing omonas | 0.01 89 | 0.02 20 | 0.00 62 | 0.00 96 | 0.00 00 | 0.33 | 0.7 3 | 0.68 | 0.88 | 0.29 | 0.73 | 0.73 | 0.87 | 0 .2 5 |
| g_Staphyl ococcus | 0.03 95 | 0.05 45 | 0.01 52 | 0.01 29 | 0.00 00 | 0.38 | 0.7 2 | 0.68 | 0.83 | 0.38 | 0.57 | 0.62 | 0.75 | 0 .1 9 |
| g_Enhydr obacter | 0.02 46 | 0.05 24 | 0.01 03 | 0.01 42 | 0.00 15 | 0.42 | 0.6 6 | 0.66 | 0.96 | 0.09 | 0.64 | 0.80 | 1.00 | 0 .1 3 |
| g_Pseudo monas | 0.07 33 | 0.04 64 | 0.03 13 | 0.03 23 | 0.00 00 | 0.43 | 0.8 2 | 0.78 | 0.86 | 0.63 | 0.79 | 0.75 | 0.76 | 0 .6 9 |
| g_Actino myces | 0.00 39 | 0.00 60 | 0.00 17 | 0.00 27 | 0.00 02 | 0.44 | 0.6 6 | 0.64 | 0.96 | 0.02 | 0.56 | 0.80 | 1.00 | 0 .1 3 |
| g_Acinet obacter | 0.03 43 | 0.03 91 | 0.01 62 | 0.02 08 | 0.00 00 | 0.47 | 0.7 5 | 0.71 | 0.94 | 0.27 | 0.79 | 0.79 | 0.98 | 0 .1 3 |
| g_Turicib acter | 0.00 09 | 0.00 26 | 0.00 30 | 0.00 60 | 0.00 52 | 3.28 | 0.6 6 | 0.69 | 0.93 | 0.23 | 0.62 | 0.79 | 0.89 | 0 .4 4 |
| g_Phascol arctobacter ium | 0.00 11 | 0.00 22 | 0.00 40 | 0.00 64 | 0.00 04 | 3.73 | 0.6 5 | 0.71 | 0.96 | 0.23 | 0.63 | 0.79 | 0.89 | 0 .4 4 |
| | | | | | | | | | | | | | | |
| g_Akker mansia | 0.01 43 | 0.01 81 | 0.05 54 | 0.06 45 | 0.00 00 | 3.88 | 0.6 7 | 0.77 | 0.97 | 0.38 | 0.68 | 0.87 | 1.00 | 0 .4 4 |
| g_Clostri dium | 0.00 15 | 0.00 24 | 0.00 90 | 0.01 10 | 0.00 00 | 6.12 | 0.7 6 | 0.79 | 0.94 | 0.50 | 0.66 | 0.86 | 0.95 | 0 .5 6 |
| g_Lactoc occus | 0.00 20 | 0.00 38 | 0.01 91 | 0.05 09 | 0.00 60 | 9.45 | 0.6 4 | 0.72 | 0.96 | 0.25 | 0.72 | 0.82 | 0.91 | 0 .5 0 |
| g_Delftia | 0.00 04 | 0.00 23 | 0.00 77 | 0.02 02 | 0.00 34 | 18.2 3 | 0.6 2 | 0.70 | 0.99 | 0.14 | 0.48 | 0.80 | 1.00 | 0 .1 3 |
| g_Stenotr ophomonas | 0.00 03 | 0.00 08 | 0.00 51 | 0.01 36 | 0.00 42 | 18.4 2 | 0.6 1 | 0.68 | 0.98 | 0.11 | 0.54 | 0.83 | 1.00 | 0 .2 5 |
| g_Candid atus Koribacter | 0.00 00 | 0.00 02 | 0.00 16 | 0.00 50 | 0.00 88 | 78.6 9 | 0.7 3 | 0.82 | 1.00 | 0.41 | 0.60 | 0.72 | 0.94 | 0 .2 6 |
| g_Therm oanaerobac terium | 0.00 00 | 0.00 00 | 0.00 25 | 0.00 38 | 0.00 00 | >10 0 | 0.7 1 | 0.82 | 1.00 | 0.41 | 0.76 | 0.83 | 1.00 | 0 .4 8 |

### Example 6. Variant Angina Diagnostic Model Based on Metagenomic Profiling of Bacteria-Derived EVs

EVs were isolated from blood samples of 80 variant angina patients and 80 normal individuals, the two groups matched in age and gender, and then metagenomic sequencing was performed thereon using the method of Example 3. For the development of a diagnostic model, first, a strain exhibiting a p value equal to of 0.05 between two groups in a t-test and a difference of two-fold or more between two groups was selected, and then an AUC, accuracy, sensitivity, and specificity, which are diagnostic performance indexes, were calculated by logistic regression analysis.

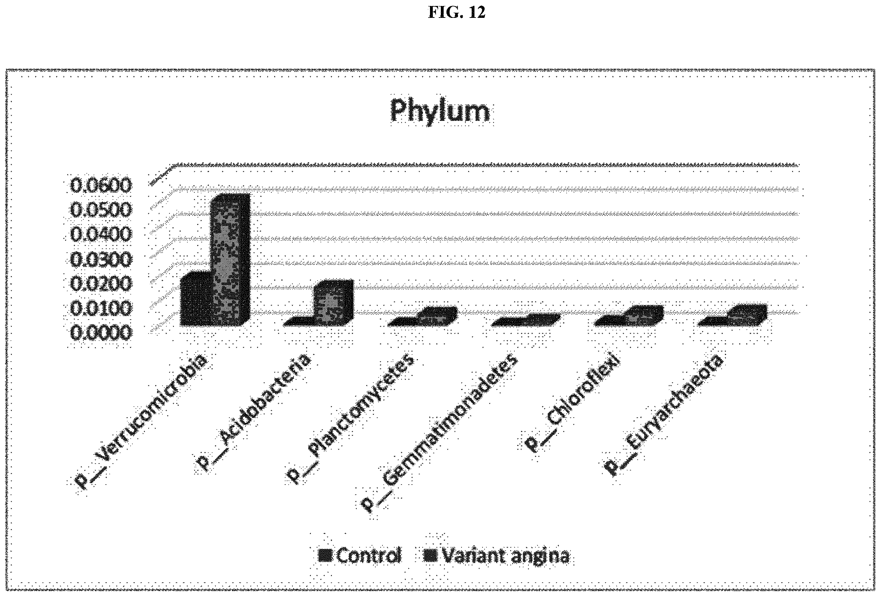

As a result of analyzing bacteria-derived EVs in blood at a phylum level, a diagnostic model developed using, as a biomarker, one or more bacteria from the phylum *Verrucomicrobia*, the phylum *Acidobacteria,* the phylum *Planctomycetes,* the phylum *Gemmatimonadetes,* the phylum *Chloroflexi,* and the phylum *Euryarchaeota* exhibited significant diagnostic performance for variant angina (see Table 12 and FIG. 12).

**[Table 12]**

| | Control | | variant angina | | t-tes t | | Training Set | | | | Test Set | | | |
|---|---|---|---|---|---|---|---|---|---|---|---|---|---|---|
| Taxon | Mea n | SD | Mea n | SD | p-va luc | Ratio | AU C | Acu rrac y | Sensit ivity | Specif icity | AU C | Acu rrac y | Sens itivit y | s p e ci fi ci ty |
| p_Verr ucomicr obia | 0.01 90 | 0.02 19 | 0.05 06 | 0.05 50 | 0.00 00 | 2.66 | 0.7 3 | 0.65 | 0.78 | 0.53 | 0.5 6 | 0.56 | 0.76 | 0. 3 5 |
| p_Chlo roflexi | 0.00 12 | 0.00 49 | 0.00 52 | 0.01 15 | 0.00 56 | 4.36 | 0.6 2 | 0.58 | 0.82 | 0.35 | 0.6 4 | 0.65 | 0.84 | 0. 4 3 |
| p_Gem matimon adetes | 0.00 03 | 0.00 11 | 0.00 18 | 0.00 34 | 0.00 03 | 6.15 | 0.6 5 | 0.65 | 0.91 | 0.40 | 0.5 9 | 0.63 | 0.92 | 0. 3 0 |
| p_Eury archaeot a | 0.00 07 | 0.00 17 | 0.00 55 | 0.01 48 | 0.00 49 | 7.98 | 0.5 4 | 0.54 | 0.71 | 0.37 | 0.6 0 | 0.52 | 0.60 | 0. 4 3 |
| p_Plan ctomyce tes | 0.00 04 | 0.00 18 | 0.00 47 | 0.01 05 | 0.00 05 | 13.28 | 0.6 8 | 0.63 | 0.87 | 0.39 | 0.6 9 | 0.71 | 0.92 | 0. 4 8 |
| p_Acid obacteri a | 0.00 07 | 0.00 20 | 0.01 54 | 0.02 84 | 0.00 00 | 21.19 | 0.7 1 | 0.67 | 0.89 | 0.46 | 0.7 9 | 0.81 | 0.96 | 0. 6 5 |

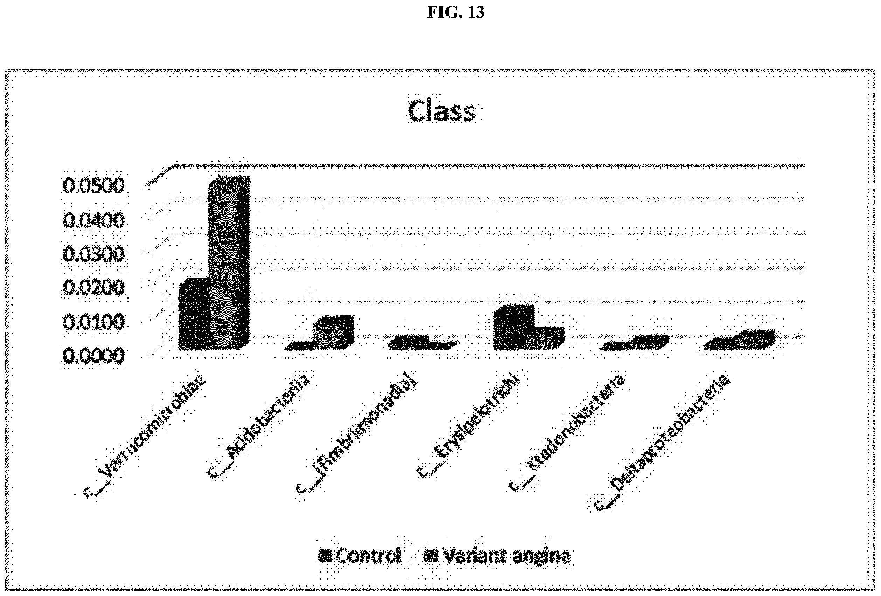

As a result of analyzing bacteria-derived EVs in blood at a class level, a diagnostic model developed using, as a biomarker, one or more bacteria from the class *Verrucomicrobiae,* the class *Acidobacteriia,* the class *Fimbriimonadia,* the class *Erysipelotrichi,* the class *Ktedonobacteria,* and the class *Deltaproteobacteria* exhibited significant diagnostic performance for variant angina (see Table 13 and FIG. 13).

**[Table 13]**

| | Control | | variant angina | | t-tes t | | Training Set | | | | Test Set | | | |
|---|---|---|---|---|---|---|---|---|---|---|---|---|---|---|
| Taxon | Me an | SD | Me an | SD | p-v alue | Rati o | AU C | Acu rrac y | Sensi tivity | Speci ficity | AU C | Acu rrac y | Sensi tivity | s p e ci fi ci ty |
| c_[Fimbr iimonadia ] | 0.0 019 | 0.0 054 | 0.0 001 | 0.0 002 | 0.0 030 | 0.03 | 0.6 5 | 0.6 2 | 0.27 | 0.96 | 0.6 2 | 0.6 3 | 0.38 | 0. 8 8 |
| c_Erysip elotrichi | 0.0 109 | 0.0 158 | 0.0 044 | 0.0 062 | 0.0 011 | 0.41 | 0.6 3 | 0.5 8 | 0.45 | 0.71 | 0.8 0 | 0.7 3 | 0.71 | 0. 7 5 |
| c_Verruc omicrobia e | 0.0 188 | 0.0 218 | 0.0 480 | 0.0 550 | 0.0 000 | 2.55 | 0.6 8 | 0.6 3 | 0.80 | 0.46 | 0.5 3 | 0.5 6 | 0.71 | 0. 4 2 |
| c_Dcltap roteobacte ria | 0.0 012 | 0.0 027 | 0.0 036 | 0.0 073 | 0.0 077 | 2.99 | 0.5 9 | 0.5 7 | 0.77 | 0.38 | 0.6 5 | 0.5 6 | 0.75 | 0. 3 8 |
| c_Ktedo nobacteria | 0.0 000 | 0.0 002 | 0.0 020 | 0.0 064 | 0.0 073 | 78.5 3 | 0.6 1 | 0.5 6 | 0.93 | 0.20 | 0.6 3 | 0.6 5 | 0.96 | 0. 3 3 |
| c_Acido bacteriia | 0.0 001 | 0.0 004 | 0.0 076 | 0.0 160 | 0.0 001 | 128. 78 | 0.6 7 | 0.6 3 | 0.95 | 0.32 | 0.7 6 | 0.7 5 | 0.92 | 0. 5 8 |

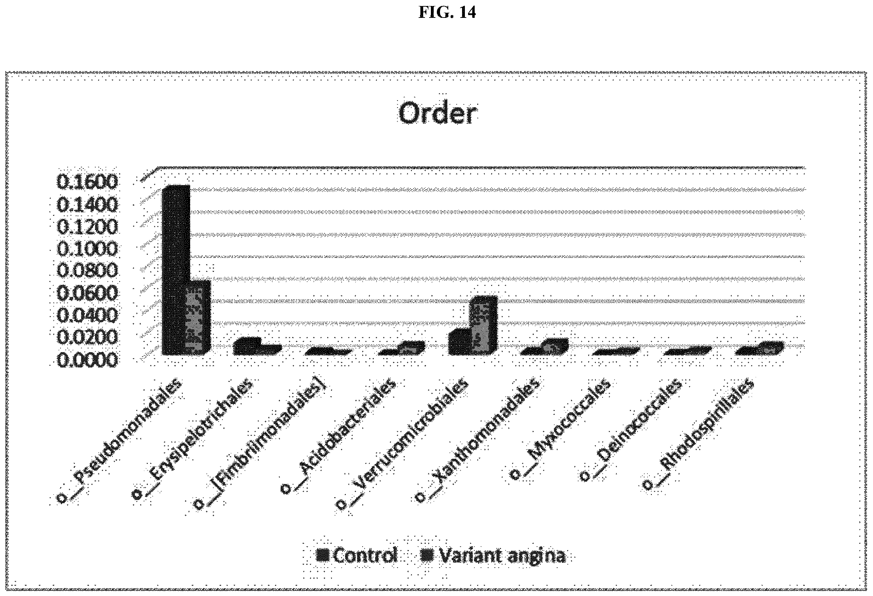

As a result of analyzing bacteria-derived EVs in blood at an order level, a diagnostic model developed using, as a biomarker, one or more bacteria from the order *Pseudomonadales,* the order *Erysipelotrichales,* the order *Fimbriimonadales,* the order *Acidobacteriales,* the order *Verrucomicrobiales,* the order *Xanthomonadales,* the order *Myxococcales,* the order *Deinococcales,* and the order *Rhodospirillales* exhibited significant diagnostic performance for variant angina (see Table 14 and FIG. 14).

**[Table 14]**

| | Control | | variant angina | | t-tes t | | Training Set | | | | Test Set | | | |
|---|---|---|---|---|---|---|---|---|---|---|---|---|---|---|
| Taxon | Mea n | SD | Mea n | SD | p-va lue | Rati o | AU C | Acu rrac y | Sens itivit y | Spec ificit y | AU C | Acu rrac y | Sensi tivity | S p e ci fi ci ty |
| o_[Fimbr iimonadalc s] | 0.00 19 | 0.00 54 | 0.00 01 | 0.00 02 | 0.00 30 | 0.03 | 0.69 | 0.66 | 0.53 | 0.81 | 0.53 | 0.52 | 0.30 | 0. 6 8 |
| o_Erysip elotrichale s | 0.01 09 | 0.01 58 | 0.00 44 | 0.00 62 | 0.00 11 | 0.41 | 0.70 | 0.66 | 0.65 | 0.67 | 0.56 | 0.63 | 0.45 | 0. 7 5 |
| o_Pseudo monadales | 0.14 79 | 0.11 76 | 0.06 20 | 0.05 14 | 0.00 00 | 0.42 | 0.83 | 0.74 | 0.72 | 0.77 | 0.62 | 0.56 | 0.50 | 0. 6 1 |
| o_Verruc omicrobial es | 0.01 88 | 0.02 18 | 0.04 80 | 0.05 50 | 0.00 00 | 2.55 | 0.67 | 0.65 | 0.82 | 0.46 | 0.65 | 0.60 | 0.95 | 0. 3 6 |
| o_Rhodo spirillales | 0.00 23 | 0.00 53 | 0.00 71 | 0.01 42 | 0.00 56 | 3.07 | 0.59 | 0.60 | 0.85 | 0.31 | 0.63 | 0.52 | 0.80 | 0. 3 2 |
| o_Deinoc occales | 0.00 06 | 0.00 16 | 0.00 25 | 0.00 61 | 0.00 85 | 4.32 | 0.62 | 0.60 | 0.82 | 0.35 | 0.51 | 0.44 | 0.75 | 0. 2 5 |
| o_Xantho monadales | 0.00 21 | 0.00 48 | 0.01 01 | 0.02 08 | 0.00 11 | 4.94 | 0.65 | 0.61 | 0.90 | 0.27 | 0.68 | 0.60 | 0.95 | 0. 3 6 |
| o_Myxoc occales | 0.00 01 | 0.00 03 | 0.00 17 | 0.00 37 | 0.00 02 | 20.1 9 | 0.64 | 0.67 | 0.93 | 0.37 | 0.59 | 0.58 | 0.95 | 0. 3 2 |
| o_Acidob acteriales | 0.00 01 | 0.00 04 | 0.00 76 | 0.01 60 | 0.00 01 | 128. 78 | 0.68 | 0.71 | 0.98 | 0.40 | 0.63 | 0.60 | 0.95 | 0. 3 6 |

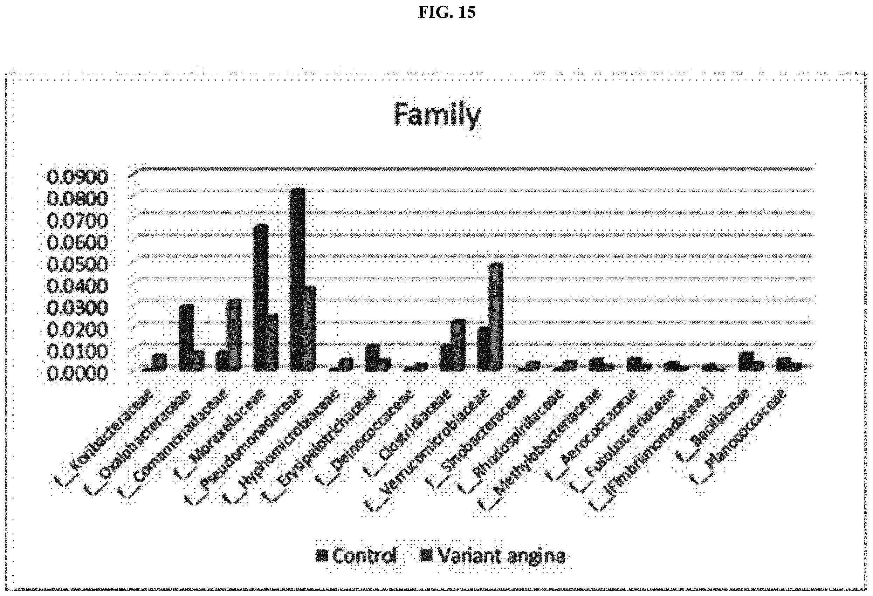

As a result of analyzing bacteria-derived EVs in blood at a family level, a diagnostic model developed using, as a biomarker, one or more bacteria from the family *Koribacteraceae,* the family *Oxalobacteraceae,* the family *Comamonadaceae,* the family *Moraxellaceae,* the family *Pseudomonadaceae,* the family *Hyphomicrobiaceae,* the family *Erysipelotrichaceae,* the family *Deinococcaceae,* the family *Clostridiaceae,* the family *Verrucomicrobiaceae,* the family *Sinobacteraceae,* the family *Rhodospirillaceae,* the family *Methylobacteriaceae,* the family *Aerococcaceae,* the family *Fusobacteriaceae,* the family *Fimbriimonadaceae,* the family *Bacillaceae,* and the family *Planococcaceae* exhibited significant diagnostic performance for variant angina (see Table 15 and FIG. 15).

**[Table 15]**

| | Control | | variant angina | | t-te st | | Training Set | | | | Test Set | | | |
|---|---|---|---|---|---|---|---|---|---|---|---|---|---|---|
| Taxon | Me an | SD | Me an | SD | p-v alue | Rati o | AU C | Acu rrac y | Sens itivit y | Speci ficity | AU C | Acu rrac y | Sensi tivity | Sp ec ifi cit y |
| f_[Fimbr iimonadac eae] | 0.0 019 | 0.0 054 | 0.0 001 | 0.0 002 | 0.0 030 | 0.03 | 0.6 2 | 0.5 9 | 0.32 | 0.86 | 0.6 7 | 0.6 7 | 0.50 | 0. 83 |
| f_Oxalob acteraceae | 0.0 291 | 0.0 505 | 0.0 081 | 0.0 103 | 0.0 005 | 0.28 | 0.7 3 | 0.6 6 | 0.57 | 0.75 | 0.6 3 | 0.5 8 | 0.38 | 0. 79 |
| f_Fusoba cteriaceae | 0.0 032 | 0.0 058 | 0.0 010 | 0.0 019 | 0.0 019 | 0.32 | 0.6 2 | 0.5 5 | 0.45 | 0.66 | 0.6 0 | 0.5 8 | 0.46 | 0. 71 |
| f_Aeroco ccaceae | 0.0 052 | 0.0 101 | 0.0 018 | 0.0 033 | 0.0 054 | 0.35 | 0.6 3 | 0.6 2 | 0.41 | 0.82 | 0.5 5 | 0.5 8 | 0.42 | 0. 75 |
| f_Morax ellaceae | 0.0 657 | 0.0 776 | 0.0 245 | 0.0 334 | 0.0 000 | 0.37 | 0.7 2 | 0.7 0 | 0.59 | 0.80 | 0.8 1 | 0.7 1 | 0.58 | 0. 83 |
| f_Methyl obacteriac eae | 0.0 048 | 0.0 062 | 0.0 020 | 0.0 048 | 0.0 014 | 0.41 | 0.6 6 | 0.6 2 | 0.43 | 0.80 | 0.7 1 | 0.6 9 | 0.58 | 0. 79 |
| f_Erysip elotrichac eae | 0.0 109 | 0.0 158 | 0.0 044 | 0.0 062 | 0.0 011 | 0.41 | 0.7 0 | 0.6 6 | 0.57 | 0.75 | 0.6 1 | 0.6 3 | 0.63 | 0. 63 |
| f_Bacilla ceae | 0.0 075 | 0.0 107 | 0.0 032 | 0.0 043 | 0.0 014 | 0.43 | 0.6 1 | 0.6 0 | 0.45 | 0.75 | 0.6 4 | 0.5 4 | 0.54 | 0. 54 |
| f_Pseudo monadace ae | 0.0 822 | 0.0 753 | 0.0 374 | 0.0 319 | 0.0 000 | 0.46 | 0.7 0 | 0.7 0 | 0.61 | 0.79 | 0.7 4 | 0.6 7 | 0.58 | 0. 75 |
| f_Planoc occaceae | 0.0 050 | 0.0 074 | 0.0 024 | 0.0 035 | 0.0 057 | 0.48 | 0.6 0 | 0.5 7 | 0.45 | 0.70 | 0.6 9 | 0.6 9 | 0.54 | 0. 83 |
| f_Clostri diaceae | 0.0 110 | 0.0 163 | 0.0 223 | 0.0 230 | 0.0 005 | 2.03 | 0.6 8 | 0.6 6 | 0.73 | 0.59 | 0.6 0 | 0.5 8 | 0.71 | 0. 46 |
| f_Verruc omicrobia ceae | 0.0 188 | 0.0 218 | 0.0 480 | 0.0 550 | 0.0 000 | 2.55 | 0.6 7 | 0.6 2 | 0.79 | 0.45 | 0.5 8 | 0.5 8 | 0.71 | 0. 46 |
| f_Coma monadace ae | 0.0 081 | 0.0 109 | 0.0 317 | 0.0 529 | 0.0 002 | 3.93 | 0.7 3 | 0.6 9 | 0.79 | 0.59 | 0.6 3 | 0.5 4 | 0.63 | 0. 46 |
| f_Deinoc occaccac | 0.0 005 | 0.0 014 | 0.0 023 | 0.0 060 | 0.0 088 | 4.89 | 0.6 9 | 0.6 3 | 0.88 | 0.38 | 0.4 2 | 0.4 6 | 0.67 | 0. 25 |
| f_Rhodo spirillacea c | 0.0 005 | 0.0 017 | 0.0 036 | 0.0 077 | 0.0 006 | 7.62 | 0.6 7 | 0.5 7 | 0.75 | 0.39 | 0.4 7 | 0.5 4 | 0.75 | 0. 33 |
| f_Hypho microbiac eae | 0.0 001 | 0.0 005 | 0.0 046 | 0.0 122 | 0.0 016 | 38.8 7 | 0.7 0 | 0.6 3 | 0.89 | 0.36 | 0.4 6 | 0.5 2 | 0.79 | 0. 25 |
| f_Sinoba cteraceae | 0.0 001 | 0.0 003 | 0.0 033 | 0.0 095 | 0.0 033 | 58.8 7 | 0.6 7 | 0.6 2 | 0.84 | 0.39 | 0.5 7 | 0.6 0 | 0.88 | 0. 33 |
| f_Koriba cteraceae | 0.0 001 | 0.0 004 | 0.0 067 | 0.0 142 | 0.0 001 | 118. 33 | 0.7 5 | 0.6 7 | 0.91 | 0.43 | 0.5 3 | 0.6 0 | 0.92 | 0. 29 |

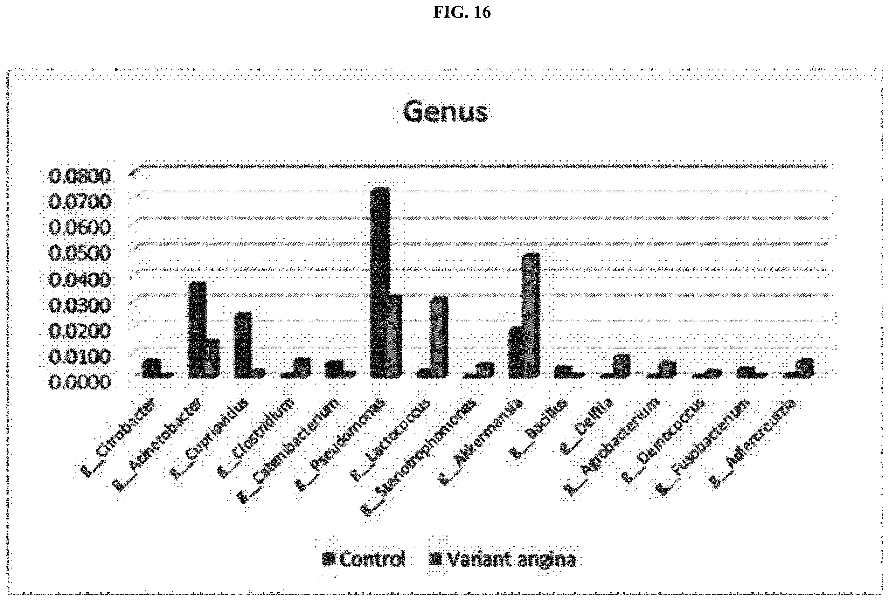

As a result of analyzing bacteria-derived EVs in blood at a genus level, a diagnostic model developed using, as a biomarker, one or more bacteria from the genus *Citrobacter,* the genus *Acinetobacter,* the genus *Cupriavidus*, the genus *Clostridium,* the genus *Catenibacterium,* the genus *Pseudomonas*, the genus *Lactococcus,* the genus *Stenotrophomonas,* the genus *Akkermansia,* the genus *Bacillus,* the genus *Delftia,* the genus *Agrobacterium,* the genus *Deinococcus,* the genus *Fusobacterium,* and the genus *Adlercreutzia* exhibited significant diagnostic performance for variant angina (see Table 16 and FIG. 16).

**[Table 16]**

| | Control | | variant angina | | t-tes t | | Training Set | | | | Test Set | | | |
|---|---|---|---|---|---|---|---|---|---|---|---|---|---|---|
| Taxon | Me an | SD | Me an | SD | p-v alue | Rati o | AU C | Acu rrac y | Sensi tivity | Speci ficity | AU C | Acu rrac y | Sensi tivity | Sp eci fic ity |
| g_Citro bacter | 0.00 63 | 0.00 80 | 0.00 09 | 0.00 18 | 0.00 00 | 0.15 | 0.81 | 0.71 | 0.54 | 0.91 | 0.69 | 0.65 | 0.57 | 0. 72 |
| g_Acin etobacter | 0.03 62 | 0.05 22 | 0.01 38 | 0.02 52 | 0.00 08 | 0.38 | 0.77 | 0.73 | 0.67 | 0.80 | 0.74 | 0.73 | 0.70 | 0. 76 |
| g_Cupri avidus | 0.02 42 | 0.04 92 | 0.00 24 | 0.00 40 | 0.00 02 | 0.10 | 0.74 | 0.68 | 0.56 | 0.80 | 0.86 | 0.81 | 0.61 | 1. 00 |
| g_Clost ridium | 0.00 11 | 0.00 24 | 0.00 66 | 0.00 87 | 0.00 00 | 6.11 | 0.73 | 0.73 | 0.95 | 0.51 | 0.60 | 0.65 | 0.87 | 0. 44 |
| g_Caten ibacteriu m | 0.00 58 | 0.01 22 | 0.00 16 | 0.00 39 | 0.00 46 | 0.28 | 0.72 | 0.67 | 0.54 | 0.80 | 0.69 | 0.73 | 0.65 | 0. 80 |
| g_Pseu domonas | 0.07 27 | 0.06 49 | 0.03 13 | 0.03 08 | 0.00 00 | 0.43 | 0.71 | 0.71 | 0.68 | 0.75 | 0.79 | 0.75 | 0.65 | 0. 84 |
| g_Lacto coccus | 0.00 26 | 0.00 48 | 0.03 03 | 0.07 10 | 0.00 09 | 11.6 4 | 0.69 | 0.62 | 0.82 | 0.40 | 0.46 | 0.54 | 0.83 | 0. 28 |
| g_Steno trophom onas | 0.00 05 | 0.00 21 | 0.00 50 | 0.01 36 | 0.00 51 | 10.0 1 | 0.66 | 0.64 | 0.93 | 0.35 | 0.50 | 0.54 | 0.91 | 0. 20 |
| g_Akke rmansia | 0.01 88 | 0.02 19 | 0.04 74 | 0.05 48 | 0.00 00 | 2.53 | 0.66 | 0.65 | 0.77 | 0.53 | 0.59 | 0.60 | 0.74 | 0. 48 |
| g_Bacil lus | 0.00 37 | 0.00 74 | 0.00 11 | 0.00 26 | 0.00 38 | 0.29 | 0.65 | 0.62 | 0.47 | 0.76 | 0.58 | 0.63 | 0.43 | 0. 80 |
| g_Delfti a | 0.00 07 | 0.00 35 | 0.00 82 | 0.02 24 | 0.00 39 | 12.3 0 | 0.64 | 0.59 | 0.82 | 0.35 | 0.46 | 0.46 | 0.74 | 0. 20 |
| g_Agro bacteriu m | 0.00 05 | 0.00 14 | 0.00 56 | 0.01 31 | 0.00 10 | 10.7 3 | 0.62 | 0.54 | 0.84 | 0.24 | 0.58 | 0.58 | 0.91 | 0. 28 |
| g_Dein ococcus | 0.00 05 | 0.00 14 | 0.00 23 | 0.00 60 | 0.00 88 | 4.89 | 0.60 | 0.59 | 0.86 | 0.31 | 0.57 | 0.60 | 0.87 | 0. 36 |
| g_Fuso bacteriu m | 0.00 32 | 0.00 58 | 0.00 10 | 0.00 19 | 0.00 17 | 0.31 | 0.59 | 0.53 | 0.35 | 0.71 | 0.60 | 0.58 | 0.43 | 0. 72 |
| g_Adler creutzia | 0.00 11 | 0.00 26 | 0.00 63 | 0.01 47 | 0.00 26 | 5.62 | 0.58 | 0.52 | 0.67 | 0.36 | 0.55 | 0.52 | 0.70 | 0. 36 |

### Example 7. Atrial Fibrillation Diagnostic Model Based on Metagenomic Profiling of Bacteria-Derived EVs

EVs were isolated from blood samples of 34 atrial fibrillation patients and 62 normal individuals, the two groups matched in age and gender, and then metagenomic sequencing was performed thereon using the method of Example 3. For the development of a diagnostic model, first, a strain exhibiting a p value of less than 0.05 between two groups in a t-test and a difference of two-fold or more between two groups was selected, and then an AUC, accuracy, sensitivity, and specificity, which are diagnostic performance indexes, were calculated by logistic regression analysis.

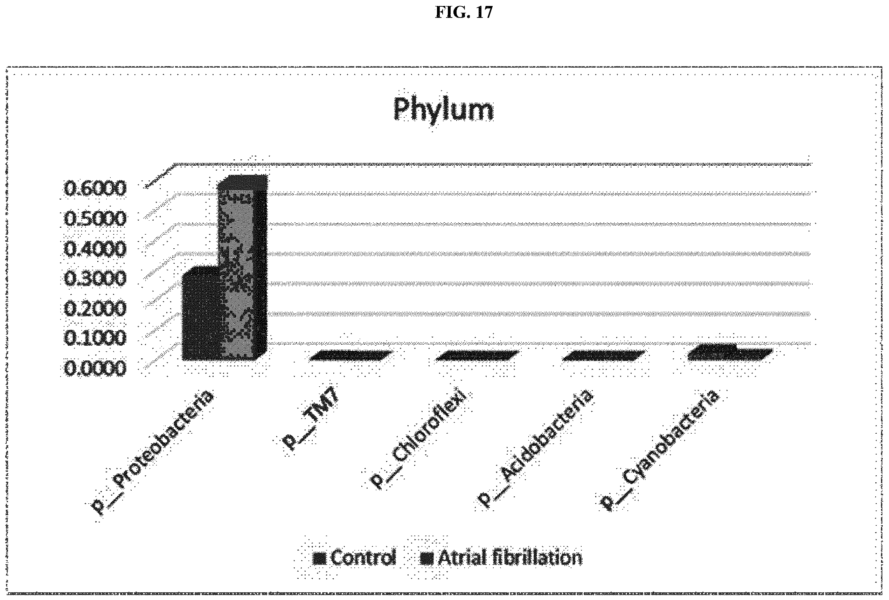

As a result of analyzing bacteria-derived EVs in blood at a phylum level, a diagnostic model developed using, as a biomarker, one or more bacteria from the phylum *Proteobacteria,* the phylum TM7, the phylum *Chloroflexi,* the phylum *Acidobacteria,* and the phylum *Cyanobacteria* exhibited significant diagnostic performance for atrial fibrillation (see Table 17 and FIG. 17).

**[Table 17]**

| | Control | | Atrial fibrillatio n | | t-tes t | | Training Set | | | | Test Set | | | |
|---|---|---|---|---|---|---|---|---|---|---|---|---|---|---|
| Taxon | Mea n | SD | Mea n | SD | p-va lue | Rati o | AU C | Acu rrac y | Sensi tivity | Speci ficity | AU C | Acur racy | Sensi tivity | s p ec ifi ci ty |
| p_Chl oroflexi | 0.00 07 | 0.00 17 | 0.00 00 | 0.00 02 | 0.00 32 | 0.06 | 0.6 6 | 0.60 | 0.95 | 0.04 | 0.63 | 0.76 | 0.91 | 0. 1 7 |
| p_Aci dobacte ria | 0.00 08 | 0.00 27 | 0.00 01 | 0.00 03 | 0.03 46 | 0.07 | 0.6 6 | 0.60 | 0.90 | 0.12 | 0.59 | 0.72 | 0.83 | 0. 3 3 |
| p_TM 7 | 0.00 21 | 0.00 43 | 0.00 02 | 0.00 08 | 0.00 10 | 0.09 | 0.7 0 | 0.67 | 0.85 | 0.38 | 0.56 | 0.62 | 0.74 | 0. 1 7 |
| p_Cya nobacte ria | 0.02 25 | 0.03 98 | 0.00 63 | 0.00 67 | 0.00 25 | 0.28 | 0.6 4 | 0.64 | 0.85 | 0.31 | 0.56 | 0.72 | 0.87 | 0. 1 7 |
| p_Prot eobacte ria | 0.28 33 | 0.10 40 | 0.58 66 | 0.20 46 | 0.00 00 | 2.07 | 0.8 6 | 0.88 | 0.95 | 0.77 | 0.85 | 0.97 | 1.00 | 0. 8 3 |

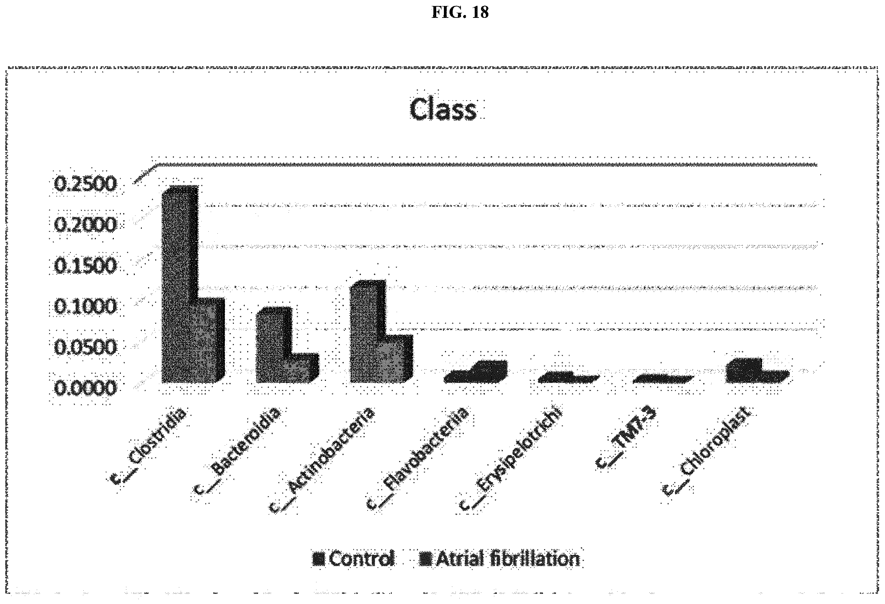

As a result of analyzing bacteria-derived EVs in blood at a class level, a diagnostic model developed using, as a biomarker, one or more bacteria from the class *Clostridia,* the class *Bacteroidia,* the class *Actinobacteria,* the class *Flavobacteriia,* the class *Erysipelotrichi,* the class TM7-3, and the class *Chloroplast* exhibited significant diagnostic performance for atrial fibrillation (see Table 18 and FIG. 18).

**[Table 18]**

| | Control | | Atrial fibrillatio n | | t-tes t | | Training Set | | | | Test Set | | | |
|---|---|---|---|---|---|---|---|---|---|---|---|---|---|---|
| Taxon | Mea n | SD | Mea n | SD | p-va lue | Rati o | AU C | Acu rrac y | Sensi tivity | Speci ficity | AU C | Acu rrac y | Sensi tivity | S p ec ifi ci ty |
| c_TM 7-3 | 0.00 20 | 0.00 43 | 0.00 02 | 0.00 08 | 0.00 14 | 0.09 | 0.68 | 0.70 | 1.00 | 0.05 | 0.56 | 0.57 | 0.83 | 0. 1 8 |
| c_Ery sipelot richi | 0.00 48 | 0.00 73 | 0.00 04 | 0.00 07 | 0.00 00 | 0.09 | 0.78 | 0.72 | 0.80 | 0.52 | 0.55 | 0.48 | 0.67 | 0. 1 8 |
| c_Chl oroplas t | 0.02 15 | 0.04 00 | 0.00 59 | 0.00 68 | 0.00 37 | 0.27 | 0.65 | 0.67 | 0.93 | 0.10 | 0.44 | 0.52 | 0.83 | 0. 0 0 |
| c_Ba cteroid ia | 0.08 38 | 0.04 66 | 0.02 78 | 0.02 25 | 0.00 00 | 0.33 | 0.85 | 0.76 | 0.83 | 0.62 | 0.89 | 0.86 | 0.89 | 0. 8 2 |
| c_Clo stridia | 0.22 98 | 0.11 46 | 0.09 46 | 0.05 89 | 0.00 00 | 0.41 | 0.86 | 0.84 | 0.91 | 0.67 | 0.88 | 0.79 | 0.89 | 0. 6 4 |
| c_Act inobact eria | 0.11 64 | 0.07 41 | 0.04 96 | 0.04 26 | 0.00 00 | 0.43 | 0.79 | 0.78 | 0.87 | 0.57 | 0.84 | 0.83 | 0.94 | 0. 6 4 |
| c_Fla vobact eriia | 0.00 68 | 0.01 59 | 0.01 88 | 0.01 25 | 0.00 03 | 2.78 | 0.79 | 0.75 | 0.96 | 0.29 | 0.80 | 0.69 | 0.89 | 0. 3 6 |

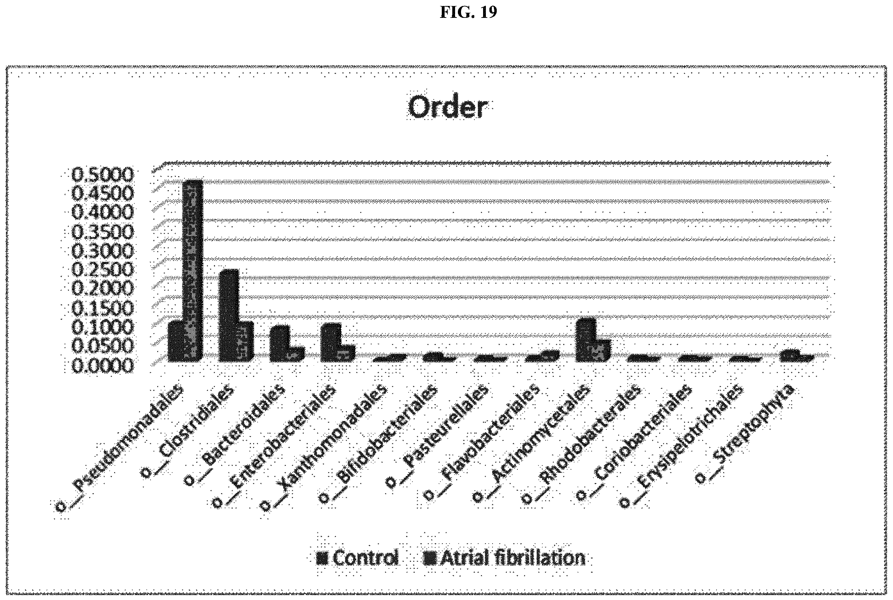

As a result of analyzing bacteria-derived EVs in blood at an order level, a diagnostic model developed using, as a biomarker, one or more bacteria from the order *Pseudomonadales,* the order *Clostridiales,* the order *Bacteroidales,* the order *Enterobacteriales,* the order *Xanthomonadales,* the order *Bifidobacteriales,* the order *Pasteurellales,* the order *Flavobacteriales,* the order *Actinomycetales,* the order *Rhodobacterales,* the order *Coriobacteriales,* the order *Erysipelotrichales,* and the order *Streptophyta* exhibited significant diagnostic performance for atrial fibrillation (see Table 19 and FIG. 19).

**[Table 19]**

| | Control | | Atrial fibrillatio n | | t-tes t | | Training Set | | | | Test Set | | | |
|---|---|---|---|---|---|---|---|---|---|---|---|---|---|---|
| Taxon | Mea n | SD | Mea n | SD | p-va lue | Rati o | AU C | Acu rrac y | Sensi tivity | Speci ficity | AU C | Acu rrac y | Sensi tivity | S p ec ifi ci ty |
| o_Erysi pelotricha les | 0.00 48 | 0.00 73 | 0.00 04 | 0.00 07 | 0.00 00 | 0.09 | 0.7 0 | 0.63 | 0.70 | 0.48 | 0.81 | 0.72 | 0.85 | 0. 4 4 |
| o_Bifid obacterial es | 0.01 36 | 0.01 54 | 0.00 18 | 0.00 20 | 0.00 00 | 0.13 | 0.8 3 | 0.73 | 0.77 | 0.65 | 0.72 | 0.66 | 0.75 | 0. 4 4 |
| o_Strept ophyta | 0.02 14 | 0.04 01 | 0.00 59 | 0.00 68 | 0.00 38 | 0.28 | 0.6 4 | 0.67 | 0.91 | 0.22 | 0.51 | 0.59 | 0.70 | 0. 3 3 |
| o_Rhod obacteral es | 0.00 81 | 0.01 44 | 0.00 26 | 0.00 45 | 0.00 71 | 0.32 | 0.7 3 | 0.69 | 0.89 | 0.30 | 0.53 | 0.59 | 0.65 | 0. 4 4 |
| o_Paste urellales | 0.00 61 | 0.00 87 | 0.00 20 | 0.00 37 | 0.00 21 | 0.33 | 0.7 7 | 0.69 | 0.89 | 0.30 | 0.56 | 0.62 | 0.70 | 0. 4 4 |
| o_Bacte roidales | 0.08 38 | 0.04 66 | 0.02 78 | 0.02 25 | 0.00 00 | 0.33 | 0.8 9 | 0.78 | 0.82 | 0.70 | 0.84 | 0.79 | 0.85 | 0. 6 7 |
| o_Enter obacterial es | 0.09 02 | 0.05 65 | 0.03 26 | 0.03 90 | 0.00 00 | 0.36 | 0.8 6 | 0.78 | 0.80 | 0.74 | 0.83 | 0.72 | 0.65 | 0. 8 9 |
| o_Clostr idiales | 0.22 94 | 0.11 44 | 0.09 46 | 0.05 89 | 0.00 00 | 0.41 | 0.8 9 | 0.81 | 0.86 | 0.70 | 0.81 | 0.76 | 0.80 | 0. 6 7 |
| o_Corio bacteriale s | 0.00 70 | 0.00 87 | 0.00 31 | 0.00 61 | 0.01 54 | 0.45 | 0.7 1 | 0.61 | 0.91 | 0.04 | 0.50 | 0.59 | 0.80 | 0. 1 1 |
| o_Actin omycetal es | 0.10 28 | 0.07 48 | 0.04 79 | 0.04 29 | 0.00 00 | 0.47 | 0.7 4 | 0.70 | 0.84 | 0.43 | 0.75 | 0.62 | 0.65 | 0. 5 6 |
| o_Flavo bacteriale s | 0.00 68 | 0.01 59 | 0.01 88 | 0.01 25 | 0.00 03 | 2.78 | 0.7 6 | 0.84 | 0.91 | 0.70 | 0.84 | 0.83 | 0.85 | 0. 7 8 |
| o_Xanth omonadal es | 0.00 23 | 0.00 43 | 0.01 03 | 0.00 71 | 0.00 00 | 4.57 | 0.8 4 | 0.79 | 0.91 | 0.57 | 0.88 | 0.93 | 1.00 | 0. 7 8 |
| o_Pseud omonadal es | 0.09 71 | 0.08 62 | 0.46 22 | 0.23 98 | 0.00 00 | 4.76 | 0.8 9 | 0.88 | 0.98 | 0.70 | 0.87 | 0.90 | 0.95 | 0. 7 8 |

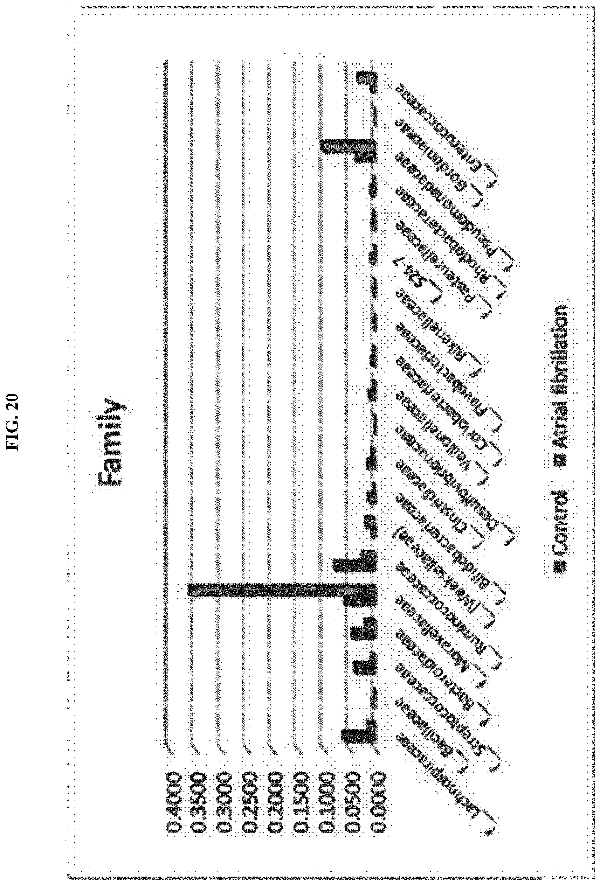

As a result of analyzing bacteria-derived EVs in blood at a family level, a diagnostic model developed using, as a biomarker, one or more bacteria from the family *Lachnospiraceae,* the family *Bacillaceae,* the family *Streptococcaceae,* the family *Bacteroidaceae,* the family *Moraxellaceae,* the family *Ruminococcaceae,* the family *Weeksellaceae,* the family *Bifidobacteriaceae,* the family *Clostridiaceae,* the family *Desulfovibrionaceae,* the family *Veillonellaceae,* the family *Coriobacteriaceae,* the family *Flavobacteriaceae,* the family *Rikenellaceae,* the family S24-7, the family *Pasteurellaceae,* the family *Rhodobacteraceae,* the family *Pseudomonadaceae,* the family *Gordoniaceae,* and the family *Enterococcaceae* exhibited significant diagnostic performance for atrial fibrillation (see Table 20 and FIG. 20).

**[Table 20]**

| | Control | | Atrial fibrillatio n | | t-tes t | | Training Set | | | | Test Set | | | |
|---|---|---|---|---|---|---|---|---|---|---|---|---|---|---|
| Taxon | Me an | SD | Me an | SD | p-v alue | Rati o | AU C | Acu rrac y | Sens itivit y | Spec ificit y | AU C | Acu rrac y | Sens itivit y | Sp ec ifi cit y |
| f_Desulf ovibrionac eae | 0.00 11 | 0.00 29 | 0.00 00 | 0.00 00 | 0.02 87 | 0.00 | 0.78 | 0.82 | 0.98 | 0.50 | 0.94 | 0.93 | 1.00 | 0. 80 |
| f_S24-7 | 0.00 86 | 0.01 92 | 0.00 01 | 0.00 03 | 0.00 08 | 0.01 | 0.70 | 0.69 | 0.93 | 0.18 | 0.72 | 0.62 | 0.89 | 0. 10 |
| f_Bacilla ceae | 0.00 58 | 0.00 72 | 0.00 05 | 0.00 11 | 0.00 00 | 0.09 | 0.86 | 0.79 | 0.76 | 0.86 | 0.75 | 0.66 | 0.68 | 0. 60 |
| f_Rikenel laceae | 0.00 22 | 0.00 59 | 0.00 02 | 0.00 05 | 0.01 14 | 0.10 | 0.71 | 0.64 | 0.84 | 0.23 | 0.55 | 0.62 | 0.89 | 0. 10 |
| f_Bifidob acteriaceae | 0.01 36 | 0.01 54 | 0.00 18 | 0.00 20 | 0.00 00 | 0.13 | 0.80 | 0.76 | 0.80 | 0.68 | 0.73 | 0.62 | 0.74 | 0. 40 |
| f_Gordon iaceae | 0.00 06 | 0.00 19 | 0.00 01 | 0.00 02 | 0.03 57 | 0.13 | 0.54 | 0.69 | 1.00 | 0.05 | 0.37 | 0.55 | 0.84 | 0. 00 |
| f Veillon ellaceae | 0.01 19 | 0.02 01 | 0.00 20 | 0.00 30 | 0.00 03 | 0.17 | 0.77 | 0.70 | 0.84 | 0.41 | 0.80 | 0.59 | 0.79 | 0. 20 |
| f_Strepto coccaceae | 0.03 95 | 0.06 38 | 0.00 77 | 0.00 62 | 0.00 02 | 0.19 | 0.85 | 0.82 | 0.93 | 0.59 | 0.81 | 0.83 | 0.89 | 0. 70 |
| f_Flavob acteriaceae | 0.00 09 | 0.00 23 | 0.00 02 | 0.00 04 | 0.01 94 | 0.22 | 0.72 | 0.70 | 0.91 | 0.27 | 0.58 | 0.55 | 0.79 | 0. 10 |
| | | | | | | | | | | | | | | |
| f_Lachno spiraceae | 0.06 30 | 0.06 94 | 0.01 53 | 0.00 74 | 0.00 00 | 0.24 | 0.88 | 0.87 | 0.98 | 0.64 | 0.92 | 0.93 | 1.00 | 0. 80 |
| f_Rumin ococcacea e | 0.07 90 | 0.05 12 | 0.02 47 | 0.02 31 | 0.00 00 | 0.31 | 0.83 | 0.78 | 0.80 | 0.73 | 0.87 | 0.76 | 0.89 | 0. 50 |
| f_Rhodo bacteracea e | 0.00 81 | 0.01 44 | 0.00 25 | 0.00 45 | 0.00 70 | 0.32 | 0.67 | 0.63 | 0.91 | 0.05 | 0.59 | 0.69 | 1.00 | 0. 10 |
| f_Pasteur ellaceae | 0.00 61 | 0.00 87 | 0.00 20 | 0.00 37 | 0.00 21 | 0.33 | 0.70 | 0.66 | 0.96 | 0.05 | 0.54 | 0.62 | 0.95 | 0. 00 |
| f_Clostri diaceae | 0.01 54 | 0.02 09 | 0.00 60 | 0.01 17 | 0.00 68 | 0.39 | 0.79 | 0.78 | 0.98 | 0.36 | 0.83 | 0.76 | 1.00 | 0. 30 |
| f_Bactero idaceae | 0.04 45 | 0.03 48 | 0.01 93 | 0.01 77 | 0.00 00 | 0.43 | 0.85 | 0.82 | 0.84 | 0.77 | 0.85 | 0.76 | 0.84 | 0. 60 |
| f_Coriob acteriaceae | 0.00 70 | 0.00 87 | 0.00 31 | 0.00 61 | 0.01 54 | 0.45 | 0.76 | 0.69 | 0.76 | 0.55 | 0.78 | 0.66 | 0.68 | 0. 60 |
| f_Pseudo monadace ae | 0.03 77 | 0.05 87 | 0.10 24 | 0.04 73 | 0.00 00 | 2.72 | 0.58 | 0.73 | 0.96 | 0.27 | 0.38 | 0.60 | 0.89 | 0. 00 |
| f_[Weeks ellaceae] | 0.00 58 | 0.01 55 | 0.01 86 | 0.01 23 | 0.00 01 | 3.19 | 0.81 | 0.72 | 0.84 | 0.45 | 0.78 | 0.76 | 0.84 | 0. 60 |
| f_Enteroc occaceae | 0.00 76 | 0.01 57 | 0.03 31 | 0.01 13 | 0.00 00 | 4.34 | 0.52 | 0.70 | 1.00 | 0.09 | 0.38 | 0.62 | 0.95 | 0. 00 |
| f_Moraxe llaceae | 0.05 94 | 0.06 28 | 0.35 96 | 0.19 72 | 0.00 00 | 6.05 | 0.84 | 0.85 | 0.89 | 0.77 | 0.81 | 0.76 | 0.79 | 0. 70 |

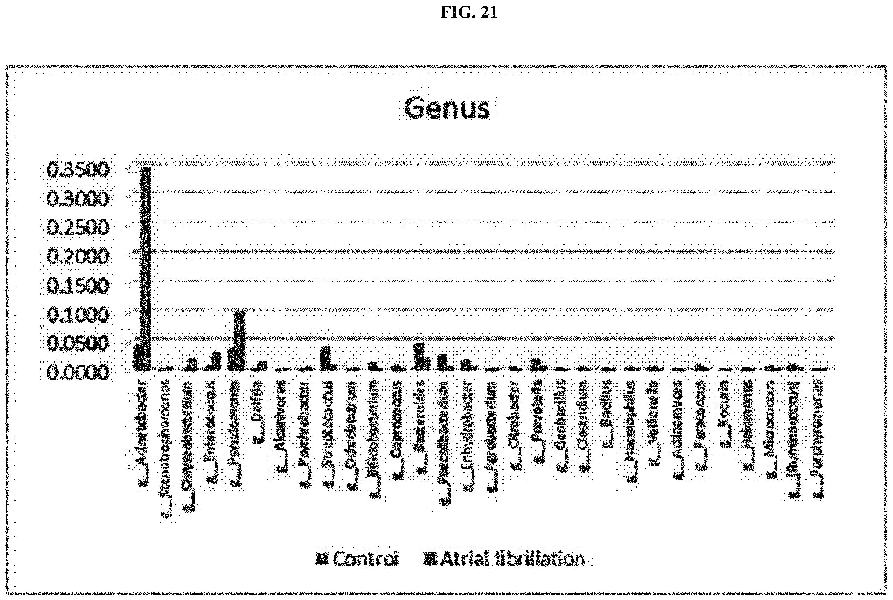

As a result of analyzing bacteria-derived EVs in blood at a genus level, a diagnostic model developed using, as a biomarker, one or more bacteria from the genus *Acinetobacter,* the genus *Stenotrophomonas,* the genus *Chryseobacterium,* the genus *Enterococcus,* the genus *Pseudomonas,* the genus *Delftia,* the genus *Alcanivorax,* the genus *Psychrobacter,* the genus *Streptococcus,* the genus *Ochrobactrum,* the genus *Bifidobacterium,* the genus *Coprococcus,* the genus *Bacteroides,* the genus *Faecalibacterium,* the genus *Enhydrobacter,* the genus *Agrobacterium,* the genus *Citrobacter,* the genus *Prevotella,* the genus *Geobacillus,* the genus *Clostridium,* the genus *Bacillus,* the genus *Haemophilus,* the genus *Veillonella,* the genus *Actinomyces,* the genus *Paracoccus,* the genus *Kocuria,* the genus *Halomonas,* the genus *Micrococcus,* the genus *Ruminococcus,* and the genus *Porphyromonas* exhibited significant diagnostic performance for atrial fibrillation (see Table 21 and FIG. 21).

**[Table 21]**

| | Control | | Atrial fibrillatio n | | t-tes t | | Training Set | | | | Test Set | | | |
|---|---|---|---|---|---|---|---|---|---|---|---|---|---|---|
| Taxon | Mea n | SD | Mea n | SD | p-va lue | Ratio | AU C | Acu rrac y | Sensi tivity | Spec ificit y | AU C | Acu rrac y | Sensi tivity | s P e ci fi ci t y |
| g_Geoba cillus | 0.00 30 | 0.00 66 | 0.00 00 | 0.00 00 | 0.01 32 | 0.00 | 0.73 | 0.66 | 0.79 | 0.42 | 0.71 | 0.69 | 0.86 | 0 .2 5 |
| g_Citrob acter | 0.00 48 | 0.01 18 | 0.00 03 | 0.00 08 | 0.00 34 | 0.06 | 0.75 | 0.69 | 0.81 | 0.46 | 0.45 | 0.48 | 0.62 | 0 .1 3 |
| g_Copro coccus | 0.00 67 | 0.00 75 | 0.00 08 | 0.00 11 | 0.00 00 | 0.12 | 0.78 | 0.64 | 0.67 | 0.58 | 0.80 | 0.69 | 0.71 | 0 .6 3 |
| g_Bifido bacterium | 0.01 30 | 0.01 54 | 0.00 16 | 0.00 20 | 0.00 00 | 0.13 | 0.84 | 0.76 | 0.87 | 0.50 | 0.66 | 0.55 | 0.71 | 0 .3 3 |
| g_Faecal ibacteriu m | 0.02 39 | 0.02 86 | 0.00 40 | 0.00 58 | 0.00 00 | 0.17 | 0.76 | 0.75 | 0.89 | 0.40 | 0.80 | 0.62 | 0.94 | 0 .17 |
| g_Bacill us | 0.00 22 | 0.00 33 | 0.00 04 | 0.00 11 | 0.00 02 | 0.19 | 0.70 | 0.67 | 0.84 | 0.38 | 0.51 | 0.69 | 0.86 | 0 .2 5 |
| g_Strept ococcus | 0.03 81 | 0.06 38 | 0.00 76 | 0.00 62 | 0.00 04 | 0.20 | 0.87 | 0.79 | 0.87 | 0.60 | 0.73 | 0.69 | 0.65 | 0 .7 5 |
| g_Clostr idium | 0.00 43 | 0.01 03 | 0.00 09 | 0.00 32 | 0.01 73 | 0.20 | 0.71 | 0.67 | 0.77 | 0.50 | 0.40 | 0.55 | 0.71 | 0 .1 3 |
| g_Veillo nella | 0.00 46 | 0.00 67 | 0.00 10 | 0.00 21 | 0.00 02 | 0.23 | 0.69 | 0.66 | 0.88 | 0.25 | 0.64' | 0.72 | 0.95 | 0 .1 3 |
| g_Parac occus | 0.00 74 | 0.01 44 | 0.00 17 | 0.00 43 | 0.00 52 | 0.23 | 0.69 | 0.63 | 0.83 | 0.15 | 0.57 | 0.52 | 0.88 | 0 .0 0 |
| g_Porph yromonas | 0.00 17 | 0.00 33 | 0.00 04 | 0.00 10 | 0.00 61 | 0.25 | 0.64 | 0.67 | 0.98 | 0.13 | 0.49 | 0.69 | 0.95 | 0 .0 0 |
| g_Kocur ia | 0.00 14 | 0.00 22 | 0.00 03 | 0.00 05 | 0.00 05 | 0.25 | 0.68 | 0.66 | 0.86 | 0.29 | 0.42 | 0.66 | 0.81 | 0 .2 5 |
| | | | | | | | | | | | | | | |
| g_Micro coccus | 0.00 66 | 0.01 13 | 0.00 17 | 0.00 16 | 0.00 13 | 0.26 | 0.67 | 0.70 | 0.98 | 0.05 | 0.62 | 0.55 | 0.94 | 0 .0 0 |
| g_Halo monas | 0.00 26 | 0.00 36 | 0.00 07 | 0.00 21 | 0.00 18 | 0.26 | 0.67 | 0.67 | 0.98 | 0.13 | 0.57 | 0.69 | 0.95 | 0 .0 0 |
| g_Prevot ella | 0.01 71 | 0.02 40 | 0.00 47 | 0.00 59 | 0.00 02 | 0.27 | 0.74 | 0.70 | 0.89 | 0.25 | 0.64 | 0.59 | 0.88 | 0 .1 7 |
| g_[Rumi nococcus] | 0.00 87 | 0.02 19 | 0.00 28 | 0.00 32 | 0.04 20 | 0.32 | 0.65 | 0.72 | 0.98 | 0.10 | 0.52 | 0.55 | 0.94 | 0 .0 0 |
| g_Enhyd robacter | 0.01 64 | 0.02 17 | 0.00 54 | 0.00 58 | 0.00 03 | 0.33 | 0.76 | 0.66 | 0.78 | 0.35 | 0.47 | 0.41 | 0.71 | 0 .0 0 |
| g_Actin omyces | 0.00 19 | 0.00 25 | 0.00 06 | 0.00 08 | 0.00 04 | 0.33 | 0.69 | 0.67 | 0.86 | 0.33 | 0.62 | 0.66 | 0.81 | 0 .2 5 |
| g_Haem ophilus | 0.00 45 | 0.00 56 | 0.00 18 | 0.00 36 | 0.00 62 | 0.40 | 0.70 | 0.61 | 0.88 | 0.13 | 0.51 | 0.69 | 0.90 | 0 .1 3 |
| g_Bacter oides | 0.04 45 | 0.03 48 | 0.01 93 | 0.01 77 | 0.00 00 | 0.43 | 0.78 | 0.72 | 0.87 | 0.35 | 0.68 | 0.59 | 0.88 | 0 .1 7 |
| g_Agrob acterium | 0.00 05 | 0.00 16 | 0.00 14 | 0.00 18 | 0.01 66 | 2.74 | 0.75 | 0.70 | 0.91 | 0.33 | 0.37 | 0.66 | 0.90 | 0 .0 0 |
| g_Pseud omonas | 0.03 56 | 0.05 78 | 0.09 80 | 0.04 63 | 0.00 00 | 2.75 | 0.91 | 0.88 | 0.94 | 0.75 | 0.70 | 0.72 | 0.88 | 0 .5 0 |
| g_Enter ococcus | 0.00 67 | 0.01 43 | 0.03 02 | 0.01 14 | 0.00 00 | 4.49 | 0.92 | 0.85 | 0.94 | 0.65 | 1.00 | 0.76 | 1.00 | 0 .4 2 |
| g_Acine tobacter | 0.04 19 | 0.05 69 | 0.34 48 | 0.19 38 | 0.00 00 | 8.22 | 0.95 | 0.91 | 0.98 | 0.75 | 0.74 | 0.83 | 1.00 | 0 .5 8 |
| g_Alcan ivorax | 0.00 02 | 0.00 11 | 0.00 19 | 0.00 19 | 0.00 00 | 9.44 | 0.89 | 0.81 | 0.98 | 0.50 | 0.74 | 0.79 | 0.95 | 0 .3 8 |
| g_Chrys eobacteri um | 0.00 17 | 0.00 32 | 0.01 81 | 0.01 26 | 0.00 00 | 10.8 4 | 0.93 | 0.90 | 0.96 | 0.75 | 0.73 | 0.83 | 1.00 | 0 .5 8 |
| g_Psych robacter | 0.00 03 | 0.00 09 | 0.00 33 | 0.00 25 | 0.00 00 | 10.8 8 | 0.88 | 0.85 | 0.93 | 0.71 | 0.69 | 0.86 | 1.00 | 0.5 0 |
| g_Stenot rophomon as | 0.00 02 | 0.00 08 | 0.00 49 | 0.00 40 | 0.00 00 | 20.5 4 | 0.94 | 0.90 | 0.95 | 0.79 | 0.70 | 0.83 | 0.90 | 0 .6 3 |
| g_Ochro bactrum | 0.00 01 | 0.00 04 | 0.00 17 | 0.00 19 | 0.00 00 | 23.1 9 | 0.87 | 0.82 | 0.95 | 0.58 | 0.73 | 0.90 | 1.00 | 0 .6 3 |
| g_Delfti a | 0.00 03 | 0.00 13 | 0.01 35 | 0.01 16 | 0.00 00 | 42.3 6 | 0.91 | 0.91 | 0.98 | 0.75 | 0.76 | 0.83 | 0.94 | 0 .6 7 |

### [Industrial Applicability]

According to the present invention, a risk group for heart disease can be diagnosed early and predicted by diagnosing a causative factor of heart disease through metagenomic analysis of bacteria-derived extracellular vesicles from a human body-derived sample, and thus the onset of heart disease can be delayed or heart disease may be prevented through appropriate management, and, even after heart disease occurs, early diagnosis for heart disease can be implemented, thereby lowering a disease rate and increasing therapeutic effects. In addition, patients diagnosed with heart disease are able to avoid exposure to causative factors predicted by metagenomic analysis, whereby the progression of heart disease is ameliorated, or recurrence of heart disease can be prevented.

## Claims

1. A method of diagnosing heart disease, the method comprising:
(a) extracting DNA from extracellular vesicles isolated from a subject sample;
(b) performing polymerase chain reaction (PCR) on the extracted DNA using a pair of primers having SEQ ID NO: 1 and SEQ ID NO: 2; and
(c) comparing an increase or decrease in content of bacteria-derived extracellular vesicles of the subject sample with that of a normal individual-derived sample through sequencing of a product of the PCR,
wherein the subject sample is blood;
wherein the heart disease is myocardial infarction, cardiomyopathy, variant angina, or atrial fibrillation; and
wherein, in process (c), myocardial infarction is predicted by comparing an increase or decrease in content of extracellular vesicles derived from one or more bacteria selected from the group consisting of the genus *Salinispora,* the genus *Candidatus Solibacter,* the genus *Citrobacter,* the genus *Collinsella,* the genus *Burkholderia,* the genus *Coprococcus,* the genus *Rhodoplanes,* the genus *Propionibacterium,* the genus *Methanobacterium,* the genus *Methanocella,* the genus *Streptacidiphilus,* the genus *Streptomyces,* the genus *Methanosaeta,* and the genus *Lysinibacillus;* or
wherein, in process (c), cardiomyopathy is predicted by comparing an increase or decrease in content of extracellular vesicles derived from one or more bacteria selected from the group consisting of the genus *Sphingomonas,* the genus *Thermoanaerobacterium,* the genus *Neisseria,* the genus *Turicibacter,* and the genus *Phascolarctobacterium;* or
wherein, in process (c), variant angina is predicted by comparing an increase or decrease in content of extracellular vesicles derived from one or more bacteria selected from the group consisting of the genus *Catenibacterium,* the genus *Deinococcus,* the genus *Fusobacterium,* and the genus *Adlercreutzia;* or
wherein, in process (c), atrial fibrillation is predicted by comparing an increase or decrease in content of extracellular vesicles derived from one or more bacteria selected from the group consisting of the genus *Chryseobacterium,* the genus *Enterococcus,* the genus *Alcanivorax,* the genus *Psychrobacter,* the genus *Bacteroides,* the genus *Geobacillus,* the genus *Haemophilus,* the genus *Veillonella,* the genus *Paracoccus,* the genus *Halomonas,* the genus *Ruminococcus,* and the genus *Porphyromonas.*

2. The method of claim 1, wherein the blood is whole blood, serum, plasma, or blood mononuclear cells.

## Patentansprüche

1. Verfahren zur Diagnose einer Herzkrankheit, wobei das Verfahren umfasst:
a) Extrahieren von DNA aus extrazellulären Vesikeln, die aus einer Patientenprobe isoliert wurden;
b) Durchführen einer Polymerase-Kettenreaktion (PCR) an der extrahierten DNA unter Verwendung eines Primer-Paares, das SEQ ID Nr: 1 und SEQ ID Nr: 2 aufweist; und
c) Vergleichen einer Erhöhung oder Verringerung des Gehalts von bakterienabgeleiteten, extrazellulären Vesikeln der Patientenprobe mit der einer normalen, individuell abgeleiteten Probe durch Sequenzierung eines Produkts der PCR,
wobei es sich bei der Probandenprobe um Blut handelt;
wobei es sich bei der Herzkrankheit um Herzinfarkt, Kardiomyopathie, Prinzmetal-Angina oder Vorhofflimmern handelt ist; und
wobei in Prozess (c) Herzinfarkt durch Vergleichen einer Erhöhung oder Verringerung des Gehalts an extrazellulären Vesikeln vorhergesagt wird, welche von einem oder mehreren Bakterien ausgewählt aus der Gruppe bestehend aus der Gattung *Salinispora,* der Gattung *Candidatus Solibacter,* der Gattung *Citrobacter,* der Gattung *Collinsella,* der Gattung *Burkholderia,* der Gattung *Coprococcus,* der Gattung *Rhodoplanes,* der Gattung *Propionibacterium,* der Gattung *Methanobacterium,* der Gattung *Methanocella,* der Gattung *Streptacidiphilus,* der Gattung *Streptomyces,* der Gattung *Methanosaeta* und der Gattung *Lysinibacillus* stammen; oder
wobei in Prozess (c) Kardiomyopathie durch Vergleichen einer Erhöhung oder Verringerung des Gehalts an extrazellulären Vesikeln vorhergesagt wird, welche von einem oder mehreren Bakterien ausgewählt aus der Gruppe bestehend aus der Gattung *Sphingomonas,* der Gattung *Thermoanaerobacterium,* der Gattung *Neisseria,* der Gattung *Turicibacter* und der Gattung *Phascolarctobacterium* stammen; oder
wobei in Prozess (c) Prinzmetal-Angina durch Vergleichen einer Erhöhung oder Verringerung des Gehalts an extrazellulären Vesikeln vorhergesagt wird, welche von einem oder mehreren Bakterien ausgewählt aus der Gruppe bestehend aus der Gattung *Catenibacterium,* der Gattung *Deinococcus,* der Gattung *Fusobacterium* und der Gattung *Adlercreutzia* stammen; oder
wobei in Prozess (c) Vorhofflimmern durch Vergleichen einer Erhöhung oder Verringerung des Gehalts an extrazellulären Vesikeln vorhergesagt wird, welche von einem oder mehreren Bakterien ausgewählt aus der Gruppe bestehend aus der Gattung *Chryseobacterium,* der Gattung *Enterokokken,* der Gattung *Alcanivorax,* der Gattung *Psychrobacter,* der Gattung *Bacteroides,* der Gattung *Geobacillus,* der Gattung *Haemophilus,* der Gattung *Veillonella,* der Gattung *Paracoccus,* der Gattung *Halomonas,* der Gattung *Ruminococcus* und der Gattung *Porphyromonas* stammen.

2. Verfahren nach Anspruch 1, wobei das Blut Vollblut, Serum, Plasma oder mononukleäre Blutzellen ist.

## Revendications

1. Procédé de diagnostic d'une maladie cardiaque, le procédé comprenant :
(a) l'extraction de l'ADN à partir de vésicules extracellulaires isolées à partir d'un échantillon sujet ;
(b) la réalisation d'une réaction en chaîne par polymérase (PCR) sur l'ADN extrait à l'aide d'une paire d'amorces présentant SEQ ID NO : 1 et SEQ ID NO : 2 ; et
(c) la comparaison d'une augmentation ou d'une diminution de la teneur en vésicules extracellulaires dérivées de bactéries de l'échantillon sujet à celle d'un échantillon dérivé d'un individu normal par séquençage d'un produit de la PCR,
dans lequel l'échantillon sujet est du sang ;
dans lequel la maladie cardiaque est un infarctus du myocarde, une cardiomyopathie, l'angor de repos ou une fibrillation auriculaire ; et
dans lequel, dans le processus (c), l'infarctus du myocarde est prédit en comparant une augmentation ou une diminution de la teneur en vésicules extracellulaires dérivées d'une ou de plusieurs bactéries sélectionnées dans le groupe constitué par le genre *Salinispora,* le genre *Candidatus Solibacler,* le genre *Citrobacter,* le genre *Collinsella,* le genre *Burkholderia,* le genre *Coprococcus,* le genre *Rhodoplanes,* le genre *Propionibacterium,* le genre *Methanobacterium,* le genre *Methanocella,* le genre *Streptacidiphilus,* le genre *Streptomyces,* le genre *Methanosaetaet* le genre *Lysinibacillus;* ou
dans lequel, dans le processus (c), la cardiomyopathie est prédite en comparant une augmentation ou une diminution de la teneur en vésicules extracellulaires dérivées d'une ou de plusieurs bactéries sélectionnées dans le groupe constitué par le genre *Sphingomonas,* le genre *Thermoanaerobacterium,* le genre *Neisseria,* le genre *Turicibacler,* et le genre *Phascolarctobacterium;* ou
dans lequel, dans le processus (c), l'angor de repos est prédit en comparant une augmentation ou une diminution de la teneur en vésicules extracellulaires dérivées d'une ou de plusieurs bactéries sélectionnées dans le groupe constitué par le genre *Catenibacterium,* le genre *Deinococcus,* le genre *Fusobacteriumet* le genre *Adlercreutzia;* ou
dans lequel, dans le processus (c), une fibrillation auriculaire est prédite en comparant une augmentation ou une diminution de la teneur en vésicules extracellulaires dérivées d'une ou de plusieurs bactéries sélectionnées dans le groupe constitué par le genre *Chryseobacterium,* le genre *Enterococcus,* le genre *Alcanivorax,* le genre *Psychrobacter,* le genre *Bacteroides,* le genre *Geobacillus,* le genre *Haemophilus,* le genre *Veillonella,* le genre *Paracoccus,* le genre *Halomonas,* le genre *Ruminococcuset* le genre *Porphyromonas.*

2. Procédé selon la revendication 1, dans lequel le sang est du sang total, du sérum, du plasma ou des cellules mononucléaires sanguines.
